(19) Europäisches Patentamt
European Patent Office
Office européen des brevets

(11) EP 3 199 540 A1

(12) EUROPEAN PATENT APPLICATION
published in accordance with Art. 153(4) EPC

(43) Date of publication:
02.08.2017 Bulletin 2017/31

(21) Application number: 15844011.5

(22) Date of filing: 25.09.2015

(51) Int Cl.:
*C07K 1/14* (2006.01) *C07K 1/04* (2006.01)

(86) International application number:
PCT/JP2015/077220

(87) International publication number:
WO 2016/047794 (31.03.2016 Gazette 2016/13)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(30) Priority: 26.09.2014 US 201462055991 P

(71) Applicants:
- Kaneka Corporation
  Osaka-shi, Osaka 530-8288 (JP)
- Anaspec, Inc.
  Fremont, CA 94555 (US)

(72) Inventors:
- TAKATSU, Keishi
  Takasago-shi
  Hyogo 676-8688 (JP)
- MAEDA, Hirofumi
  Takasago-shi
  Hyogo 676-8688 (JP)
- FUJII, Akio
  Takasago-shi
  Hyogo 676-8688 (JP)
- LI, Fengying
  Sunnyvale, California 94087 (US)
- WANG, Xiaobing
  Redmond, Washington 98052 (US)

(74) Representative: Hoffmann Eitle
Patent- und Rechtsanwälte PartmbB
Arabellastraße 30
81925 München (DE)

# (54) METHOD FOR PRODUCING HYDROPHOBIC PEPTIDE

(57) An object of the present invention is to provide a producing method: that allows any hydrophobic peptide to be used as an object to be purified, in hydrophilizing a hydrophobic peptide, synthesized by solid-phase peptide synthesis, with a hydrophilic unit and purifying the hydrophobic peptide by HPLC, regardless of the type of the amino acid residue at the N-terminus; that allows the hydrophilic unit to be flexibly selected in accordance with the type of the hydrophobic peptide; and that is excellent in versatility.

The present invention is characterized by a method for producing a purified peptide from a supported crude peptide including a support and a first peptide chain bonded to the support at a C-terminus, the method comprising: a hydrophilization step A of introducing a linker and a hydrophilic unit to an amino group of the supported crude peptide in this order stepwise or at a single step to obtain a support-free hydrophilized peptide;
a support cleavage step B of cleaving a bond between the first peptide chain and the support at any stage before bonding the linker to the supported crude peptide until the hydrophilized peptide is obtained, or after a supported hydrophilized peptide is obtained;
a chromatographic purification step of treating the support-free hydrophilized peptide obtained by the hydrophilization step A and the support cleavage step B, by liquid chromatography; and
a linker cleavage step of cleaving a bond between the linker and the first peptide chain included in the chromatographically-purified support-free hydrophilized peptide, by chemical treatment.

EP 3 199 540 A1

## Description

### TECHNICAL FIELD

The present invention relates to a method for producing a purified peptide by simply purifying a crude peptide to which a support is bonded, and a linker suitable for the method for producing the purified peptide.

### BACKGROUND ART

Typical examples of known methods for chemically synthesizing peptides and proteins include: solid-phase peptide synthesis in which beads of polystyrene polymer gel or the like are used as a solid phase, and a condensation reaction of an amino acid and deprotection of a terminal protecting group are repeated in the solid phase thereby to sequentially extend a peptide chain; and liquid-phase peptide synthesis in which a condensation reaction of an amino acid and deprotection of a terminal protecting group are repeated in a liquid phase thereby to extend a peptide chain. Among them, in solid-phase peptide synthesis, polymer gel is bonded to a produced peptide chain to be insolubilized, and thus crude purification or removal of soluble impurities such as a raw material reagent is easily performed by filtration or the like. A peptide obtained by detaching the polymer gel can be further purified by reversed-phase HPLC or the like if the peptide is hydrophilic. However, if the produced peptide is hydrophobic, the peptide cannot be purified by reversed-phase HPLC, so that a problem arises that it is difficult to achieve a high purity.

As a technique to purify a hydrophobic peptide synthesized by solid-phase peptide synthesis, a method is known in which, while a solid phase support is cleaved and removed from the hydrophobic peptide, a hydrophilic peptide is bonded to hydrophilize the hydrophobic peptide once, HPLC purification is performed, and then the hydrophilic peptide is cleaved and removed to obtain a high-purity hydrophobic peptide. For example, Patent Literature 1 describes a technique to bond a hydrophilic peptide to a hydrophobic peptide by applying biotechnology. However, in the case with biotechnology, the production efficiency of a desired hydrophobic peptide greatly depends on the sequence of the desired hydrophobic peptide, and mass production is difficult in some cases, so that there is a problem that the versatility of the technique is poor.

Examples of known techniques to continuously bond hydrophilic amino acids to a hydrophobic peptide to hydrophilize the hydrophobic peptide and perform HPLC purification include, in addition to the above-described peptide producing method using biotechnology, techniques using Boc solid-phase peptide synthesis (Non-Patent Literature 1) and techniques using Fmoc solid-phase peptide synthesis (Non-Patent Literatures 2 to 5, etc.). In the case of forming a peptide by using these chemical methods, deprotection of a protecting group of an amino acid and peptide synthesis are continuously repeated, and thus it is necessary that a bond between a hydrophobic peptide and a solid phase support is not cleaved under an amino acid deprotection reaction condition. For example, in Boc solid-phase peptide synthesis, a protecting group of an amino acid is deprotected by TFA, which is a strong acid, and thus a bond between a hydrophobic peptide and a solid phase support has resistance to a strong acid. HF is generally used for cleaving the bond between the hydrophobic peptide and the solid phase support. However, HF is dangerous and hazardous, and thus a special production apparatus is required.

In Fmoc solid-phase peptide synthesis, deprotection is possible under a basic condition, and thus the flexibility in bonding means to a solid phase support is high. For example, Non-Patent Literatures 2 or 3 discloses a method in which a hydrophilic peptide is synthesized on a solid phase support by Fmoc solid-phase peptide synthesis, 4-hydroxymethylbenzoic acid is then bonded as a linker to the N-terminus of the hydrophilic peptide, and a hydrophobic peptide is synthesized by Fmoc solid-phase peptide synthesis. In this method, cleavage between the solid phase support and a peptide chain is easy, and cleavage of the bond between the linker and the hydropbobic peptide is also easy. However, with this method, it is difficult to change the hydrophilic peptide according to the amino acid sequence of the desired peptide (hydrophobic peptide) as appropriate, so that the versatility of the method is poor. In addition, in removing the hydrophilic peptide from the hydroxyl group side of the linker, a strong base condition is required. Thus, the purity may be decreased due to a side reaction such as aspartimide formation, so that there is also a problem that the form of the C-terminus of the obtained hydrophobic peptide is limited only to a carboxylic acid.

The methods of Non-Patent Literatures 4 and 5 are superior to the methods of Non-Patent Literatures 2 and 3, since a hydrophobic peptide (desired peptide) can be previously synthesized by solid-phase peptide synthesis even though Fmoc solid-phase peptide synthesis is similarly employed. In these methods, it is necessary to complete the sequence of a hydrophobic peptide after a hydrophilic peptide and an amino acid having a phenylene type compound at the N-terminus thereof are synthesized. Thus, these methods can be said to be complicated processes, since a hydrophilic peptide and an amino acid having a phenylene type compound at the N-terminus thereof are required for each hydrophobic peptide. In addition, the finally obtained hydrophobic peptide cannot have cysteine or methionine at the N-terminus thereof, so that the versatility is poor. Furthermore, in these methods, it is difficult to change the hydrophilic peptide according to the amino acid sequence of a desired peptide (hydrophobic peptide) as appropriate, and the

versatility of the methods is poor also in this respect.

CITATION LIST

[PATENT LITERATURE]

Patent Literature 1: JP H07-102000

[NON PATENT LITERATURE]

Non-Patent Literature 1: Englebretsen, D.R. et al., Tetrahedron 1999, 55, 6623.
Non-Patent Literature 2: Hossain, M.A et al., Bioconjugate. Chem. 2009, 20, 1390.
Non-Patent Literature 3: Choma, C.T. et al., Tetrahedron Letters 1998, 39, 2417.
Non-Patent Literature 4: Vorherr, T., Chimica Oggi 2007, 25, 22.
Non-Patent Literature 5: Dick, F et al., Eur. Pept. Symp. 29th, Gdansk 2006, Poster Th009.

SUMMARY OF THE INVENTION

PROBLEMS TO BE SOLVED BY THE INVENTION

Therefore, an object of the present invention is to provide a producing method: that allows any hydrophobic peptide to be used as an object to be purified, in hydrophilizing a hydrophobic peptide, synthesized by solid-phase peptide synthesis, with a hydrophilic unit and purifying the hydrophobic peptide by HPLC, regardless of the type of the amino acid residue at the N-terminus; that does not limit the form of the C-terminus of the hydrophobic peptide to a carboxylic acid; that allows the hydrophilic unit to be flexibly selected in accordance with the type of the hydrophobic peptide; and that is excellent in versatility.

SOLUTION TO THE PROBLEMS

As a result of thorough research for solving the above-described problems, the present inventors first have made a hydrophilic unit selectable flexibly, by later introducing a linker and the hydrophilic unit to a desired peptide synthesized by solid-phase peptide synthesis. Accordingly, hydrophilization has been enabled without the form of the C-terminus being limited to a carboxylic acid. Then, the present inventors have found that, when it is made possible to cleavea bond between the desired peptide and the linker by chemical treatment in introducing the linker and the hydrophilic unit, the bond can be formed regardless of the type of the N-terminus amino acid of the desired peptide so that the type of the desired peptide can be expanded; and such a bond has resistance to an amino acid protecting group deprotection condition (a piperidine condition which is an Fmoc deprotection condition, etc.) and cleaving from a solid phase support (a TFA condition, etc.), it is possible to produce a support-free hydrophilized peptide (a bonded compound of the desired peptide, the linker, and the hydrophilic unit) for HPLC purification, and it is possible to simply cleave and remove the hydrophilic unit and the linker after the purification. Accordingly, the present inventors have completed the present invention.

That is, the present invention is characterized as follows.

1. A method for producing a purified peptide from a supported crude peptide including a support and a first peptide chain bonded to the support at a C-terminus, the method comprising:

a hydrophilization step A of introducing a linker and a hydrophilic unit to an amino group of the supported crude peptide in this order stepwise or at a single step to obtain a support-free hydrophilized peptide;
a support cleavage step B of cleaving a bond between the first peptide chain and the support at any stage before bonding the linker to the supported crude peptide until the hydrophilized peptide is obtained, or after a supported hydrophilized peptide is obtained;
a chromatographic purification step of treating the support-free hydrophilized peptide obtained by the hydrophilization step A and the support cleavage step B, by liquid chromatography; and
a linker cleavage step of cleaving a bond between the linker and the first peptide chain included in the chromatographically-purified support-free hydrophilized peptide, by chemical treatment.

2. The method for producing the purified peptide according to the above 1, wherein the linker cleavage step is performed by catalytic reduction or an acid.
3. The method for producing the purified peptide according to the above 2, wherein the linker cleavage step is performed by using a mixed solution containing an acid and a Lewis acid.
4. The method for producing the purified peptide according to the above 3, wherein the linker cleavage step is performed by using a mixed solution containing TFA and a Lewis acid.
5. The method for producing the purified peptide according to the above 4, wherein the linker cleavage step is performed by using a mixed solution containing TFA, TMSOTf, and thioanisole.
6. The method for producing the purified peptide according to the above 2, wherein the linker cleavage step is performed by using an acid having pKa of not higher than -2.
7. The method for producing the purified peptide according to the above 2, wherein the linker cleavage step is performed by using a metal supported catalyst.
8. The method for producing the purified peptide according to the above 1 to 7, wherein the hydrophilic unit is a hydrophilic peptide, a polyether, or a polyamine.
9. The method for producing the purified peptide according to the above 1 to 8, wherein

the hydrophilization step A comprises a linker bonding step of bonding the linker to the amino group of the crude peptide to obtain a linker-bonded compound, and a hydrophilic unit bonding step of bonding the hydrophilic unit to a linker moiety of the obtained linker-bonded compound,
wherein, the hydrophilic unit bonding step comprises
bonding a plurality of amino acids to the linker moiety stepwise by Fmoc solid-phase peptide synthesis,
bonding a previously prepared hydrophilic peptide chain to the linker moiety,
bonding a previously prepared polyether to the linker moiety, or
bonding a previously prepared polyamine to the linker moiety.

10. The method for producing the purified peptide according to the above 1 to 9, wherein the support cleavage step B is performed after the supported hydrophilized peptide is obtained by the hydrophilization step A.
11. The method for producing the purified peptide according to the above 1 to 10, wherein washing with water or a water-containing solvent is performed after the linker cleavage step.
12. The method for producing the purified peptide according to any one of the above 1 to 11, wherein a plurality of amino acids are sequentially bonded to the support by Fmoc solid-phase peptide synthesis to form the supported crude peptide.
13. The method for producing the purified peptide according to the above 1 to 12, wherein the linker is bonded to the first peptide chain by forming a benzyloxycarbonylamino group or a benzylamino group.
14. The method for producing the purified peptide according to the above 1 to 13, wherein the linker has a first group capable of forming a benzyloxycarbonylamino group or a benzylamino group together with the crude peptide and a second group capable of making a chemical bond to the hydrophilic unit.
15. The method for producing the purified peptide according to the above 1 to 14, wherein the hydrophilic unit has a log P (P is an octanol-water partition coefficient) value of not higher than -1.
16. The method for producing the purified peptide according to the above 1 to 15, wherein the hydrophilic unit is a hydrophilic peptide chain having two or more residues of at least one or more types of amino acid residues selected from the group of arginine, asparagine, glutamine, histidine, and lysine.
17. The method for producing the purified peptide according to the above 1 to 16, wherein the hydrophilic unit is a hydrophilic peptide chain having two or more residues of at least one or more types of amino acid residues selected from the group of aspartic acid and glutamic acid.
18. The method for producing the purified peptide according to the above 8 to 17, wherein the number of the amino acid residues of the hydrophilic peptide chain is not less than 2 and not greater than 35.
19. A linker, comprising:

a first group capable of forming a benzyloxycarbonylamino group or a benzylamino group together with a primary or secondary amine, and
a second group capable of making a chemical bond to a hydrophilic unit.

20. A linker represented by formula (1):

[Chemical formula 1]

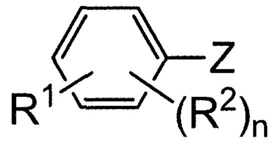

(1)

wherein $R^1$ is an organic group having a protected primary amino group, $R^2$ is an electron-withdrawing group, n is an integer of 0 to 4, and Z is a halogenated methyl group, a formyl group, or a carbonate group represented by formula (a):

[Chemical formula 2]

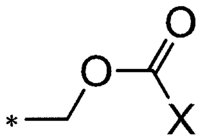

(a)

wherein X is a leaving group and * indicates a site bonded to the benzene ring of the compound (1).

21. A linker represented by formula (2), (4), or (6):

[Chemical formula 3]

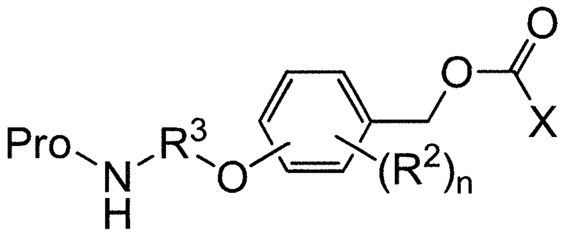

(2)

wherein $R^2$ is an electron-withdrawing group, n is an integer of 0 to 4, Pro is an amino-protecting group, X is $-OR^x$ ($R^x$ is a heterocyclic imide group), and $R^3$ is an alkylene group having 1 to 10 carbon atoms;

[Chemical formula 4]

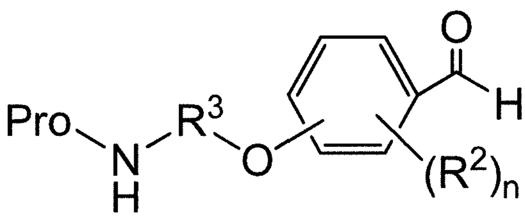

(4)

wherein $R^2$ is an electron-withdrawing group, n is an integer of 0 to 4, Pro is an amino-protecting group, and $R^3$ is an alkylene group having 1 to 10 carbon atoms; and

[Chemical formula 5]

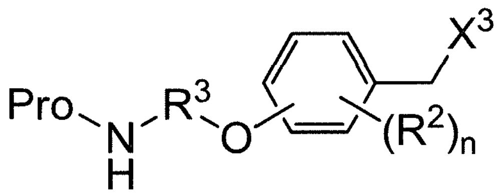

(6)

wherein $R^2$ is an electron-withdrawing group, n is an integer of 0 to 4, Pro is an amino-protecting group, $R^3$ is an alkylene group having 1 to 10 carbon atoms, and $X^3$ is a halogen atom.
22. A linker represented by formula (3a):

[Chemical formula 6]

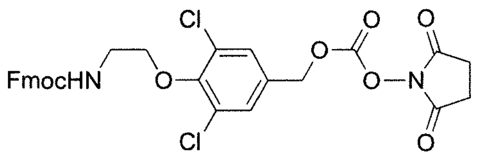

(3a)

ADVANTAGEOUS EFFECTS OF THE INVENTION

According to the present invention, the linker and the hydrophilic unit are introduced to the desired peptide in this order later, and a bond between the desired peptide and the linker may be optimized to be cleaved by chemical treatment. Thus, the form and the sequence of the desired peptide are not limited. Therefore, the type of the desired peptide can be expanded, and cleavage of the hydrophilic unit after purification is also easy.

BRIEF DESCRIPTION OF THE DRAWINGS

[FIG. 1] FIG. 1 shows HPLC analysis results of a hydrophilized peptide (crude product) obtained in Example 1.
[FIG. 2] FIG. 2 shows mass spectrometry analysis results of the hydrophilized peptide (crude product) obtained in Example 1.
[FIG. 3] FIG. 3 shows HPLC analysis results of the hydrophilized peptide after purification obtained Example 1.
[FIG. 4] FIG. 4 shows mass spectrometry analysis results of the hydrophilized peptide after purification obtained in Example 1.
[FIG. 5] FIG. 5 shows HPLC analysis results of a high-purity hydrophobic peptide GILTVSVAV obtained in Example 1.
[FIG. 6] FIG. 6 shows mass spectrometry analysis results of the high-purity hydrophobic peptide GILTVSVAV obtained in Example 1.
[FIG. 7] FIG. 7 shows HPLC analysis results of a hydrophilized peptide (crude product) obtained in Example 4.
[FIG. 8] FIG. 8 shows mass spectrometry analysis results of the hydrophilized peptide (crude product) obtained in Example 4.
[FIG. 9] FIG. 9 shows HPLC analysis results of the hydrophilized peptide after purification obtained in Example 4.
[FIG. 10] FIG. 10 shows mass spectrometry analysis results of the hydrophilized peptide after purification obtained in Example 4.
[FIG. 11] FIG. 11 shows HPLC analysis results of a high-purity hydrophobic peptide LVFFAEDVGSNKGAIIGLMVGGW obtained in Example 4.
[FIG. 12] FIG. 12 shows mass spectrometry analysis results of the high-purity hydrophobic peptide LVFFAEDVGSNKGAIIGLMVGGVV obtained in Example 4.

DESCRIPTION OF EMBODIMENTS

<Method for producing purified peptide>

Scheme I shown below indicates an outline of a production process of the present invention. Hereinafter, the present invention will be described in detail with reference to the scheme as appropriate.

[Chemical formula 7]

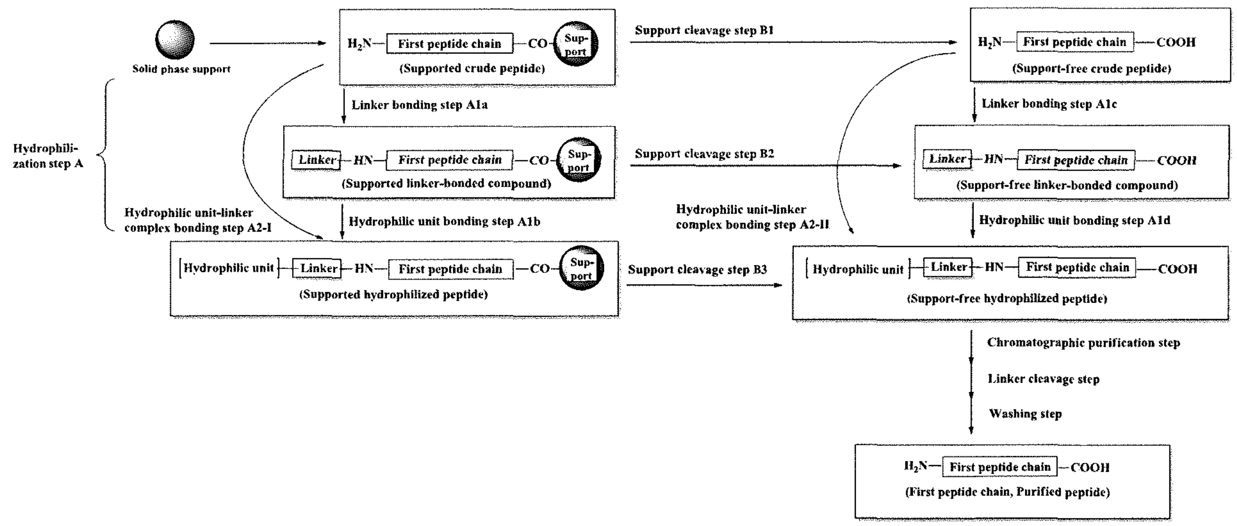


The method for producing a purified peptide according to the present invention uses, as an object to be purified (target), a supported crude peptide including a support and a first peptide chain bonded to the support at a C-terminus. The method is intended to sequentially treat the object to be purified to finally obtain the purified free first peptide chain (purified peptide).

The treatment procedure from the object to be purified (supported crude peptide) to the intended object (first peptide chain) includes:

a hydrophilization step A (a step A1a, A1b, or A2-I in Scheme I) of introducing a linker and a hydrophilic unit to an amino group of a crude peptide (either a supported crude peptide or a support-free crude peptide in Scheme I) in this order stepwise or at a single step to obtain a hydrophilized peptide (either a supported hydrophilized peptide or a support-free hydrophilized peptide in Scheme I);
a support cleavage step B (any one of steps B1, B2, and B3 in Scheme I) of cleaving a bond between the first peptide chain and the support at any stage before bonding the linker to the supported crude peptide until the hydrophilized peptide is obtained, or after the supported hydrophilized peptide is obtained;
a chromatographic purification step of dissolving the support-free hydrophilized peptide obtained by the hydrophilization step A and the support cleavage step B, in water and treating the support-free hydrophilized peptide by liquid chromatography; and
a linker cleavage step of cleaving a bond between the linker and the first peptide chain included in the chromatographically-purified support-free hydrophilized peptide, by chemical treatment.

In this method, the first peptide chain (desired peptide chain) is previously formed on the support, and the hydrophilic unit is bonded later. Thus, the hydrophilic unit can be flexibly selected, and the flexibility in process design can be increased. In addition, according to the present invention, the hydrophilic unit is bonded to the first peptide chain in the hydrophilization step A, and the support is removed in the cleavage steps B1 to B3. Thus, the first peptide chain can be hydrophilized, and chromatographic purification is enabled. In particular, in the method of the present invention, the bond between the linker and the first peptide chain is a bond that can be cleaved by chemical treatment, and such a bond has durability against an amino acid protecting group removal condition. Thus, for example, it is possible to form the hydrophilic unit by Fmoc synthesis. Additionally, since such a bond has durability against a solid phase support cleavage removal condition (a TFA condition, etc.), the solid phase support can be removed even after the hydrophilic unit is introduced (as a matter of course, before introducing the hydrophilic unit), and thus the flexibility in design is very high. Furthermore, this bond can be formed regardless of the type of the N-terminus amino acid of the first peptide chain, and thus it is possible to increase the versatility of the first peptide chain (desired peptide). Since the hydrophilic unit can be cleaved and removed by the chemical treatment for each linker after the chromatographic purification as described above, a special facility for the cleavage and removal is unnecessary, and the purified first peptide chain (purified peptide) can be simply produced. Hereinafter, the present invention will be described in detail sequentially for the steps.

1. Step of producing supported crude peptide

First, a step of producing the supported crude peptide, which is an object to be purified, will be described. The "supported crude peptide" refers to a bonded compound including a support and a first peptide chain bonded to the support at a C-terminus. The "first peptide chain" refers to a compound polymerized by two or more amino acids forming peptide bonds between amino groups and carboxyl groups, and the present invention is intended to purify the first peptide chain. In the present specification, the "crude peptide" is merely a concept including both a supported crude peptide and a support-free crude peptide. The step of producing the supported crude peptide is not essential for the present invention, and the purification method of the present invention is also applicable to a crude peptide obtained through another process. It is easy to produce the supported crude peptide through the following step. In the following step of producing the supported crude peptide, solid-phase peptide synthesis is carried out in which a plurality of amino acids are sequentially bounded to the N-terminus of the support.

Each of the amino acids is a compound having at least one or more basic amino groups (-NH2) and at least one or more acidic carboxyl groups (-COOH). The amino acid is not particularly limited in the present invention, but preferable examples of the amino acid include: α-amino acids such as glycine, alanine, 3,3,3-trifluoroalanine, 2-aminobutanoic acid, 2-amino-2-methylbutanoic acid, norvaline, 5,5,5-trifluoronorvaline, valine, 2-amino-4-pentenoic acid, 2-amino-2-methylpentenoic acid, propargylglycine, 2-amino-cyclopentanecarboxylic acid, norleucine, leucine, isoleucine, tert-leucine, 2-amino-4-fluoro-4-methylpentanoic acid, 4-aminocyclohexanecarboxylic acid, serine, O-methylserine, O-allylserine, threonine, homoserine, cysteine, 2-methylcysteine, methionine, penicillamine, aspartic acid, asparagine, arginine, histidine, glutamic acid, glutamine, 2-aminoadipic acid, ornithine, thyrosin, tryptophan, lysine, (1R,2S)-1-amino-2-vinyl-cyclopropanoic acid, (1R,2S)-1-amino-2-ethyl-cyclopropanoic acid, sarcosine, phenylalanine, N-methylalanine, and N,N-dimethylalanine; cyclic amino acids such as aziridine carboxylic acid, azetidine carboxylic acid, proline, 1-methylproline, 2-methylproline, 2-ethylproline, 3-methylproline, 4-methylproline, 5-methylproline, 4-methyleneproline, 4-hydroxyproline, 4-fluoroproline, pipecolic acid, nipecotic acid, and isonipecotic acid; ßamino acids such as ß-alanine, isoserine, 3-aminobutanoic acid, 3-aminopentanoic acid, and B-leucine; and γ-amino acids such as 4-aminobutanoic acid, 4-amino-3-methylpropionic acid, 4-amino-3-propylbutanoic acid, 4-amino-3-isopropylbutanoic acid, and 4-amino-2-hydroxybutanoic acid. Preferable amino acids are natural amino acids, α-amino acids, and the like, and natural α-amino acids. In the present invention, the numbers of amino groups and carboxyl groups in one molecule of the amino acid are not particularly limited, and, for example, each of the numbers may be one, or may be a plural number. In addition, the numbers of amino groups and carboxyl groups in one molecule of the amino acid may be equal to each other or may be different from each other.

The solubility of the first peptide chain in water is evaluated on the basis of a GRAVY (grand average of hydropathy) score. The GRAVY score means an average hydropathy score for all the amino acids in a protein. The GRAVY score can be calculated according to the method reported by Kyte et al. (J. Kyte, RF. Doolittle, J Mol. Biol 152 (1982) 105). Generally, a peptide having a positive score tends to be hydrophobic, and a peptide having a negative score tends to be hydrophilic. According to the present invention, it is possible to purify a hydrophobic peptide of which purification is conventionally difficult. The GRAVY score of such a hydrophobic peptide is preferably not less than -0.5, more preferably not less than 0, and further preferably not less than 0.5. The upper limit is not particularly limited, but the GRAVY score of such a hydrophobic peptide is generally not greater than 10 and may be not greater than 5.

In addition, according to the present invention, it is possible to purify even a first peptide chain containing a sulfur-containing amino acid, such as cysteine and methionine, of which synthesis is conventionally difficult. Thus, the present invention is also useful for purifying a peptide containing a sulfur-containing amino acid.

The number of the amino acid residues of the first peptide chain is, for example, preferably not less than 2, more preferably not less than 4, and further preferably not less than 6. The upper limit is not particularly limited, but the number of the amino acid residues is preferably not greater than 200, more preferably not greater than 150, and further preferably not greater than 100.

In the step of producing the supported crude peptide, the first peptide chain is produced by solid-phase peptide synthesis. The solid-phase peptide synthesis is a method in which the C termini of amino acids are sequentially bonded to a water-insoluble solid phase support such as polystyrene via a linker bonded directly to the support, to sequentially extend a peptide. After a desired sequence is completed, a desired peptide can be produced by cleaving the produced peptide chain from the support. According to this synthesis method, a wide range of peptides can be synthesized without depending on an amino acid sequence, and thus automation is also easy.

As a method for sequentially bonding a plurality of amino acids to the support to synthesize the supported crude peptide, Fmoc solid-phase peptide synthesis using a Fmoc group as a protecting group at an N-terminus is preferable. Generally, in Fmoc solid-phase peptide synthesis, deprotection is performed under a basic condition such as an amide-based solvent (DMF, etc.) and an amine-based base (in particular, piperidine), and the produced peptide can be cleaved from the support under an acidic condition (TFA, etc.).

As amino acids to be used in Fmoc solid-phase peptide synthesis, general amino acids of which the N-terminus

is protected can be used. In the case of bonding a linker to the main chain or side chain of a peptide, an amino acid having, as a protecting group, a group that can be deprotected under a condition in which piperidine and TFA are not used, can also be used. In addition, a protecting group may be introduced to any amino acid residue after the first peptide chain is formed.

In the case of performing peptide synthesis by Fmoc solid-phase peptide synthesis, an amino acid of which the N-terminus is Fmoc-protected and of which the carboxylic acid group is activated is reacted with the N-terminus of a peptide to form a new peptide bond. Then the Fmoc protecting group is deprotected such that a new N-terminus appears, and the next amino acid of which the N-terminus is Fmoc-protected and of which the carboxylic acid group is activated by the above method is reacted with the new N-terminus. Subsequently, the same procedure is repeated thereby to synthesize a peptide chain.

The above water-insoluble solid phase support is not particularly limited, but the water-insoluble solid phase support which is widely used in solid-phase peptide synthesis, such as Rink amide resin, Rink Amide AM resin, Rink amide PEGA resin, Rink amide MBHA resin, Rink amide AM resin, Rink Amide NovaGel, NovaSyn TGR resin, NovaSyn TGA resin, TentaGel resin, Wang resin, HMPA-PEGA resin, HMP-NovaGel, HMPA-NovaGel, 2-Chlorotrityl resin, NovaSyn TGT alcohol resin, HMPB-AM, Sieber Amide resin, NovaSyn TG Sieber resin, 4-(4-Formyl-3-methoxyphenoxy)butyryl AM resin, 4-Sulfamylbutyryl AM resin, 4-Sulfamylbutyryl AM PEGA, 4-Fmoc-hydrozinobenzoyl AM resin, HMBA-AM resin, HMBA-NovaGel, and DHP HM, is preferably used. The solid phase support preferably has, on the surface thereof, an amino group that is a starting point of solid-phase peptide synthesis, and the amino group may be protected. If an amino group is present on the surface of the solid phase support, the first peptide chain and the support can be bonded to each other via an amide group.

In synthesis of a peptide, a peptide bond may be formed by activating the carboxyl group of an amino acid by various active ester methods or a coupling reagent, typified by: triazoles such as 1-hydroxybenzotriazole (HOBt) and 1-hydroxy-7-azabenzotriazole (HOAt); fluorophosphates such as O-(benzotriazol-1-yl)-N,N,N',N'-tetramethyluronium hexafluorophosphate (HBTU), O-(7-azabenzotriazol-1-yl)-N,N,N',N'-tetramethyluronium hexafluorophosphate (HATU), O-(6-chlorobenzotriazol-1-yl)-N,N,N',N'-tetramethyluronium hexafluorophosphate (HCTU), N,N,N',N'-tetramethyl-O-(N-succinimidyl)uronium hexafluoroborate (TSTU), (1-cyano-2-ethoxy-2-oxoethylideneaminooxy)dimethylaminomorpholinocarbenium hexafluorophosphate (COMU), and 1H-benzotriazol-1-yloxytripyrrolidinophosphonium hexafluorophosphate (PyBOP); carbodiimides such as 1-(3-dimethylaminopropyl)-3-ethylcarbodiimide hydrochloride (EDC-HCl), diisopropylcarbodiimide (DIC), and dicyclohexylcarbodiimide (DCC); propanephosphonic acid anhydride (T3P); and the like, and reacting the activated carboxyl group of the amino acid with the N-terminus amino group of the peptide.

After the supported crude peptide is synthesized by the Fmoc solid-phase peptide synthesis, the supported crude peptide may be filtered and washed with an appropriate organic solvent (e.g., DMF, methylene chloride, etc.) as necessary. By performing such filtration and washing, soluble impurities (raw materials, etc.) can be simply removed, so that the purity of the supported crude peptide can be increased. Before the support is cleaved and removed, this filtration and washing step can be performed as appropriate even in the step described below.

2. Hydrophilization step

The hydrophilization step is the initial step in the present invention, and is characterized by introducing the linker and the hydrophilic unit to the amino group of the crude peptide (either a supported crude peptide or a support-free crude peptide) in this order stepwise or at a single step to obtain a hydrophilized peptide (either a supported hydrophilized peptide or a support-free hydrophilized peptide).

As the crude peptide to be used in the hydrophilization step, peptides obtained through various processes can be used, but the supported crude peptide produced by the above step is preferably used since the above step is easy. The method for introducing the linker and the hydrophilic unit to the supported crude peptide produced by the above step is not particularly limited, and may include a linker bonding step (A1a in Scheme I) of bonding the linker to the amino group of the supported crude peptide and a hydrophilic unit bonding step (A1b in Scheme I) of bonding the hydrophilic unit to the linker moiety of the obtained supported linker-bonded compound, or a hydrophilic unit-linker complex bonding step (A2-I in Scheme I) of bonding a complex obtained in advance by combining the hydrophilic unit and the linker, to the amino group of the supported crude peptide may be carried out.

In addition, in the hydrophilization step, a support-free crude peptide can be used. A linker bonding step (A1c in Scheme I) of bonding the linker to the support-free crude peptide, a hydrophilic unit bonding step (A1d in Scheme I), and a hydrophilic unit-linker complex bonding step (A2-II in Scheme I) can be carried out similarly to A1a, A1b, and A2-I.

The bonded position of the linker may either be a terminus or a side chain of the first peptide chain as long as the position is at an amino group in the crude peptide that is bonded to at least one or more hydrogen atoms.

<Linker bonding step A1a>

In the present invention, the "linker" refers to a compound that has a first bonding group capable of being bonded to the first peptide chain (e.g., an amino group) and also a second bonding group capable of being bonded to the hydrophilic unit described later and that can be present between the first peptide chain and the hydrophilic unit in a state where the compound is bonded to the first peptide chain and the hydrophilic unit after bonding. In the present specification, a product obtained by the linker bonding step is referred to as a "linker-bonded compound", such a product to which the support is bonded is particularly referred to as a "supported linker-bonded compound", and such a product from which the support is removed is particularly referred to as a "support-free linker-bonded compound". In the present invention, as described later, after the linker is bonded to the supported crude peptide, an amino acid may be connected to the linker by peptide synthesis to prepare a hydrophilic unit. Thus, it is necessary that a bond between the linker and the first peptide chain is not cleaved under a peptide synthesis condition (in particular, an amino acid deprotection condition), and, for example, in the case with Fmoc synthesis, the bond between the linker and the first peptide chain is required to have durability against a basic condition that is an Fmoc deprotection condition. In addition, the bond between the linker and the first peptide chain is required to have resistance to an agent (e.g., an acidic condition such as TFA in the case of Fmoc solid-phase peptide synthesis) used when cleaving the first peptide chain from the support. This is for making it possible to bond the hydrophilic unit, by preventing the bond between the linker and the first peptide chain from being cleaved when the first peptide chain and the support are cleaved.

In order to meet these requirement characteristics, the linker has, as the first group at the first peptide chain side thereof, a first group capable of forming a benzyloxycarbonylamino group or a benzylamino group together with a primary or secondary amine (e.g., the amino group of the first peptide chain) having at least one or more hydrogen atoms. Such a first group not only can bond the C-terminus or the like of the first peptide chain and the linker to each other but also has resistance to an acidic or basic condition. Meanwhile, the second group at the hydrophilic unit side of the linker is a group capable of making a chemical bond to the hydrophilic unit.

Examples of a preferable linker having such a first group and such a second group include a compound represented by the following formula (1):

[Chemical formula 8]

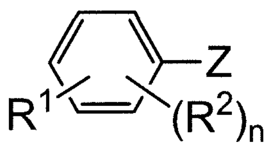

(1)

wherein $R^1$ is an organic group having a protected primary amino group, $R^2$ is an electron-withdrawing group, n is an integer of 0 to 4, and Z is a halogenated methyl group, a formyl group, or a carbonate group represented by the following formula (a):

[Chemical formula 9]

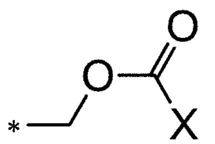

(a)

wherein X is a leaving group and * indicates a site bonded to the benzene ring of the compound (1). The leaving group X in the formula (a) is not particularly limited, but examples of the leaving group X include halogen atoms such as a fluorine atom, a chlorine atom, a bromine atom, and an iodine atom; and an organic group represented by $-OR^x$ ... (9) ($R^x$ represents an aliphatic hydrocarbon group, an alicyclic hydrocarbon group, an aromatic hydrocarbon group, or a heterocyclic group, and these groups may each have a substituent).

The aliphatic hydrocarbon group preferably has 1 to 20 carbon ato ms, and specific examples of the aliphatic hydrocarbon group include: alkyl gro ups such as a methyl group, an ethyl group, an n-propyl group, an isopropyl g roup, an n-butyl group, an isobutyl group, an s-butyl group, a t-butyl group, a n n-pentyl group, an n-hexyl group, an n-octyl group, an n-decyl group, an n-d odecyl group, and an n-octadecyl group; alkenyl groups such as a vinyl group and an allyl group; and alkynyl groups such as an ethynyl group and a propa rgyl group.

In addition, the alicyclic hydrocarbon group preferably has 3 to 20 carbon atoms, and specific examples of the alicyclic hydrocarbon group include: cycloalkyl groups such as a cyclopropyl group, a cyclobutyl group, a cyclopent yl group, a cyclohexyl group, a cyclooctyl group, a norbornyl group, and an ad amanthyl group; cycloalkylalkyl groups such as a cyclopentylmethyl group and a cyclohexylmethyl group; and the like.

The aromatic hydrocarbon group preferably has 6 to 20 carbon atoms, and specific examples of the aromatic hydrocarbon group include: aryl groups such as a phenyl group, a naphthyl group, and an indenyl group; and aralkyl groups such as a benzyl group and a 4-phenylbutyl group; and the like.

Furthermore, the heterocyclic group is obtained by substituting a part of the carbons forming the ring with an atom other than carbon, such as nitrogen, oxygen, and sulfur, in the alicyclic hydrocarbon group or aromatic hydrocarbon group, and preferably has 3 to 20 carbon atoms. The heterocyclic group is more preferably a nitrogen-containing heterocyclic imide group having at least one imide group in a nitrogen-containing heterocyclic group. Examples of such a nitrogen-containing heterocyclic imide group include saturated or unsaturated nitrogen-containing heterocyclic imide groups shown below.

[Chemical formula 10]

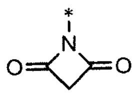
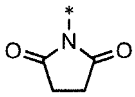
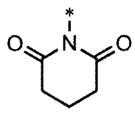
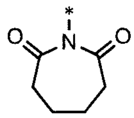
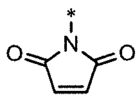
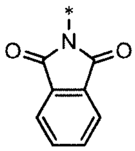

Particularly preferable examples of such a heterocyclic imide group include a succinimidyl group, a maleimidyl group, a phthalimidyl group, and the like.

The aliphatic hydrocarbon group, the alicyclic hydrocarbon group, t he aromatic hydrocarbon group, or the heterocyclic group may have a substitu ent, and examples of the substituent include: alkyl groups; halogen atoms such as a fluorine atom, a chlorine atom, a bromine atom, and an iodine atom; an amino group; a hydroxy group; a nitro group; and the like.

Examples of such $-OR^x$ include N-succinimidyloxy group, an N-mal eimidyloxy group, an N-phthalimidyloxy, a p-nitrophenyloxy group, a benzyloxy group, and the like. Among them, an N-succinimidyloxy group is preferable.

Because of such a structure, the linker can be bonded to the first peptide chain by forming a benzyloxycarbonylamino group or a benzylamino group. Hereinafter, in the present specification, these compounds are referred to as a carbonate type linker, an aldehyde type linker, or a halogen type linker, depending on the structure of the Z group.

In the formula (1), $R^1$ is an organic group having a protected primary amino group. Such a substituent is not particularly limited as long as a protected primary amino group is present at the terminal of the organic group, but such a substituent is preferably, for example, an organic group having a protected primary amino group represented by the following formula (b):

[Chemical formula 11]

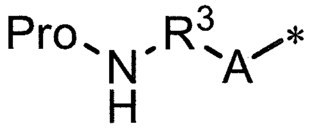 **(b)**

wherein $R^3$ is a bivalent hydrocarbon group, A is one substituent selected from $-CH_2-$, -O-, -S-, and -NH-, Pro is an amino-protecting group, and * indicates a site bonded to the benzene ring of the compound (1).

In the formula (b), $R^3$ is a bivalent hydrocarbon group, is more pr eferably a bivalent aliphatic hydrocarbon group which may be saturated or un saturated, and is particularly preferably an alkylene group, and an alkylene gr oup having 1 to 10 carbon atoms is preferable, and an alkylene group having 2 to 5 carbon atoms is more preferable. Examples of preferable alkylene grou ps include $-CH_2-$, $-CH_2CH_2-$, $-CH_2CH_2CH_2-$, $-CH_2CH_2CH_2CH_2-$, $-CH(CH_2)CH_2-$, $-CH(C_2Hs)CH_2-$, $-C(CH_2)_2CH_2-$, $-CH_2CH_2CH_2CH_2CH_2-$, $-CH_2CH_2CH_2CH_2CH_2CH_2-$, and the like. Among them, $-CH_2CH_2-$ (ethylene group), $-CH(CH_2)CH_2-$ (propyle ne group) or $-CH_2CH_2CH_2-$ (trimethylene group) is preferable, and

$-CH_2CH_2-$ (e thylene group) is the most preferable.

In the formula (b), A is one substituent selected from $-CH_2-$, -O-, -S-, and -NH-. Among them, -O- or -S- is preferable, and -O- is more preferable.

In the formula (b), Pro is not particularly limited as long as Pro is an amino-protecting group, and examples of Pro include acyl-based groups, carbamate-based groups, imide-based groups, sulfonamide-based groups, and the like. Among them, carbamate-based protecting groups, such as an acetyl group, a tert-butoxycarbonyl group (-Boc), a benzyloxycarbonyl group (-Z), a 9-fluorenylmethyloxycarbonyl group (-Fmoc), a 2,2,2-trichloroethoxycarbonyl group (-Troc), and an allyloxycarbonyl group (-Alloc), are preferable. In particular, since a subsequent reaction can proceed with an amino group in the first peptide chain as a starting point, the above Pro is preferably one that is the same as an amino-protecting group in the first peptide chain, and is more preferably a 9-fluorenylmethyloxycarbonyl group (-Fmoc).

In the formula (b), $R^2$ is an electron-withdrawing group and is more preferably at least one substituent selected from a halogen, a cyano group, a nitro group, a sulfo group, an alkyloxycarbonyl group, and an acyl group.

Examples of the halogen include fluorine, chlorine, bromine, and iodine. Among them, chlorine is particularly preferable.

Examples of the alkyloxycarbonyl group include a methoxycarbonyl group, an ethoxycarbonyl group, a propoxycarbonyl group, a butoxycarbonyl group, a pentyloxycarbonyl group, a hexyloxycarbonyl group, a heptyloxycarbonyl group, an octyloxycarbonyl group, a decyloxycarbonyl group, an octadecyloxycarbonyl group, a trifluoromethyloxycarbonyl group, and the like.

Examples of the acyl group include an acetyl group, a propanoyl group, a butanoyl group, a pentanoyl group, a hexanoyl group, a heptanoyl group, an octanoyl group, a nonanoyl group, a decanoyl group, a trifluoroacetyl group, a benzoyl group, a 1-naphthoyl group, a 2-naphthoyl group, and the like. Among them, an acyl group having 2 to 15 carbon atoms is preferable, and an acyl group having 2 to 10 carbon atoms is more preferable.

In the formula (1), n is an integer of 0 to 4, more preferably 1, 2, or 3, and further preferably 2. When n is not less than 2, a plurality of $R^2$s may be the same or different from each other.

The bonded positions of $R^1$ and $R^2$ to the benzene ring are not particularly limited. The bonded position of $R^1$ may be any of the 2 to 6 positions as seen from the carbonate group, further preferably the 2 position, the 3 position, the 4 position, or the 5 position, more preferably the 2 position or the 4 position, and particularly preferably the 4 position. In addition, in the case where $R^2$ is bonded, the bonded position of $R^2$ may be any of the 2 to 6 positions, and, in a more preferred embodiment, $R^2$ may be bonded to at least each of the 3 position and the 5 position of the benzene ring.

Regarding the above-described linker, the carbonate type linker is preferably, for example, a compound represented by the following formula (2):

[Chemical formula 12]

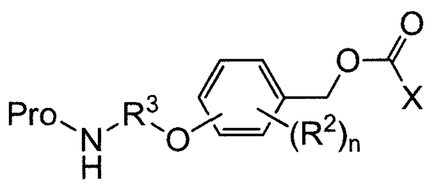

(2)

wherein $R^2$ is an electron-withdrawing group, n is an integer of 0 to 4, Pro is an amino-protecting group, X is $-OR^x$ ($R^x$ is a heterocyclic imide group), and $R^3$ is an alkylene group having 1 to 10 carbon atoms. X is preferably an N-succinimidyloxy group, and $R^2$, n, Pro, and $R^3$ are preferably the same as those in formula (1). Among them, the most preferable carbonate type linkers are, for example, compounds represented by the following formulas (3a) to 3(e):

[Chemical formula 13]

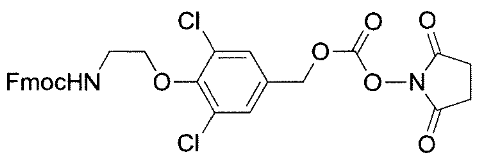

(3a)

[Chemical formula 14]

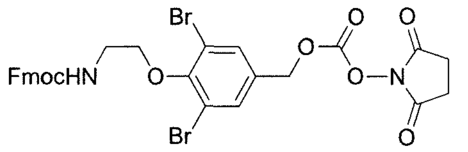

(3b)

[Chemical formula 15]

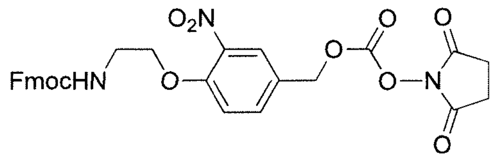

(3c)

[Chemical formula 16]

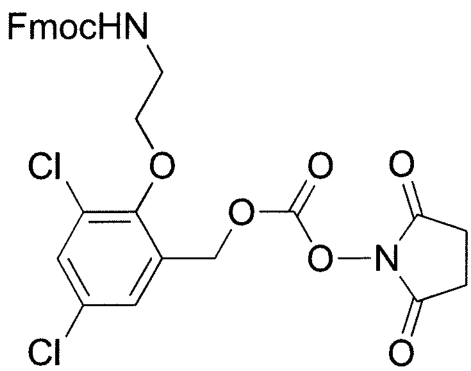

(3d)

[Chemical formula 17]

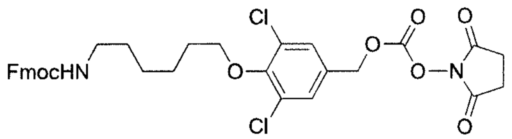

(3e)

In addition, the aldehyde type linker is preferably, for example, a compound represented by the following formula (4):

[Chemical formula 18]

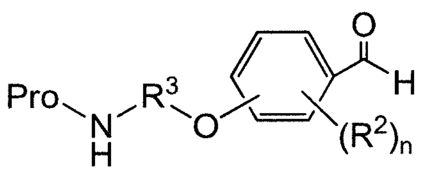

(4)

wherein $R^2$ is an electron-withdrawing group, n is an integer of 0 to 4, Pro is an amino-protecting group, and $R^3$ is an

alkylene group having 1 to 10 carbon atoms. $R^2$, n, Pro, and $R^3$ are preferably the same as those in formula (1). Among them, the most preferable aldehyde type linker is a compound represented by the following formula (5):

[Chemical formula 19]

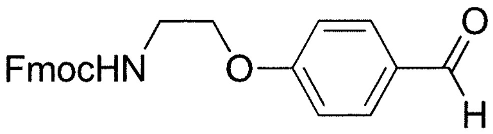

(5)

Furthermore, the halogen type linker is, for example, the following formula (6):

[Chemical formula 20]

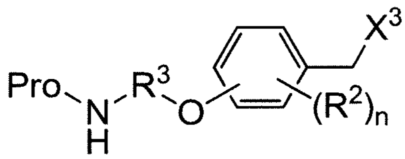

(6)

wherein $R^2$ is an electron-withdrawing group, n is an integer of 0 to 4, Pro is an amino-protecting group, $R^3$ is an alkylene group having 1 to 1 0 carbon atoms, and $X^3$ is a halogen atom.

The methods for producing various linkers according to the present invention are not particularly limited, but each of the methods for producing the carbonate type linker and the aldehyde type linker includes at least a step of carbonizing, oxidizing, or halogenating the hydroxymethyl group of a benzyl alcohol derivative represented by the following formula (7):

[Chemical formula 21]

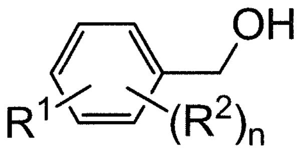

(7)

wherein $R^1$, $R^2$, and n are the same as described above.

The reaction of carbonizing the hydroxymethyl group of the benzyl alcohol derivative represented by the formula (7) is characterized by reacting, in the presence of a base, the benzyl alcohol derivative represented by the formula (7) and a compound represented by the following formula (8):

[Chemical formula 22]

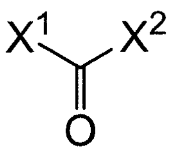

(8)

wherein $X^1$ and $X^2$ are each a leaving group.

In formula (8), the leaving groups that are $X^1$ and $X^2$ each represent, for example: a halogen atom such as a fluorine atom, a chlorine atom, a bromine atom, and an iodine atom; or an organic group represented by $-OR^x$ ... (9) ($R^x$ represents an aliphatic hydrocarbon group, an alicyclic hydrocarbon group, an aromatic hydrocarbon group, or a heterocyclic group, and these groups may each have a substituent).

The aliphatic hydrocarbon group preferably has 1 to 20 carbon ato ms, and specific examples of the aliphatic hydrocarbon group include: alkyl gro ups such as a methyl group, an ethyl group, an n-propyl group, an isopropyl g roup, an n-butyl group, an isobutyl group, an s-butyl group, a t-butyl group, a n n-pentyl group, an n-hexyl group, an n-octyl group, an n-decyl group, an n-d odecyl group, and an n-octadecyl group; alkenyl groups such as a vinyl group and an allyl group; and alkynyl groups such as an ethynyl group and a propa rgyl group, and the like.

In addition, the alicyclic hydrocarbon group preferably has 3 to 20 carbon atoms, and specific examples of the alicyclic hydrocarbon group include: cycloalkyl groups such as a cyclopropyl group, a cyclobutyl group, a cyclopent yl group, a cyclohexyl group, a cyclooctyl group, a norbornyl group, and an ad amanthyl group; and cycloalkylalkyl groups such as a cyclopentylmethyl group and a cyclohexylmethyl group; and the like.

The aromatic hydrocarbon group preferably has 6 to 20 carbon atoms, and specific examples of the aromatic hydrocarbon group include: aryl groups such as a phenyl group, a naphthyl group, and an indenyl group; and aralkyl groups such as a benzyl group and a 4-phenylbutyl group; and the like.

Furthermore, the heterocyclic group is obtained by substituting a part of the carbons forming the ring with an atom other than carbon, such as nitrogen, oxygen, and sulfur, in the alicyclic hydrocarbon group or aromatic hydrocarbon group, and preferably has 3 to 20 carbon atoms. The heterocyclic group is more preferably a nitrogen-containing heterocyclic imide group having at least one imide group in a nitrogen-containing heterocyclic group. Examples of such a nitrogen-containing heterocyclic imide group include saturated or unsaturated nitrogen-containing heterocyclic imide groups shown below.

[Chemical formula 23]

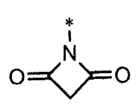
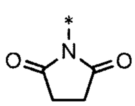
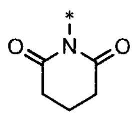
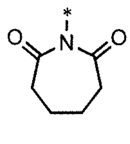
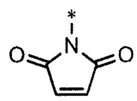
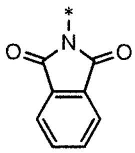

Particularly preferable examples of such a heterocyclic imide group include a succinimidyl group, a maleimidyl group, a phthalimidyl group, and the like.

The aliphatic hydrocarbon group, the alicyclic hydrocarbon group, the aromatic hydrocarbon group, or the heterocyclic group may have a substituent, and examples of the substituent include: alkyl groups; halogen atoms such as a fluorine atom, a chlorine atom, a bromine atom, and an iodine atom; an amino group; a hydroxy group; a nitro group; and the like.

Examples of such -$OR^x$ include N-succinimidyloxy group, an N-mal eimidyloxy group, an N-phthalimidyloxy group, a p-nitrophenyloxy group, a ben zyloxy group, and the like.

In formula (8), $X^1$ and $X^2$ may be the same or different from each other. Examples of preferable compounds (8) include carbonic acid derivatives (e.g., di-N-succinimidyl carbonate, etc.), chloroformate compounds (e.g., N-succin imidyl chloroformate, p-nitrophenyl chloroformate), and the like.

The use amount of the compound represented by the formula (8) and having a carbonate group in the carbonization reaction with respect to 1 mol of the benzyl alcohol derivative represented by the formula (7) is preferably not less than 0.5 times by mole, more preferably not less than 0.8 times by mole, and further preferably not less than 1 time by mole, and is preferably not greater than 3 times by mole, more preferably not greater than 2.5 times by mole, and further preferably not greater than 2 times by mole.

The base is not particularly limited, but examples of the base include: tertiary amines such as triethylamine, tri-n-butylamine, N-methylmorpholine, N-methylpiperidine, diisopropylethylamine, pyridine, N,N-dimethylaminopyridine, and 1,4-diazabicyclo[2, 2, 2]octane; metal hydroxides such as lithium hydroxide, sodium hydroxide, potassium hydroxide, barium hydroxide, and magnesium hydroxide; metal carbonates such as lithium carbonate, sodium carbonate, and potassium carbonate; metal hydrogen carbonates such as lithium hydrogen carbonate, sodium hydrogen carbonate, and potassium hydrogen carbonate; and metal alkoxides such as lithium methoxide, lithium ethoxide, sodium methoxide, sodium ethoxide, potassium methoxide, potassium ethoxide, and potassium tert-butoxide. Among them, tertiary amines are preferable, and pyridine or N,N-dimethylaminopyridine is more preferable. Only one of these bases may be used solely, or two or more of these bases may be used in combination.

The reaction of oxidizing the hydroxymethyl group of the benzyl alcohol derivative represented by the formula (7) is characterized by oxidizing the benzyl alcohol derivative represented by the formula (7), by applying an oxidizer.

The oxidizer is not particularly limited, but examples of the oxidizer include: chlorates such as potassium chlorate, sodium chlorate, ammonium chlorate, barium chlorate, and calcium chlorate; perchlorates such as potassium perchlorate, sodium perchlorate, and ammonium perchlorate; inorganic peroxides such as potassium peroxide, sodium peroxide, calcium peroxide, magnesium peroxide, and barium peroxide; chlorites such as potassium chlorite, sodium chlorite, and copper chlorite; bromates such as potassium bromate, sodium bromate, and magnesium bromate; nitrates such as ammonium nitrate, sodium nitrate, potassium nitrate, barium nitrate, and silver nitrate; iodates such as potassium iodate, sodium iodate, and calcium iodate; permanganates such as potassium permanganate and ammonium permanganate; dichromates such as sodium dichromate and ammonium dichromate; periodates such as sodium periodate and metaperiodic acid; manganese dioxide; chromates such as chromium trioxide, chromic anhydride, chromic acid, and dichromic acid; lead dioxide, sodium nitrite, potassium hypochlorite, sodium hypochlorite, potassium peroxydisulfate, sodium peroxydisulfate, ammonium peroxydisulfate, potassium perborate, ammonium perborate, ozone, perchloric acid, tungstic oxide, or hydrogen peroxide, etc. Among them, manganese dioxide is preferable, since manganese dioxide is easily available. Only one of these oxidizers may be used solely, or two or more of these oxidizers may be used in combination.

In the linker bonding step, it is necessary to adjust the reaction condition in accordance with the characteristics of each linker. Hereinafter, the reaction condition will be described individually for the carbonate type linker and the aldehyde type linker which are typically used in the present invention.

«Case of carbonate type linker»

The linker bonding step when the carbonate type linker is used will be described. When the carbonate type linker is used, the linker-bonded compound is formed by bonding the linker to the amino group of the first peptide chain by: mixing the carbonate type linker and the crude peptide; and forming a benzyloxycarbonylamino group.

The use amount of the carbonate type linker in this reaction with respect to 1 mol of the crude peptide is preferably not less than 0.5 times by mole, more preferably not less than 1 time by mole, and further preferably not less than 2 times by mole, and is preferably not greater than 20 times by mole and more preferably not greater than 10 times by mole.

The reaction between the carbonate type linker and the crude peptide is preferably carried out in the presence of a solvent. For example, organic solvents including: ether-based solvents such as diisopropyl ether, t-butyl methyl ether, dibutyl ether, dioxane, and diglyme; modified ethers such as propylene glycol monomethyl ether acetate; ester-based solvents such as ethyl acetate and butyl acetate; halogen-based solvents such as chloroform and dichloromethane; and the like, can be used as the reaction solvent. Moreover, aqueous solvents including: alcohols such as methanol and ethanol; ethers such as ethylene glycol, dimethoxyethane, and tetrahydrofuran; nitriles such as acetonitrile; amides such as dimethylformamide, N,N-dimethylacetamide, and N-methylpyrrolidone; and the like, can also be used as the reaction solvent. Only one of these reaction solvents may be used solely, or two or more of these reaction solvents may be used in combination. Among them, amides are preferable, and dimethylformamide is more preferable.

The use amount of the reaction solvent with respect to the crude peptide is preferably not greater than 50 times by weight and further preferably not greater than 20 times by weight, and the lower limit is not particularly limited, but the amount of the reaction solvent is preferably not less than 2 times by weight.

Furthermore, in this reaction, for the purpose of accelerating the reaction, a tertiary amine, such as triethylamine, tri-n-butylamine, N-methylmorpholine, N-methylpiperidine, diisopropylethylamine, pyridine, N,N-dimethylaminopyridine, and 1,4-diazabicyclo[2, 2, 2]octane, may be caused to coexist as a reaction accelerator.

The reaction temperature is not particularly limited as long as the reaction temperature is not higher than the boiling point of the solvent to be used. For example, the reaction temperature is preferably not lower than 0°C, more preferably not lower than 10°C, and further preferably not lower than 20°C, and is preferably not higher than 100°C and more preferably not higher than 80°C.

The reaction time is also not particularly limited, and is preferably not shorter than 1 hour and more preferably not shorter than 2 hours and is preferably not longer than 100 hours and more preferably not longer than 50 hours.

After end of the reaction, the obtained linker-bonded compound is preferably washed to remove unnecessary components. As a solvent for washing, various solvents described in detail in the section of the reaction solvent can be used.

«Case of aldehyde type linker»

The linker bonding step when the aldehyde type linker is used will be described. When the aldehyde type linker is used, the linker-bonded compound can be produced by: performing dehydration condensation of the aldehyde type

linker and the crude peptide to produce an imine resin in which the aldehyde type linker is bonded to the crude peptide via an imino group; applying a reducing agent to the obtained imine resin to reduce the imino group; and forming a benzylamino group to bond the linker to the amino group of the first peptide chain.

The dehydration condensation reaction between the aldehyde type linker and the crude peptide is carried out by mixing the aldehyde type linker and the crude peptide. In producing the imine resin, the use amount of the aldehyde type linker with respect to 1 mol of the crude peptide is preferably not less than 1 time by mole, more preferably not less than 3 times by mole, and further preferably not less than 5 times by mole, and is preferably not greater than 50 times by mole, more preferably not greater than 30 times by mole, and further preferably not greater than 20 times by mole.

The reaction between the aldehyde type linker and the crude peptide is preferably carried out in the presence of a solvent. For example, organic solvents including: aromatic hydrocarbon solvents, such as benzene, toluene, and xylene; ether-based solvents such as diisopropyl ether, t-butyl methyl ether, dibutyl ether, and diglyme; modified ethers such as propylene glycol monomethyl ether acetate; ester-based solvents such as ethyl acetate and butyl acetate; halogen-based solvents such as chloroform and dichloromethane; cyclic hydrocarbon solvents such as cyclohexane, methylcyclohexane, and ethylcyclohexane; chain hydrocarbon solvents such as pentane, hexane, heptane, octane, isooctane, and isododecane; and the like, can be used as the reaction solvent. Moreover, aqueous solvents including: alcohols such as methanol and ethanol; ethers such as ethylene glycol, dimethoxyethane, and tetrahydrofuran; nitriles such as acetonitrile; amides such as dimethylformamide, N,N-dimethylacetamide, and N-methylpyrrolidone; and the like, can also be used as the reaction solvent. Only one of these reaction solvents may be used solely, or two or more of these reaction solvents may be used in combination. Among them, halogen-based solvents are preferable, and dichloromethane is more preferable.

The use amount of the reaction solvent with respect to the crude peptide is preferably not greater than 100 times by weight and further preferably not greater than 50 times by weight, and the lower limit is not particularly limited, but the amount of the reaction solvent is preferably not less than 2 times by weight.

In addition, in the dehydration condensation reaction, a dehydrating agent is preferably used as appropriate. Preferable examples of the dehydrating agent include: ortho acid esters typified by orthoformates such as trimethyl orthoformate, triethyl orthoformate, and tripropyl orthoformate, orthoacetates such as trimethyl orthoacetate, triethyl orthoacetate, and tripropyl orthoacetate, orthopropionates such as trimethyl orthopropionate, triethyl orthopropionate, and tripropyl orthopropionate, and the like; acetals typified by dimethoxymethane, 1,1-dimethoxyethane, 1,1-dimethoxypropane, diethoxyethane, 1,1-diethoxyethane, 1,1-diethoxypropane, and the like; and hemiacetals typified by 1-methoxy-1-ethanol, 1-methoxy-1-propanol, 1-methoxy-1-butanol, 1-ethoxy-1-ethanol, 1-ethoxy-1-propanol, 1-ethoxy-1-butanol, and the like. Among them, orthoformates are preferable, and trimethyl orthoformate is more preferable.

When the dehydrating agent is used, the use amount of the dehydrating agent with respect to the crude peptide is preferably not greater than 100 times by weight and further preferably not greater than 50 times by weight, and the lower limit is not particularly limited, but the amount of the dehydrating agent is preferably not less than 1 time by weight.

The reaction temperature is not particularly limited as long as the reaction temperature is not higher than the boiling point of the solvent to be used. For example, the reaction temperature is preferably not lower than 0°C, more preferably not lower than 10°C, and further preferably not lower than 20°C, and is preferably not higher than 100°C, more preferably not higher than 80°C, and further preferably not higher than 60°C.

The reaction time is also not particularly limited, and is preferably not shorter than 1 hour and more preferably not shorter than 12 hours and is preferably not longer than 20 days and more preferably not longer than 15 days.

After end of the reaction, the obtained linker-bonded compound is preferably washed to remove unnecessary components. As a solvent for washing, various solvents described in detail in the section of the reaction solvent can be used.

Examples of the reducing agent in the reduction reaction of the imine resin include: aluminium hydrides such as lithium aluminium hydride, diisobutylaluminium hydride, and sodium bis(2-methoxyethoxy)aluminium hydride; borohydride compounds such as alkali metal borohydrides, including lithium borohydride, sodium borohydride, and potassium borohydride, calcium borohydride, sodium cyanoborohydride, sodium triacetoxyborohydride, lithium triethylborohydride, lithium tri(sec-butyl)borohydride, and potassium tri(sec-butyl)borohydride; tributyltin hydroxide; and borane, etc. Among them, from the standpoint of chemoselectivity, borohydride compounds are preferable, and sodium cyanoborohydride is more preferable.

The use amount of the reducing agent with respect to 1 part by mass of the imine resin is preferably not less than 0.1 parts by mass, more preferably not less than 0.3 parts by mass, and further preferably not less than 0.6 parts by mass, and is preferably not greater than 3 parts by mass, more preferably not greater than 2.5 parts by mass, and further preferably not greater than 2 parts by mass.

The reduction reaction of the imine resin is preferably carried out in the presence of a solvent. For example, organic solvents including: aromatic hydrocarbon solvents, such as benzene, toluene, and xylene; ether-based solvents such as diisopropyl ether, t-butyl methyl ether, dibutyl ether, and diglyme; modified ethers such as propylene glycol monomethyl ether acetate; ester-based solvents such as ethyl acetate and butyl acetate; carboxylic acid-based solvents

such as acetic acid and propionic acid; halogen-based solvents such as chloroform and dichloromethane; cyclic hydrocarbon solvents such as cyclohexane, methylcyclohexane, and ethylcyclohexane; chain hydrocarbon solvents such as pentane, hexane, heptane, octane, isooctane, and isododecane; and the like, can be used as the reaction solvent. Moreover, aqueous solvents including: alcohols such as methanol and ethanol; ethers such as ethylene glycol, dimethoxyethane, and tetrahydrofuran; nitriles such as acetonitrile; amides such as dimethylformamide, N,N-dimethylacetamide, and N-methylpyrrolidone; and the like, can also be used as the reaction solvent. Only one of these reaction solvents may be used solely, or two or more of these reaction solvents may be used in combination. When an alcohol or a carboxylic acid-based solvent is used, an increase in the reaction speed can also be expected. In the present invention, among them, alcohol-based solvents, carboxylic acid-based solvents, and amides are preferable.

The use amount of the reaction solvent with respect to the imine resin is preferably not greater than 100 times by weight and further preferably not greater than 50 times by weight, and the lower limit is not particularly limited, but the amount of the reaction solvent is preferably not less than 5 times by weight.

The reaction temperature is not particularly limited as long as the reaction temperature is not higher than the boiling point of the solvent to be used. For example, the reaction temperature is preferably not lower than 0°C, more preferably not lower than 10°C, and further preferably not lower than 20°C, and is preferably not higher than 100°C, more preferably not higher than 80°C, and further preferably not higher than 60°C.

The reaction time is also not particularly limited, and is preferably not shorter than 1 hour and more preferably not shorter than 10 hours and is preferably not longer than 50 hours and more preferably not longer than 30 hours.

After end of the reaction, the obtained linker-bonded compound is preferably washed to remove unnecessary components. As a solvent for washing, various solvents described in detail in the section of the reaction solvent can be used.

In the obtained linker-bonded compound, the linker and the crude peptide are bonded to each other via $-CH_2-NH-$. However, the hydrogen atom in NH- has high reactivity and may have an effect on a subsequent reaction. Thus, an N-protection reaction of the linker-bonded compound after the reduction reaction (referred to as a reduced form resin) is preferably carried out as necessary. Examples of a protecting group for an amino group include carbamate-based groups, imide-based groups, sulfonamide-based groups, and the like. Among them, carbamate-based protecting groups, such as a tert-butoxycarbonyl group (-Boc), a benzyloxycarbonyl group (-Z), a 9-fluorenylmethyloxycarbonyl group (-Fmoc), a 2,2,2-trichloroethoxycarbonyl group (-Troc), and an allyloxycarbonyl group (-Alloc), are preferable. When the amino-protecting group in the aldehyde type linker is Fmoc, for example, a tert-butoxycarbonyl group (-Boc) is preferably selected in order that influence of the deprotection condition for the Fmoc is prevented.

Introduction of the protecting group can be carried out under a basic condition by adding a protecting agent corresponding to each protecting group, to the reaction solution after the reduction reaction. Examples of the protecting agent include carbamating agents including: chlorides such as methoxycarbonyl chloride, ethoxycarbonyl chloride, isopropoxycarbonyl chloride, allyloxycarbonyl chloride, benzyloxycarbonyl chloride, and phenyloxycarbonyl chloride; or anhydrides such as di-tert-butyldicarbonate, etc.

The use amount of the protecting agent with respect to 1 mol of the reduced form resin is preferably 1 to 30 times by mole and more preferably 10 to 25 times by mole.

In addition, in the protection, a base may be further added for the purpose of neutralizing an acid, produced as a byproduct, to accelerate the reaction. Examples of the base include: metal carbonates such as lithium carbonate, sodium carbonate, potassium carbonate, magnesium carbonate, and calcium carbonate; metal hydrogen carbonates such as lithium hydrogen carbonate, sodium hydrogen carbonate, potassium hydrogen carbonate, and cesium hydrogen carbonate; metal hydroxides such as lithium hydroxide, sodium hydroxide, and potassium hydroxide; and tertiary amines such as triethylamine, N-methylmorpholine, ethyl diisopropylamine, and pyridine. Tertiary amines are preferable, and N-methylmorpholine is further preferable.

The use amount of the base with respect to 1 mol of the reduced form resin is preferably 0.5 to 20 times by mole and more preferably 3 to 15 times by mole.

The N-protection reaction may be carried out in the presence of a solvent. The reaction solvent is not particularly limited as long as the reaction solvent does not have an effect on the reaction. As the reaction solvent, for example: water; ether-based solvents such as tetrahydrofuran, diethyl ether, 1,4-dioxane, and ethylene glycol dimethyl ether; aromatic hydrocarbon-based solvents such as benzene and toluene; aliphatic hydrocarbon-based solvents such as pentane, hexane, heptane, and methylcyclohexane; halogen-based solvents such as carbon tetrachloride, chloroform, methylene chloride, 1,2-dichloroethane, and chlorobenzene; ester-based solvents such as ethyl acetate, isopropyl acetate, and tert-butyl acetate; sulfoxide-based solvents such as dimethyl sulfoxide; amide-based solvents such as N,N-dimethylformamide and N,N-dimethylacetamide; urea-based solvents such as dimethylpropyleneurea; phosphonic triamide-based solvents such as hexamethylphosphonic triamide; ketone-based solvents such as acetone and methyl ethyl ketone; and nitrile-based solvents such as acetonitrile and propionitrile, can be used. Preferable examples of the reaction solvent include: water; aromatic hydrocarbon-based solvents such as benzene and toluene; ether-based solvents such as tetrahydrofuran, 1,4-dioxane, and methyl tert-butyl ether; ester-based solvents such as ethyl acetate,

isopropyl acetate, and tert-butyl acetate; halogen-based solvents such as carbon tetrachloride, chloroform, methylene chloride, 1,2-dichloroethane, and chlorobenzene; and amide-based solvents such as N,N-dimethylformamide and N,N-dimethylacetamide, and the reaction solvent is further preferably DMF. These solvents may be used solely, or two or more of these solvents may be used in combination.

An excessive amount of the solvent is not preferable in terms of cost and post-treatment, and thus the use amount of the solvent with respect to the reduced form resin is preferably not greater than 50 times by weight and more preferably not greater than 30 times by weight.

The reaction temperature is not particularly limited as long as the reaction temperature is not higher than the boiling point of the solvent to be used. For the purpose of being able to terminate the reaction in a short time and inhibiting a side reaction, the reaction temperature is preferably not lower than - 10°C and more preferably not lower than 0°C, and is preferably not higher than 90°C and more preferably not higher than 50°C.

The reaction time is also not particularly limited, and is preferably not shorter than 1 hour and more preferably not shorter than 3 hours and is preferably not longer than 100 hours and more preferably not longer than 50 hours.

<Hydrophilic unit bonding step A1b>

In the hydrophilic unit bonding step, the hydrophilic unit is bonded to the linker-bonded compound obtained as described above. The "hydrophilic unit" refers to a unit that can be bonded to the linker moiety of the linker-bonded compound (the bonded compound of the first peptide and the linker) and that can impart hydrophilicity to the linker-bonded compound, and, for example, the hydrophilic unit itself has hydrophilicity with a log P (P is an octanol-water partition coefficient) of not higher than -1. As a result of bonding such a hydrophilic unit to the first peptide chain via the linker, the first peptide chain can be hydrophilized when the support is cleaved and removed, so that chromatographic purification of the first peptide chain is enabled. In the present specification, a product obtained by this hydrophilic unit bonding step, that is, a connected compound including three components, the first peptide, the linker, and the hydrophilic unit, is referred to as a "hydrophilized peptide", such a compound to which the support is bonded is particularly referred to as a "supported hydrophilized peptide", and such a compound from which the support is removed is particularly referred to as a "support-free hydrophilized peptide".

A log P value is a parameter of the hydrophobicity of a compound, and a partition coefficient P in a typical octanol-water system is obtained as follows. First, a compound (the hydrophilic unit in the present invention) is dissolved in octanol (or water), water (or octanol) is added thereto in the same amount, and the solution is shaken for 30 minutes by a Griffin flask shaker manufactured by Griffin & George Ltd. Thereafter, centrifugal separation is performed at 2000 rpm for 1 to 2 hours, each of the concentrations of the compound in the octanol layer and the water layer is measured spectroscopically or by various methods such as GLC, and the partition coefficient P is obtained by the following formula.

$$P = Coct/Cw$$

Coct: the concentration of the compound in the octanol layer
Cw: the concentration of the compound in the water layer

The log P values of various compounds have been actually measured by many researchers so far, and these actual measured values have been arranged by C. Hansch et al. (see PARTITION COEFFICIENTS AND THEIR USES; Chemical Reviews, vol. 71, P525, 1971).

The log P value may be obtained by the above method, but, in the present invention, a value calculated at 25°C by ACD/Log P DB Release 100 Product Version 10.01 manufactured by Advanced Chemistry Development, Inc., is used in principle.

As a result of considering compounds having various log P values when an effective structure is searched for as the hydrophilic unit, it has been found that if the log P value of a hydrophilizing unit is, for example, not higher than -1, the hydrophilizing unit is effective for hydrophilizing the crude peptide. Further, if the log P value exceeds -1, sufficient hydrophilicity is not exerted, so that the purification efficiency of the support-free hydrophilized peptide may decrease. The log P value is more preferably not higher than -1.2 and further preferably not higher than -1.5. In addition, the lower limit of the log P is not particularly limited, but the log P value is, for example, preferably not less than -10.

Examples of the hydrophilic unit having such a log P value include a hydrophilic peptide chain, a hydrophilic polyether, or a hydrophilic polyamine, etc. The hydrophilic peptide chain may be prepared by sequentially connecting amino acids to the linker-bonded compound. In this case, the hydrophilic peptide chain is desirably prepared by Fmoc solid-phase peptide synthesis. In addition, regarding the hydrophilic peptide chain, a previously prepared hydrophilic peptide chain may separately be connected to the linker-bonded compound.

Therefore, in a preferred embodiment of the hydrophilic unit bonding step, the hydrophilic unit is preferably bonded to the linker moiety by the following steps:

(1) bonding a plurality of amino acids to the linker moiety stepwise by Fmoc solid-phase peptide synthesis;
(2) bonding a previously prepared hydrophilic peptide chain to the linker moiety;
(3) bonding a previously prepared polyether to the linker moiety; and
(4) bonding a previously prepared polyamine to the linker moiety. Hereinafter, each of the above steps will be described.

(1) Formation of hydrophilized peptide by Fmoc solid-phase peptide synthesis.

In this method, the hydrophilic peptide is obtained by sequentially forming a peptide at the amino group of the linker-bonded compound obtained by the above step, by Fmoc solid-phase peptide synthesis. When a hydrophilic peptide is sequentially formed as in this method, the properties (e.g., hydrophilicity, etc.) of a hydrophilized peptide formed each time one cycle ends can be verified, so that no extra agent is consumed and thus this method is preferable. The specific procedure of the Fmoc solid-phase peptide synthesis is not particularly limited, and may be, for example, the same as that in the case of the above-described step of producing the supported crude peptide. In any case, a cycle, in which deprotection of Fmoc is performed in the presence of a base and dehydration condensation of an amino acid or a peptide is performed to extend a main chain, is preferably continued until a desired hydrophilic peptide chain is obtained.

The amino acids forming the hydrophilic peptide are not particularly limited, and various amino acids described in detail in the section of the first peptide chain can be used. For the hydrophilic peptide, different types of amino acids among these amino acids may be used in combination, or the same type of amino acids may be used solely. In the present invention, in order to increase the hydrophilicity of the hydrophilic peptide, the hydrophilic peptide preferably has, for example, a unit derived from an amino acid having a basic side chain. Specifically, the hydrophilic peptide chain has preferably two or more residues, more preferably 4 or more residues, among all amino acid residues, as at least one or more types of amino acid residues selected from arginine, asparagine, glutamine, histidine, and lysine. The upper limit is not particularly limited, but the number of such amino acid residues is preferably not greater than 35, more preferably not greater than 20, and further preferably not greater than 10. For the same reason, the hydrophilic peptide preferably has, for example, a residue derived from an amino acid having an acidic side chain. Specifically, the hydrophilic peptide chain has desirably two or more residues, more preferably 4 or more residues, among all amino acid residues, as at least one or more types of amino acid residues selected from aspartic acid and glutamic acid. The upper limit is not particularly limited, but the number of such amino acid residues is preferably not greater than 35, more preferably not greater than 20, and further preferably not greater than 10.

The number of the amino acid residues of the hydrophilic peptide chain is not particularly limited, but is, for example, not less than 2, more preferably not less than 3, and further preferably not less than 4, is preferably not greater than 35, may be not greater than 20, and may be not greater than 10. If the number of the amino acid residues forming the hydrophilic peptide increases, the contribution ratio of the hydrophilic peptide chain to hydrophilization in the bonded compound of the linker-bonded compound and the hydrophilic peptide chain (the hydrophilized peptide) increases, so that it becomes easy to control the characteristics of the hydrophilized peptide.

(2) Formation of hydrophilized peptide by previously prepared hydrophilic peptide chain.

Before the hydrophilic peptide chain is bonded to the linker-bonded compound, a desired hydrophilic peptide chain may be produced in advance by liquid-phase peptide synthesis or solid-phase peptide synthesis, and the obtained hydrophilic peptide chain may be bonded to the linker.

(3) Formation of hydrophilized peptide by previously prepared polyether.

The hydrophilic unit is not limited to the polypeptide, and may be a polyether or a polyamine. The hydrophilic polyether is not particularly limited, and is, for example, a compound having repeating units represented by $-R^y-O-$, in a main chain backbone thereof. Examples of $R^y$ include $-CH_2CH_2-$, $-CH(CH_3)CH_2-$, $-CH(C_2H_5)CH_2-$, $-C(CH_3)_2CH_2-$, $-(CH_2)_4-$, and the like. Among them, $-CH_2CH_2-$ and $-CH(CH_3)CH_2-$ are preferable. The hydrophilic polyether may be a homopolymer having one type of repeating units among these repeating units, or may be a copolymer.

The number of the repeating units represented by $-R^y-O-$ is, for example, preferably 1 to 200 and more preferably 50 to 150.

The hydrophilic polyether contains preferably not less than 50% by weight, further preferably not less than 80% by weight, and more preferably not less than 95% by weight of the repeating units represented by $-R^y-O-$ in the hydrophilic polyether.

The polystyrene-equivalent number average molecular weight of the hydrophilic polyether obtained by gel permeation chromatography (GPC) is, for example, preferably about 1000 to 100,000.

(4) Formation of hydrophilized peptide by previously prepared polyamine.

The polyamine is not particularly limited, examples of the polyamine include an aliphatic hydrocarbon to which at least two or more amino groups are bonded, and the aliphatic hydrocarbon may be saturated (chain or cyclic) or unsaturated. Each of the hydrogen atoms bonded to the nitrogen atoms of the polyamine may be substituted or unsubstituted, but is preferably unsubstituted, in order to increase the hydrophilicity.

Specific examples of such a polyamine include chain aliphatic polyamines such as diethylenetriamine, N-(2-aminoethyl)-1,3-propanediamine, N-(3-aminopropyl)-1,3-propanediamine, spermidine, bis(hexamethylene)triamine, 4-(aminomethyl)-1,8-octanediamine, triethylenetetramine, 1,4,7,11-tetraazaundecane, N,N'-bis(3-aminopropyl)ethylenediamine, N,N'-bis(2-aminoethyl)-1,3-propanediamine, N,N'-bis(3-aminopropyl)-1,3-propanediamine, spermine, tris(2-aminoethyl)amine, tetraethylenepentamine, and pentaethylenehexamine; and cyclic aliphatic polyamines such as 1,4,7-triazacyclononane, 1,5,9-triazacyclododecane, cyclen, 1,4,8,11-tetraazacyclotetradecane, 1,4,8,12-tetraazacyclopentadecane, hexacyclen, 3,3'-diaminobenzidine, and 1,2,4,5-benzenetetramine.

<Hydrophilic unit-linker complex bonding step A2>

In order to bond the linker and the hydrophilic unit to the crude peptide to obtain a hydrophilized peptide, a hydrophilic unit-linker complex in which the hydrophilic unit and the linker are previously bonded as a single compound may be introduced to the amino group of the crude peptide. By previously preparing the complex, the process for producing the hydrophilic peptide from the crude peptide can be shortened, so that the efficiency of the entire process can be increased.

As the hydrophilic unit and the linker in the complex, the same ones as those in the linker bonding step A1a or the hydrophilic unit bonding step A1b can be used. As the method for preparing the complex, the same method as that in the hydrophilic unit bonding step A1b can be adopted. In the case of bonding the complex to the crude peptide, the same method as that in the linker bonding step A1a can be adopted.

After the hydrophilic unit-linker complex is bonded to the crude peptide, the amino acid at the hydrophilic unit side may be further extended as necessary. For example, limited types as the hydrophilic unit-linker complex are prepared as basic units, the basic unit is bonded to the crude peptide, and then, for example, the amino acid is extended in accordance with the characteristics of the crude peptide. This method allows the number of the types of the hydrophilic unit-linker complexes prepared as the basic units to be reduced, and thus is easy.

In addition, A2-II is also preferably carried out by the same method as that for A2-I.

3. Support cleavage step

The support cleavage step refers to a step of separating the support and the first peptide chain from each other. While the linker and the hydrophilic unit are bonded to the first peptide chain, the first peptide chain can be hydrophilized by cleavage the support. The first peptide chain to which the linker and the hydrophilic unit are bonded and from which the support is cleaved is referred to as a support-free hydrophilized peptide in the present specification.

The support cleavage step may be carried at any stage before the chromatographic purification step described later. However, the bond between the first peptide chain and the support is preferably cleaved at any stage before bonding the linker to the supported crude peptide until the hydrophilized peptide is obtained, or after the supported hydrophilized peptide is obtained. Specifically, the support may be cleaved from a supported crude peptide including the first peptide chain and the support (see the support cleavage step B1 in Scheme I), the support may be cleaved from a supported linker-bonded compound in which the linker is bonded to the supported crude peptide (see the support cleavage step B2 in Scheme I), or the support may be cleaved from a supported hydrophilized peptide in which the linker and the hydrophilic unit are bonded to the supported crude peptide (see the support cleavage step B3 in Scheme I). Even when the support is cleaved from the supported crude peptide (the support cleavage step B1), a support-free linker-bonded compound can be obtained from the obtained support-free crude peptide by carrying out the linker bonding step A1a similarly to the above (A1c in Scheme I). In addition, a support-free hydrophilized peptide can be obtained from the support-free crude peptide by carrying out the hydrophilic unit-linker complex bonding step A2. Moreover, a support-free hydrophilized peptide can be obtained, from a support-free linker-bonded compound obtained by carrying out the support cleavage step B2 or treating the support-free crude peptide in the linker bonding step A1a, by carrying out the hydrophilic unit bonding step A1b similarly to the above (A1d in Scheme I). In the present invention, after the hydrophilized peptide is obtained by the hydrophilization step, the support cleavage step (i.e., the support cleavage step B3 in Scheme I) is preferably carried out.

In these support cleavage steps B1, B2, and B3, an amide bond or ester bond connecting the first peptide chain and the support is hydrolyzed. In cleaving the first peptide chain from the support, an operation that is widely used in solid-phase peptide synthesis is preferably carried out. For example, in the case where the first peptide chain or the hydrophilic unit is formed by Fmoc solid-phase peptide synthesis, the first peptide chain is preferably cleaved from the support by treatment with a cleaving reagent.

As the cleaving reagent, a reagent that is used under a general condition for cleaving from the support can be used. When the cleaving reagent is used as an aqueous solution, the concentration of the cleaving reagent in 100% of this reagent, as a volume ratio, is preferably not less than 40%, more preferably not less than 50% and is preferably not greater than 100%, but there is no problem if the concentration is not greater than 97%.

In addition, the cleaving reagent may contain TIS (triisopropylsilane). The concentration of TIS in 100% of the reagent, as a volume ratio, is preferably not less than 1% and more preferably not less than 1.5%, and is preferably not greater than 20% and more preferably not greater than 10%.

The cleavage step may be carried out in the absence of a solvent (a solvent mixed from the above reagent is permitted), or may be carried out in the presence of a solvent as necessary. Examples of the solvent include water, an organic solvent, an aqueous solvent, etc. Examples of the organic solvent include halogen-based solvents such as carbon tetrachloride, chloroform, methylene chloride, 1,2-dichloroethane, chlorobenzene, etc. Examples of the aqueous solvent include: alcohols such as methanol and ethanol; ethers such as ethylene glycol, dimethoxyethane, and tetrahydrofuran; ketones such as acetone and dioxane; nitriles such as acetonitrile; amides such as dimethylformamide, N,N-dimethylacetamide, and N-methylpyrrolidone; and the like. These solvents may be used solely, or two or more of these solvents may be used in combination.

The treatment temperature is not particularly limited as long as the treatment temperature is not higher than the boiling point of the solvent to be used. For example, the treatment temperature is preferably not lower than 0°C and more preferably not lower than 10°C, and is preferably not higher than 100°C, more preferably not higher than 80°C, and further preferably not higher than 60°C.

In addition, the treatment time is, for example, preferably not shorter than 0.5 hour, more preferably not shorter than 1 hour, and further preferably not shorter than 1.5 hours, and is preferably not longer than 20 hours and more preferably not longer than 10 hours.

As post-treatment after the reaction, general treatment for obtaining a product from a reaction solution is preferably performed. Specifically, the cleaved support is separated by filtration or the like, and concentration and washing are performed as necessary. Then, the purity may be further increased by a general purification method such as an operation of adding an organic solvent to deposit and obtain a solid.

The protecting group at the side chain of the first peptide may be deprotected or may not be deprotected, by treatment in the support cleavage step. If the protecting group bonded to the side chain of the first peptide can be deprotected by the treatment in the support cleavage step, a deprotection step for the side chain can be omitted, and thus the method is easy. In addition, in the case where the side chain of the first peptide is not deprotected in the support cleavage step, deprotection of the side chain of the first peptide chain may be separately carried out. The protecting group bonded to the side chain of the first peptide chain may be deprotected at any stage before or after the hydrophilic unit is introduced. In addition, deprotection of the side chain can be carried out by a general method, depending on the protecting group.

4. Chromatographic purification step

The first peptide chain (support-free hydrophilized peptide) to which the linker and the hydrophilic unit are bonded and from which the support is cleaved as described above has hydrophilicity, and thus chromatographic purification can be performed thereon. Thus, in the chromatographic purification step, the support-free hydrophilized peptide obtained by the hydrophilization step and the support cleavage step is treated by liquid chromatography. At this time, the hydrophilized peptide is preferably dissolved in water beforehand.

A column and an eluent to be used in the chromatographic purification can be selected as appropriate in accordance with the type of the support-free hydrophilized peptide to be purified and impurities to be separated. The filler of the column is not particularly limited, and a wide range of publicly known fillers can be used, but typical examples of the filler preferably include silica gel, octadecyl group-bonded silica gel, octyl group-bonded silica gel, butyl group-bonded silica gel, trimethylsilyl group-bonded silica gel, Florisil, alumina, zirconia, hydroxyapatite, a styrene-vinylbenzene copolymer, polymethacrylate, polyhydroxymethacrylate, polyvinyl alcohol, an ion-exchange resin, activated carbon, etc. Columns packed with these fillers may be used solely, or two or more of these columns may be used in combination.

In addition, the eluent can be prepared as appropriate in accordance with the column to be used, the object to be purified, and impurities to be removed. For example, a mixed solution obtained by combining water and a hydrophilic organic solvent (e.g., alcohols such as methanol and ethanol; ethers such as ethylene glycol, dimethoxyethane, and tetrahydrofuran; ketones such as acetone and dioxane; nitriles such as acetonitrile; amides such as dimethylformamide,

N,N-dimethylacetamide, N-methylpyrrolidone; and the like) as appropriate, is used. An appropriate acid (e.g., TFA, formic acid, acetic acid, etc.) may be dissolved in this mixed solution.

The chromatographic purification step may be carried out two or more times as necessary, and the column (filler) and/or the eluent may be changed every time.

5. Linker cleavage step

In the linker cleavage step, the bond between the linker and the first peptide chain included in the chromatographically-purified support-free hydrophilized peptide is cleaved by chemical treatment. Examples of the chemical treatment include treatment by an acid or catalytic reduction, and the like.

< Cleavage of linker by catalytic reduction reaction>

Cleavage of the linker by a catalytic reduction reaction is preferably carried out in the presence of a solvent. Examples of the solvent include: alcohol-based solvents such as methanol, ethanol, n-propanol, isopropanol, n-butanol, 2-butanol, and n-pentanol; carboxylic acid-based solvents such as acetic acid, formic acid, and propionic acid; ethers such as tetrahydrofuran, diethyl ether, and dioxane, ester-based solvents such as methyl acetate, ethyl acetate, n-propyl acetate, isopropyl acetate, n-butyl acetate, sec-butyl acetate, isobutyl acetate, and tert-butyl acetate; halogenated hydrocarbons such as dichloromethane, chloroform, and carbon tetrachloride; aromatic hydrocarbons such as toluene; ketone-based solvents such as acetone, 2-butanone, 3-methyl-2-butanone, 2-pentanone, 4-methyl-2-pentanone, and 2-hexanone; amides such as N,N-dimethylformamide, N,N-dimethylacetamide, N-methylpyrrolidone, N-ethylpyrrolidone; and nitriles such as acetonitrile. Only one of these solvents may be used solely, or two or more of these solvents may be used in combination. Among them, an alcohol-based solvent having 3 to 8 carbon atoms is preferably contained in an amount of 10 to 100% by weight and more preferably 30 to 100% by weight in 100% by weight of the solvent.

The use amount of the solvent to be used in the catalytic reduction reaction is not particularly limited, but, for example, the use amount of the solvent as a weight ratio with respect to the hydrophilized peptide is preferably not less than 2 times and more preferably not less than 5 times, and is preferably not greater than 500 times and more preferably not greater than 400 times.

As a hydrogen source for the catalytic reduction reaction, for example, hydrogen gas, formic acid, or a salt thereof can be used, but hydrogen gas is preferably used from the standpoint of cost efficiency. In the case of using hydrogen gas, normally, the hydrogen pressure is preferably adjusted in the range of the atmospheric pressure ($1.013\times10^5$ Pa) to 0.5 MPa.

As a catalyst for the catalytic reduction reaction, for example, catalysts containing platinum, palladium, and the like can be used. Among them, catalysts containing palladium are preferable, and metal supported catalysts such as a palladium catalyst supported on activated carbon are particularly preferable.

The catalytic reduction reaction is preferably carried out by reacting a mixture of the hydrophilized peptide and the catalyst in the presence of the solvent and the hydrogen source. The reaction temperature is not particularly limited as long as the reaction temperature is not higher than the boiling point of the solvent to be used. For example, the reaction temperature is preferably not lower than 0°C, more preferably not lower than 15°C, and further preferably not lower than 30°C, and is preferably not higher than 70°C and more preferably not higher than 60°C. In addition, the reaction time is not limited, but is, for example, preferably not shorter than 1 hour, more preferably not shorter than 3 hours, and further preferably not shorter than 5 hours, and is preferably not longer than 50 hours and more preferably not longer than 30 hours.

After end of the catalytic reduction reaction, the first peptide chain from which the linker is removed can be isolated and purified by post-treatment that is normally performed, for example, operations such as concentration, dissolution in an organic solvent, filtration, washing, and deposition.

<Cleavage of linker by acid>

Examples of the acid to be used in this step include: halogenated carboxylic acids such as TFA (trifluoroacetic acid) and TCA (trichloroacetic acid); mineral acids such as hydrobromic acid, hydrochloric acid, and sulfuric acid; sulfonic acids such as p-toluenesulfonic acid, methanesulfonic acid, trifluoromethanesulfonic acid, and fluorosulfonic acid, etc. An acid having pKa of not higher than 0 is preferable, an acid having pKa of not higher than -2 is further preferable, and the lower limit is not particularly limited, but the pKa is generally not lower than -20 and preferably not lower than -18. Only one of these acids may be used solely, or two or more of these acids may be used in combination. The use amount of the acid with respect to the hydrophilized peptide, as a weight ratio, is preferably not less than 10 times and more preferably not less than 20 times, and is preferably not greater than 100 times and more preferably not greater than 80 times.

The reaction for cleaving the linker by the acid may be carried out in the presence of a solvent. Examples of the solvent include carboxylic acid-based solvents such as acetic acid and propionic acid, and water, etc. Only one of these solvents may be used solely, or two or more of these solvents may be used in combination. The use amount of the solvent with respect to the hydrophilized peptide, as a weight ratio, is preferably not less than 1 time and more preferably not less than 2 times, and is preferably not greater than 100 times and more preferably not greater than 50 times.

As such a solvent, a solvent containing TFA as an essential component is preferable, and a mixed solution containing both TFA and a mineral acid, which is not TFA, or a mixed solution containing TFA and a Lewis acid is more preferable. As the mixed solution containing both TFA and a mineral acid, which is not TFA, for example, a mixed solution containing HBr (its concentration is preferably 15 to 40%), acetic acid, thioanisole, and TFA is preferable. In addition, examples of the mixed solution containing TFA and a Lewis acid include a mixed solution containing TFA, trimethylsilyl trifluoromethanesulfonate (TMSOTf), and thioanisole, and a mixed solution containing TFA, TMSOTf, and meta-cresol, etc. From the standpoint of practicality, the mixed solution containing TFA and a Lewis acid is more preferably used.

Furthermore, in the reaction for cleaving the linker by the acid, a mixed solution containing the acid and, as a reaction accelerator, a Lewis acid such as trimethylsilyl trifluoromethanesulfonate (TMSOTf), trimethylsilyl bromide (TMSBr), trimethylsilyl iodide (TMSI), a $BF_3$-diethyl ether complex, and $Cp_2HfCl_2$-$AgClO_4$ can also be used. The use amount of the reaction accelerator with respect to the hydrophilized peptide, as a weight ratio, is preferably not less than 1 time and more preferably not less than 5 times, and is preferably not greater than 100 times and more preferably not greater than 50 times.

In the reaction for cleaving the linker by the acid, an additive may be added for the purpose of accelerating the reaction. Examples of the additive include phenol, m-cresol, p-cresol, anisole, thioanisole, diphenyl sulfide, and dimethyl sulfide. Only one of these additives may be used, or two or more of these additives may be used in combination.

The reaction temperature is not particularly limited as long as the reaction temperature is not higher than the boiling point of the solvent to be used. For example, the reaction temperature is preferably not lower than -35°C, more preferably not lower than -20°C, and further preferably not lower than -10°C, and is preferably not higher than 100°C, more preferably not higher than 80°C, and further preferably not higher than 60°C.

The reaction time is also not particularly limited, and is preferably not shorter than 0.1 hour, more preferably not shorter than 1 hour, and further preferably not shorter than 2 hours, and is preferably not longer than 50 hours, more preferably not longer than 30 hours, and further preferably not longer than 20 hours.

After end of the reaction by the acid, the first peptide chain from which the linker is removed can be isolated and purified by performing post-treatment that is normally performed, for example, an operation of adding a general organic solvent to deposit and obtain the peptide, etc.

6. Washing step

In order to further purify the hydrophobic peptide obtained by the above step, a washing step of removing the bonded compound of the hydrophilic unit and the linker by washing may be carried out. In this step, the support-free and linker-free first peptide chain obtained by the linker cleavage step is washed with water or a water-containing solvent which may contain an aid.

The water-containing solvent is, for example, a mixture of water and a hydrophilic solvent, and examples of the hydrophilic solvent include: aqueous solvents including alcohols such as methanol and ethanol; ethers such as ethylene glycol, dimethoxyethane, and tetrahydrofuran; ketones such as acetone and dioxane; and nitriles such as acetonitrile; amides such as dimethylformamide, N,N-dimethylacetamide, and N-methylpyrrolidone; and the like. The water or the water-containing solvent may contain an aid such as a neutralizer and a buffer as necessary. Examples of the neutralizer include inorganic base compounds such as sodium bicarbonate, sodium hydroxide, potassium hydroxide, and slaked lime. In addition, examples of the buffer include carboxylates or phosphates such as disodium hydrogen phosphate, sodium dihydrogen phosphate, dipotassium hydrogen phosphate, and potassium dihydrogen phosphate.

The washing operation may be carried out two or more times as necessary, and the water or the water-containing solvent to be used for washing may be changed every time. Preferably, the purified peptide after the washing operation is dried, and the residue is removed therefrom, to obtain a product.

As described above, by treating the supported crude peptide by the hydrophilization step A and the support cleavage step B and then treating the obtained peptide by the chromatographic purification step and the linker cleavage step, it is made possible to highly and simply purify the peptide. In addition, in the present invention, the type of the peptide does not matter, so that the present invention is excellent in versatility and is suitable for application of simply purifying various peptides.

The present application claims for benefit of priority based on US Patent Application No. 62/055,991 filed on September 26, 2014. The entirety of the specification of US Patent Application No. 62/055,991 filed on September 26, 2014 is incorporated herein for reference.

EXAMPLES

Hereinafter, the present invention will be described more specifically by way of examples. The present invention is not limited to examples described below, and can also be carried out with appropriate modifications within the range adaptable to the gist described above and below, and such modifications are included in the technical scope of the present invention.

The apparatuses used in the examples of the present application are as follows.

- Automatic peptide synthesizer: Symphony manufactured by Protein Technologies, Inc.
- NMR: "JMTC-500" manufactured by JEOL Ltd.
- Mass spectrometry analyzer: "microflex" manufactured by Bruker Dartonics K.K. was used, and dihydroxybenzoic acid was used as a matrix.
- HPLC analyzer: "SPD-10Avp", "LC-10ADvp", "SCL-10Avp", "DGU-12A", "CTO-10ASvp", and "C-R8A" manufactured by Shimadzu Corporation.
- HPLC purifying apparatus: "ProStar (preparative chromatography apparatus)" manufactured by Varian, Inc. and "DYNAMAX Absorbance Detector model uv-D II (UV detector/preparative chromatography apparatus)' manufactured by Rainin Instrument LLC.

Synthesis Example 1 4-(2-Fmoc-amino)ethoxy-3,5-dichlorobenzyl succinimizyl carbonate (3a)

[Chemical formula 24]

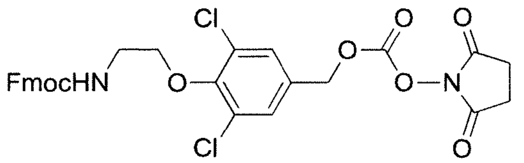

**(3a)**

4-(2-Fmoc-amino)ethoxy-3,5-dichlorobenzylalcohol (purity 98.3%, 200 mg, 0.429 mmol) was dissolved in methylene chloride (4 mL). An acetonitrile (8 mL) solution of di(N-succinimidyl)carbonate (165 mg, 0.643 mmol) and pyridine (51.8 μL, 0.643 mmol) were added to the solution in order. This solution was stirred at 25°C for 3 hours, and then was concentrated. Ethyl acetate (50 mL) was added to the residue to dissolve the residue therein, and washed with a 10% sodium bicarbonate solution (25 mL, three times), $H_2O$ (25 mL, twice), and a brine (25 mL, once) in order. The organic layer was dried over magnesium sulfate, and then filtration and concentration were performed to obtain a white solid of an activated carbonate type linker (3a) (276.7 mg, quantitative yield).
1H NMR (500 MHz, $CDCl_3$): δ7.77 (d, 3JHH = 7.0 Hz, 2H), 7.62 (d, 3JHH = 7.5 Hz, 2H), 7.40 (dd, 3JHH = 15.0 and 7.5 Hz, 2H), 7.33 (t, 3JHH = 7.5 Hz, 2H), 5.51 (bs, 1H), 5.21 (s, 2H), 4.41 (d, 3JHH = 7.0 Hz, 2H), 4.25 (t, 3JHH = 7.0 Hz, 1H), 4.20-4.16 (m, 2H), 3.68-3.65 (m, 2H)

Synthesis Example 2 4-(2-Fmoc-amino)ethoxy-1-formylbenzene (5)

[Chemical formula 25]

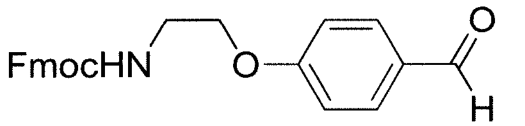

**(5)**

Manganese dioxide (4.47 g, 51.4 mmol) was added to a methylene chloride (30 mL) solution of 4-(2-Fmoc-amino)ethoxybenzylalcohol (1.00 g, 2.57 mmol), and this solution was stirred at 25°C for 35 hours. Celite filtration was performed, and the filtrate was concentrated. Then, silica gel column chromatography (ethyl acetate/hexane/methylene chloride = 1/1/1) purification was performed to obtain an aldehyde type linker (5) as 0.92 g of a white solid (2.37 mmol, 92% yield).

1H NMR (500 MHz, $CDCl_3$): δ9.90 (s, 1H), 7.85 (d, 3JHH = 8.5 Hz, 2H), 7.75 (d, 3JHH = 7.5 Hz, 2H), 7.59-7.57 (m, 2H), 7.40-7.39 (m, 2H), 7.31-7.26 (m, 2H), 7.01 (d, 3JHH = 8.0 Hz, 2H), 5.19 (s, 1H), 4.45 (d, 3JHH = 7.0 Hz, 2H), 4.22 (t, 3JHH = 6.5 Hz, 1H), 4.14-4.10 (m, 2H), 3.66-3.63 (m, 2H)

Synthesis Example 3 2-(2-Fmoc-amino)ethoxy-3,5-dichlorobenzyl su ccinimizyl carbonate (3d)

[Chemical formula 26]

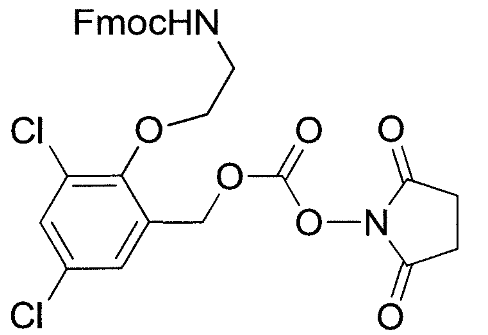

(3d)

An acetonitrile (28 mL) solution of di(N-succinimidyl)carbonate (384 mg, 1.5 mmol) was added to a $CH_2Cl_2$ (14 mL) solution of 2-(2-Fmoc-amino)ethoxy-3,5-dichlorobenzylalcohol (458 mg, 1.00 mmol) . Pyridine (0.121 mL, 1.5 mmol) was added dropwise to this solution for 1 minute, and then the solution was stirred at 25°C for 11 hours. The solvent was removed under reduced pressure, and EtOAc (100 mL) was added to the residue, thereafter washed with a saturated sodium bicarbonate solution (50 mL, three times), water (50 mL, four times), and a brine (50 mL, once) in order. The obtained EtOAc layer was dried over $MgSO_4$, filtered, and concentrated under reduced pressure to obtain 2-(2-Fmoc-amino)ethoxy-3,5-dichlorobenzyl succinimizyl carbonate as a white solid (purity 94.7%; 615 mg, 0.972 mmol, 97% yield).
1H NMR (500 MHz, $CDCl_3$): δ7.77 (d, 3JHH = 7.5 Hz, 2H), 7.63 (d, 3JHH = 7.5 Hz, 2H), 7.45 (d, 4JHH = 2.0 Hz, 1H), 7.40 (t, 3JHH = 7.5 Hz, 2H), 7.33-7.30 (m, 2H), 7.32 (d, 4JHH = 2.5 Hz, 1H), 5.57 (t, 3JHH = 6.0 Hz, 1H), 5.32 (s, 2H), 4.40 (d, 3JHH = 7.0 Hz, 2H), 4.25 (t, 3JHH = 7.0 Hz, 1H), 4.12(t, 3JHH = 5.0 Hz, 2H), 3.66 (dt, 3JHH = 5.5 Hz and 5.0 Hz, 2H), 2.79 (s, 4H)

Synthesis Example 4 4-(2-Fmoc-amino)ethoxy-3,5-dibromochlorobenzyl succinimizyl carbonate (3b)

[Chemical formula 27]

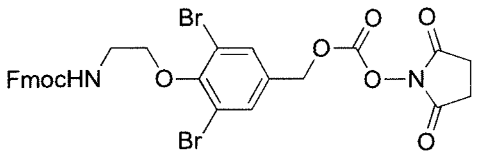

(3b)

An acetonitrile (28 mL) solution of di(N-succinimidyl)carbonate (384 mg, 1.5 mmol) was added to a $CH_2Cl_2$ (14 mL) solution of 4-(2-Fmoc-amino)ethoxy-3,5-dibromobenzylalcohol (547 mg, 1.00 mmol). Pyridine (0.121 mL, 1.5 mmol) was added dropwise to this solution for 1 minute, and then the solution was stirred at 25°C for 11 hours. The solvent was removed under reduced pressure, and EtOAc (100 mL) was added to the residue, thereafter washed with a saturated sodium bicarbonate solution (50 mL, three times), water (50 mL, four times), and a brine (50 mL, once) in order. The obtained EtOAc layer was dried over $MgSO_4$, filtered, and concentrated under reduced pressure to obtain a white solid (purity 93.2%; 674 mg, 0.913 mmol, 91% yield).
1H NMR (500 MHz, $CDCl_3$): δ7.77 (d, 3JHH = 7.5 Hz, 2H), 7.62 (d, 3JHH = 7.5 Hz, 2H), 7.57 (s, 2H), 7.41 (t, 3JHH = 7.5 Hz, 2H), 7.32 (t, 3JHH = 7.5 Hz, 2H), 5.52 (t, 3JHH = 6.0 Hz, 1H), 5.21 (s, 2H), 4.42 (d, 3JHH = 6.5 Hz, 2H), 4.25

(t, 3JHH = 6.5 Hz, 1H), 4.13 (t, 3JHH = 6.5 Hz, 2H), 3.69-3.66 (m, 2H), 2.85 (s, 4H)

Synthesis Example 5 4-(2-Fmoc-amino)ethoxy-3-nitrobenzyl succinimizyl carbonate (3c)

[Chemical formula 28]

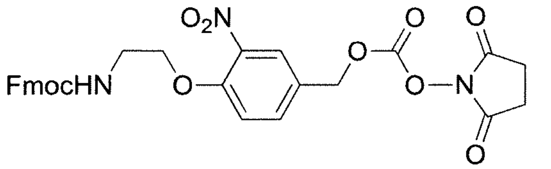

(3c)

An acetonitrile (28 mL) solution of di(N-succinimidyl)carbonate (530 mg, 2.07 mmol) was added to a $CH_2Cl_2$ (14 mL) solution of 4-(2-Fmoc-amino)ethoxy-3-nitrobenzylalcohol (purity 94.5%; 0.63 g, 1.37 mmol). Pyridine (0.167 mL, 2.07 mmol) was added dropwise to this solution for 1 minute, and then the solution was stirred at 25°C for 4 hours. The solvent was removed under reduced pressure, and EtOAc (100 mL) was added to the residue, thereafter washed with a saturated sodium bicarbonate solution (50 mL, three times), water (50 mL, four times), and a brine (50 mL, once) in order. The obtained EtOAc layer was dried over $MgSO_4$, filtered, and concentrated under reduced pressure to obtain 4-(2-Fmoc-amino)ethoxy-3-nitrobenzyl succinimizyl carbonate (3c) as a white solid (purity 92.9%; 794 mg, 1.28 mmol, 93% yield).
1H NMR (500 MHz, $CDCl_3$): δ7.97 (d, 4JHH = 1.5 Hz, 1H), 7.74 (d, 3JHH = 7.5 Hz, 2H), 7.62-7.59 (m, 1H), 7.60 (d, 3JHH = 7.0 Hz, 2H), 7.38 (t, 3JHH = 7.5 Hz, 2H), 7.29 (t, 3JHH = 7.5 Hz, 2H), 7.11 (d, 3JHH = 8.5 Hz, 1H), 5.48 (t, 3JHH = 5.5 Hz, 1H), 5.29 (s, 2H), 4.38 (d, 3JHH = 7.0 Hz, 2H), 4.23-4.19 (m, 3H), 3.69-3.66 (m, 2H), 2.84 (s, 4H)

Synthesis Example 6 4-(6-Fmoc-amino)hexoxy-3,5-dichlorobenzyl succinimizyl carbonate (3e)

[Chemical formula 29]

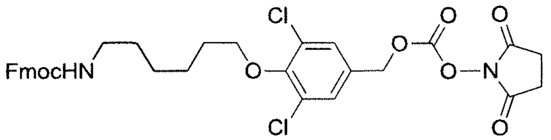

(3e)

An acetonitrile (10 mL) solution of di(N-succinimidyl)carbonate (269 mg, 1.05 mmol) was added to a $CH_2Cl_2$ (5 mL) solution of 4-(6-Fmoc-amino)hexoxy-3,5-dichlorobenzylalcohol (360 mg, 0.7 mmol), further an acetonitrile (1 mL) solution of pyridine (83mg, 1.05 mmol) was added dropwise to this solution, and then the solution was stirred at 25°C for 4 hours. The solvent was removed under reduced pressure, and EtOAc (20 mL) was added to the residue, thereafter washed with a saturated sodium bicarbonate solution (10 mL, three times), water (10 mL, once), and a brine (10, mL once) in order. The obtained EtOAc layer was dried over magnesium sulfate. Then, the magnesium sulfate was separated by filtration, and the filtrate was concentrated under reduced pressure to obtain 4-(6-Fmoc-amino)hexoxy-3,5-dichlorobenzyl succinimizyl carbonate (3e) as a white solid having a high viscosity (383 mg, 96% yield).
1H NMR (500 MHz, $CDCl_3$): δ7.77 (d, J = 7.5 Hz, 2H), 7.60 (d, J = 7.5 Hz, 2H), 7.40 (t, J = 7.5 Hz, 2H), 7.34 (s, 2H), 7.32 (t, J = 7.0 Hz, 2H), 4.7 (d, J = 6 Hz, 2H), 4.40 (d, J = 7 Hz, 2H), 4.23-4.20 (m, 1H), 4.03 (t, J = 6.5 Hz, 2H), 3.25-3.18 (m, 2H), 2.85 (s, 4H), 1.88-1.80 (m, 2H), 1.60-1.51 (m, 4H), 1.47-1.38 (m, 2H)

Example 1

<Production of supported crude peptide: Rink Amide resin-supported 9-residue hydrophobic peptide GILTVSVAV (GRAVY score: 2.31)>

The titled supported crude peptide was synthesized by Fmoc solid-phase peptide synthesis with the automatic peptide synthesizer, using 0.41 mmol/g of Rink Amide resin and O-(6-chlorobenzotriazol-1-yl)-N,N,N',N'-tetramethyluronium hexafluorophosphate (HCTU) as a coupling reagent.

<Linker bonding step (connection of activated carbonate type linker (3a) to Rink Amide resin-supported 9-residue hydrophobic peptide GILTVSVAV)>

The activated carbonate type linker (3a) (73.7 mg, 0.123 mmol) dissolved in DMF (0.7 mL) was mixed with the Rink Amide resin-supported 9-residue hydrophobic peptide GILTVSVAV (0.41 mmol/g, 100 mg, 0.041 mmol), and the mixture was shaken at 25°C for 14 hours. Next, the mixture was filtered, and the obtained supported linker-bonded compound was washed with DMF (10 mL) and methylene chloride (10 mL) to obtain 122 mg of a supported linker-bonded compound to which the activated carbonate type linker (3a) is connected.

The obtained linker-bonded compound was transformed into a high-purity hydrophobic peptide by the following scheme.

[Chemical formula 30]

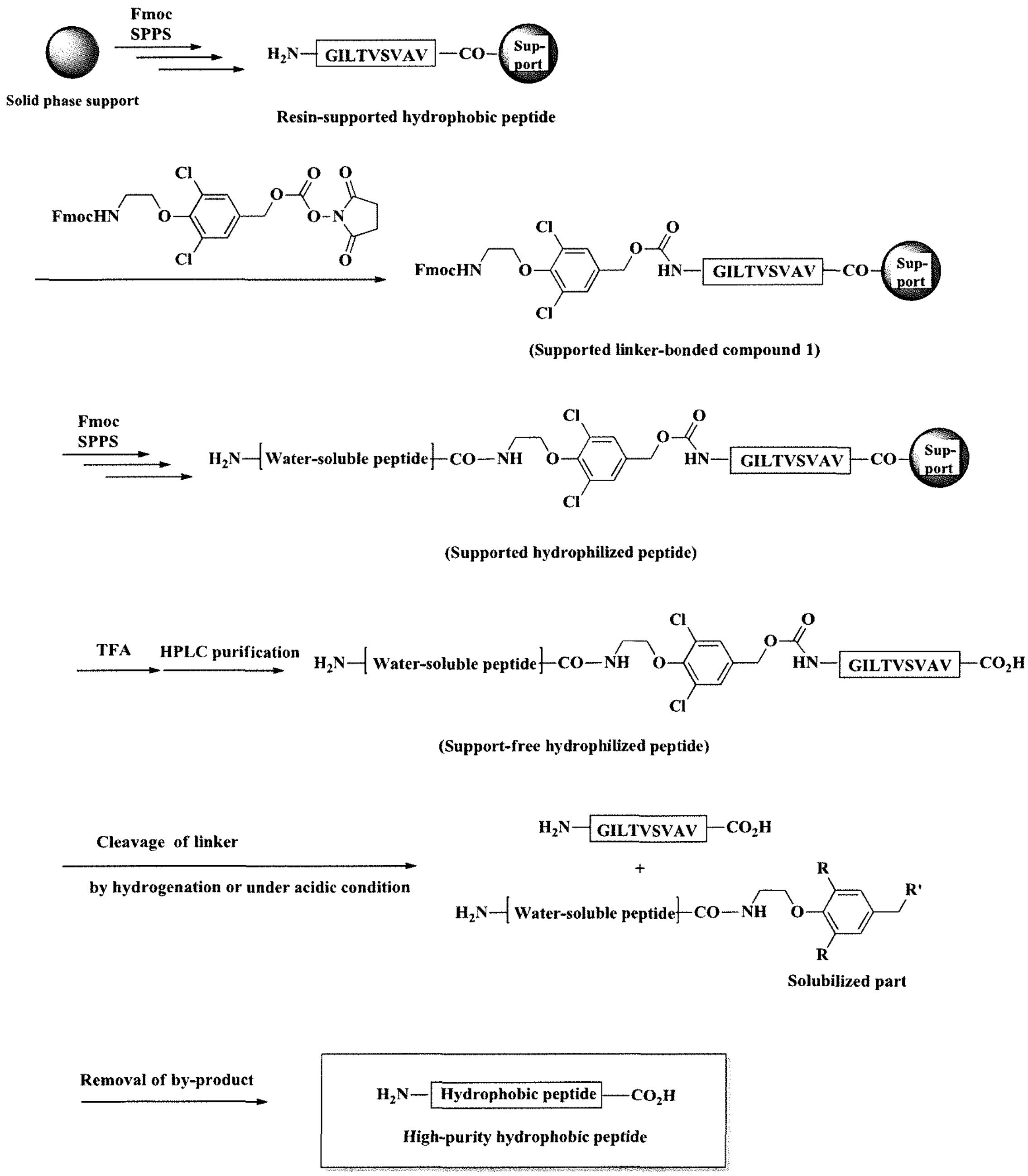

<Hydrophilic unit bonding step>

The supported linker-bonded compound (616 mg) was washed with DMF (3 mL, twice) and further washed with $CH_2Cl_2$ (3 mL, once), and then the excessive solvent was removed under reduced pressure. An Fmoc deprotection reaction was carried out by adding a 20% (v/v) piperidine/DMF solution (3 mL) to the supported linker-bonded compound, shaking the mixture at 25°C for 20 minutes, and then performing filtration. This piperidine treatment operation was repeated four times in total, and finally washed with DMF (3 mL). An Fmoc amino acid was coupled by adding a DMF (3 mL) solution prepared in a ratio of Fmoc amino acid : HOBt : HBTU : DIPEA (diisopropylethylamine) = 3 mol: 3 mol: 6 mol with respect to 1 mol of the supported linker-bonded compound after the Fmoc deprotection, agitating the mixture at 25°C for 20 minutes, and then performing filtration. This coupling operation was repeated three times in total. After absence of any unreacted amino group was confirmed by a color reaction of a ninhydrin reagent, an Fmoc deprotection reaction by a 20% piperidine/DMF solution and a coupling operation were repeated until a desired sequence was obtained. Accordingly, 963 mg of a supported hydrophilized peptide was obtained in which a 11-residue peptide chain composed of (Fmoc)Gly, (Fmoc)Lys(Boc), (Fmoc)Gly, (Fmoc)Lys(Boc), (Fmoc)Gly, (Fmoc)Lys(Boc), (Fmoc)Gly, (Fmoc)Lys(Boc), (Fmoc)Gly, (Fmoc)Lys(Boc), and (Fmoc)Gly in this order is connected to the linker molecule.

<Support cleavage step>

A mixed solution of TFA (trifluoroacetic acid) : TIS (triisopropylsilane) : $H_2O$ = 95:2.5:2.5 (volume ratio, 2 mL) was added to the supported hydrophilized peptide (200 mg), and the mixture was shaken at 25°C for 2 hours. The support was separated by filtration, and the residue was washed with TFA (1 mL). The filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 71 mg of a crude product of a support-free hydrophilized peptide as a white solid.

HPLC analysis and mass spectrometry analysis were performed on the obtained hydrophilized peptide. The results are shown below, and the obtained charts are shown in FIGS. 1 and 2.

(Support-free hydrophilized peptide (crude product)>

HPLC column: SUPELCO Discovery BIO Wide Pore C18-5, 25 cm X 4.6 mm, 5 $\mu$m
Flow rate: 1 mL/min
Column temperature: 40°C
Wavelength: 220 nm
Eluent: 0.1% TFA acetonitrile/0.1% TFA aqueous solution = 0 $\rightarrow$ 100 (0-20 minutes), 100 (20-34.5 minutes), 100 $\rightarrow$ 0 (34.5-35 minutes), 0 (35-39.9 minutes)
Retention time: 13.7 minutes
MALDI-TOF MS: (M+H+) m/z = 2101.917 (calculated value: 2101.14)

<Chromatographic purification step>

The support-free hydrophilized peptide was purified by high performance liquid chromatography (HPLC) under the following conditions. The execution conditions and the results are shown. In addition, the obtained charts are shown in FIGS. 3 and 4.

HPLC purification conditions
Column: SUPELCO C18 column
Eluent: 0.1% TFA aqueous solution/0.1% acetonitrile solution = 85/15 $\rightarrow$ 50/50 (70 min)
Flow rate: 20 mL/min
Detection wavelength: 225 nm

<Support-free hydrophilized peptide (purified product)>

HPL Column: SUPELCO C18 column
Flow rate: 1.0 mL/min
Column temperature: 40°C
Detection wavelength: 225 nm
Eluent: 0.1% TFA aqueous solution/0.1% acetonitrile solution = 95/5 → 40/60 (8 min)
Retention time: 4.8 min
MALDI-TOF MS: (M+H+) m/z = 2103.6 (calculated value 2101.14)

<Linker cleavage step (cleavage of linker by catalytic reduction reaction>

The support-free hydrophilized peptide (10 mg) was dissolved in methanol (3 mL), and 10% Pd/C (10% water wet, 30 mg) was added thereto. The atmosphere is replaced by introducing hydrogen gas into the vessel so that it became under a hydrogen atmosphere of 1 atm. The mixture was stirred at 50°C for 15 hours, and then the solvent was removed under reduced pressure. Trifluoroacetic acid (1.0 mL) was added to the obtained residue, and the mixture was filtered. The solid was washed with TFA (1 mL), the filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 11.8 mg of white powder.

<Obtaining hydrophobic peptide (removal of solubilized part)>

The linker-cleaved hydrophobic peptide obtained by the previous step was washed with 1.0 mL of 10% sodium hydrogencarbonate and 1.0 mL of acetonitrile/$H_2O$ = 1/1 (volume ratio) in order. The residue was vacuum-dried to obtain 5.0 mg of a hydrophobic peptide GILTVSVAV as white powder. The HPLC analysis results and the mass spectrometry analysis results of the obtained hydrophobic peptide are shown in FIGS. 5 and 6.

Example 2

<Production of supported crude peptide: TentaGel resin-supported amyloid β (17 to 40 fragment) LVFFAEDVGSNKGAIIGLMVGGVV (GRAVY score: 1.63)>

The titled supported crude peptide was synthesized by solid-phase peptide synthesis with the automatic peptide synthesizer, using TentaGel resin of 0.41 mmol/g and O-(6-chlorobenzotriazol-1-yl)-N,N,N',N'-tetramethyluronium hexafluorophosphate (HCTU) as a coupling reagent.

<Linker bonding step (connection of activated carbonate type linker (3a) to TentaGel resin-supported amyloid β (17 to 40 fragment)>

The activated carbonate type linker (3a) (144 mg, 0.24 mmol) dissolved in DMF (3 mL) was mixed with the TentaGel resin-supported amyloid 6 (17 to 40 fragment) (0.15 mmol/g, 800 mg, 0.12 mmol), and the mixture was shaken at 25°C for 2 hours. Next, the supported linker-bonded compound was washed with DMF (10 mL) and methylene chloride (10 mL) to obtain 846 mg of a supported linker-bonded compound to which the activated carbonate type linker (3a) is connected.

<Hydrophilic unit bonding step>

Deprotection of an Fmoc group and a coupling operation of an Fmoc amino acid were repeated on the supported linker-bonded compound (846 mg) by the same Fmoc solid-phase peptide synthesis as in Example 1. Accordingly, a peptide chain composed of 11 amino acids in total including (Fmoc)Gly, (Fmoc)Lys(Boc), (Fmoc)Gly, (Fmoc)Lys(Boc), (Fmoc)Gly, (Fmoc)Lys(Boc), (Fmoc)Gly, (Fmoc)Lys(Boc), (Fmoc)Gly, (Fmoc)Lys(Boc), and (Fmoc)Gly in this order was connected to the linker molecule, and 992 mg of a supported hydrophilized peptide 2 was obtained.

(Support cleavage step (case where sequence includes S atom)>

Three milliliters of a mixed solution of TFA : thioanisole : 3,6-dioxa-1,8-octanedithiol : $H_2O$ = 90:5:3:5 (volume ratio) was added to the supported hydrophilized peptide (400 mg), and the mixture was shaken at 25°C for 2 hours. The resin was separated by filtration, and the residue was washed with TFA. The filtrate and the washing liquid were mixed,

hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 133 mg of a crude product of a support-free hydrophilized peptide.

HPLC analysis and mass spectrometry analysis were performed on the obtained hydrophilized peptide. The results are shown below.

<Support-free hydrophilized peptide (crude product)>

HPLC column: SUPELCO Discovery BIO Wide Pore C18-5, 25 cm X 4.6 mm, 5 μm
Flow rate: 1 mL/min
Column temperature: 40°C
Wavelength: 220 nm
Eluent: 0.1% TFA acetonitrile/0.1% TFA aqueous solution = 0 → 100 (0-20 minutes), 100 (20-34.5 minutes), 100 → 0 (34.5-35 minutes), 0 (35-39.9 minutes)
Retention time: 15.7 minutes
MALDI-TOF MS: (M+H+) m/z = 3635.872 (calculated value: 3635.887)

<Chromatographic purification step>

The chromatographic purification step is preferably performed by the same method as in Example 1.

<Linker cleavage step (cleavage of linker under acidic condition)>

1.0 mL of a mixed solution of TFA : m-cresol: TMSOTf = 68:12:20 (volume ratio) was added to the support-free hydrophilized peptide (5 mg), and the mixture was stirred at 0°C for 4 hours. Hexane (5 mL) was added to the mixture, and the mixture was vigorously shaken. Then, the mixture was allowed to stand to be separated into two layers. After the hexane layer (upper layer) was removed, diethyl ether (5 mL) was added to the remaining TFA layer to afford a solid precipitate. After centrifugal separation, the supernatant was removed, and the solid was further washed with diethyl ether three times. Diethyl ether was removed under reduced pressure to obtain 10.1 mg of a crude product.

Example 3

<Supported linker bonding step (connection of aldehyde type linker (5) to Rink Amide resin-supported 9-residue hydrophobic peptide GILTVSVAV) (GRAVY score: 2.31)>

The Rink Amide resin-supported 9-residue hydrophobic peptide GILTVSVAV (0.41 mmol/g; 150 mg, 0.063 mmol), the aldehyde type linker (5) (240 mg, 0.615 mmol), and a methylene chloride/trimethyl orthoformate mixed solution (volume ratio 2/1; 3.0 mL) were mixed, and the mixture was shaken at 25°C for 13 days. After filtration, the residue was washed with methylene chloride (5 mL) and dried under reduced pressure to obtain 155.9 mg of an imine resin.

$NaBH_3CN$ (32.0 mg, 0.51 mmol) and a DMF/acetic acid/methanol mixed solution (9/9/2. 6 mL) were added to 26 mg of the obtained imine resin, and the mixture was shaken at 25°C for 24 hours. 28 mg of a reduced form resin was obtained by filtration and washing with DMF (3 mL) and methylene chloride (3 mL).

Di-tert-butyldicarbonate (164 mg, 0.75 mmol), N-methylmorpholine (83 μL, 0.75 mmol), and DMF (1 mL) were mixed into the obtained reduced form resin (183 mg, 0.075 mmol), and the mixture was shaken at 25°C for 19 hours. Di-tert-butyldicarbonate (164 mg, 0.75 mmol), N-methylmorpholine (83 μL, 0.75 mmol), and DMF (1 mL) were added to the resin again after filtration and washing with DMF (3 mL), and the mixture was shaken at 25°C for 23 hours. Filtration, washing with DMF (3 mL) and methylene chloride (3 mL), and drying under reduced pressure were performed to obtain 209 mg of a supported linker-bonded compound to which the aldehyde type linker (5) is connected.

The obtained supported linker-bonded compound was transformed into a high-purity hydrophobic peptide by the following scheme.

[Chemical formula 31]

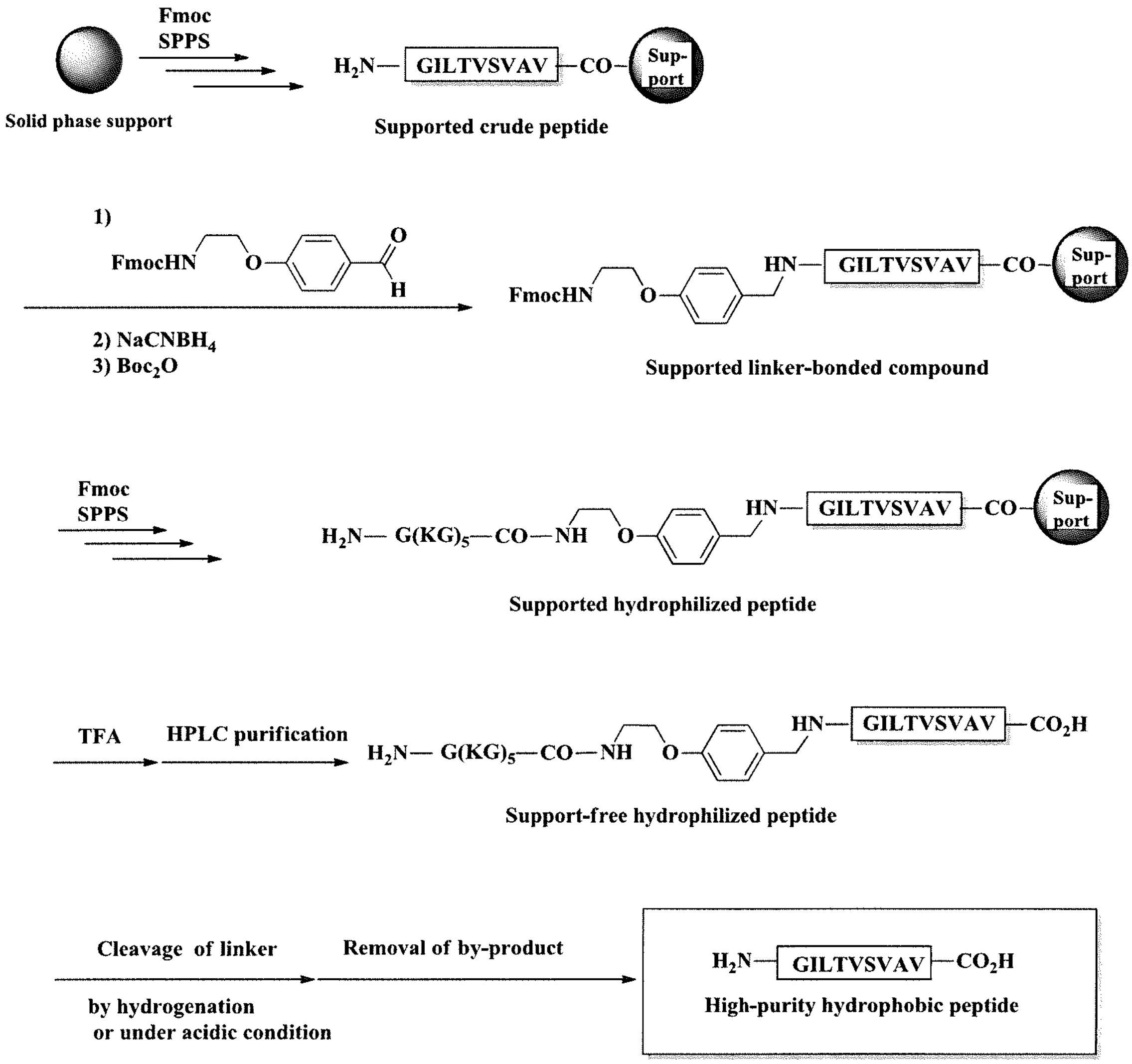


<Hydrophilic unit bonding step>

Deprotection of an Fmoc group and a coupling operation of an Fmoc amino acid were repeated on the supported linker-bonded compound (400 mg) by the same Fmoc solid-phase peptide synthesis as in Example 1. Accordingly, a peptide chain composed of 11 amino acids in total including (Fmoc)Gly, (Fmoc)Lys(Boc), (Fmoc)Gly, (Fmoc)Lys(Boc), (Fmoc)Gly, (Fmoc)Lys(Boc), (Fmoc)Gly, (Fmoc)Lys(Boc), (Fmoc)Gly, (Fmoc)Lys(Boc), and (Fmoc)Gly in this order was connected to the linker molecule, and 472 mg of a supported hydrophilized peptide was obtained.

<Support cleavage step>

A crude product (78 mg) of a support-free hydrophilized peptide w as obtained from the hydrophilized peptide (200 mg) by the same method as i n Example 1. HPLC analysis and mass spectrometry analysis were performed on the obtained hydrophilized peptide.

<Support-free hydrophilized peptide (crude product)>

MALDI-TOF MS: (M+H+) m/z = 1989.330 (calculated value: 1989.230)

<Chromatographic purification step>

The chromatographic purification step is preferably performed by the same method as in Example 1.

Example 4

<Production of supported crude peptide: TentaGel resin-supported 24-residue hydrophobic peptide LVFFAEDVGSNKGAIIGLMVGGVV (GRAVY score: 1.63)>

A solid phase resin (TentaGel resin) supporting a 24 residue peptide composed of Leu-Val-Phe-Phe-Ala-Glu(tBu)-Asp(tBu)-Val-Gly-Ser(tBu)-Asn(Trt)-Lys(Boc)-Gly-Ala-Ile-Ile-Gly-Leu-Met-Val-Gly-Gly-Val-Val was obtained by Fmoc solid-phase peptide synthesis using the automatic peptide synthesizer.

<Linker bonding step (connection of activated carbonate type linker (3a) to TentaGel resin-supported 24-residue hydrophobic peptide LVFFAEDVGSNKGAIIGLMVGGVV)>

The activated carbonate type linker (3a) (144 mg, 0.24 mmol) dissolved in DMF (3 mL) was mixed with the TentaGel resin-supported 24-residue hydrophobic peptide LVFFAEDVGSNKGAIIGLMVGGVV (0.15 mmol/g, 800 mg, 0.12 mmol), and the mixture was shaken at 25°C for 2 hours. Next, the mixture was filtered, and the obtained supported linker-bonded compound was washed with DMF (10 mL) and methylene chloride (10 mL) to obtain 846 mg of a supported linker-bonded compound to which the activated carbonate type linker (3a) is connected.

<Hydrophilic unit bonding step>

The supported linker-bonded compound (846 mg) was washed with DMF (3 mL, twice) and $CH_2Cl_2$ (3 mL, once) in order, and the excessive solvent was removed under reduced pressure. Deprotection of an Fmoc group was performed by adding a 20% (v/v) piperidine/DMF solution (3 mL) to the supported linker-bonded compound, shaking the mixture at 25°C for 20 minutes, and then performing filtration. This piperidine treatment operation was repeated four times in total. After washing with DMF, deprotection of an Fmoc group and coupling of an Fmoc amino acid were repeated by the same Fmoc solid-phase peptide synthesis as in Example 1. Accordingly, a 3-residue peptide chain composed of (Fmoc)Lys(Boc), (Fmoc)Lys(Boc), and (Fmoc)Lys(Boc) in this order was connected to the linker molecule, and 860 mg of a supported hydrophilized peptide was obtained.

<Support cleavage step>

A mixed solution of TFA : phenol: water : thioanisole : 3,6-dioxa-1,8-octanedithiol = 80:8:5:5:2 (volume ratio, 5 mL) was added to the well-dried supported hydrophilized peptide (1.24 g, 0.0954 mmol), and the mixture was shaken at 25°C for 2 hours. The support was separated by filtration, and the solid was washed with TFA. The filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 196 mg of a crude product of a support-free hydrophilized peptide.

HPLC analysis and mass spectrometry analysis were performed on the obtained hydrophilized peptide. The results are shown below, and the obtained charts are shown in FIGS. 7 and 8.

<Support-free hydrophilized peptide (crude product)>

HPLC column: SUPELCO Discovery BIO Wide Pore C18-5, 25 cm X 4.6 mm, 5 μm
Flow rate: 1 mL/min
Column temperature: 40°C
Wavelength: 220 nm
Eluent: 0.1% TFA acetonitrile/0.1% TFA aqueous solution = 0 → 100 (0-20 minutes), 100 (20-34.5 minutes), 100 → 0 (34.5-35 minutes), (35-39.9 minutes)
Retention time: 13.7 minutes
MALDI-TOF MS: (M+H+) m/z = 3038.027 (calculated value: 3037.568)

<Chromatographic purification step>

The support-free hydrophilized peptide was purified by high performance liquid chromatography (HPLC) under the following conditions. The execution conditions and the results are shown. In addition, the obtained charts are shown in FIGS. 9 and 10.

HPLC purification conditions
Column: SUPELCO C18 column
Eluent: 0.1% TFA aqueous solution/0.1% acetonitrile solution = 85/15 → 50/50 (70 min)
Flow rate: 20 mL/min
Detection wavelength: 225 nm

<Support-free hydrophilized peptide (purified product)>

HPL Column: SUPELCO C18 column
Flow rate: 1.0 mL/min
Column temperature: 40°C
Detection wavelength: 225 nm
Eluent: 0.1% TFA aqueous solution/0.1% acetonitrile solution = 85/15 → 25/75 (20 min)
Retention time: 14.5 min
MALDI-TOF MS: (M+H+) m/z = 3039 (calculated value 3037.568)

<Linker cleavage step (cleavage of linker under acidic condition)>

4.0 mL of a mixed solution of TFA : thioanisole : TMSOTf = 68:12:20 (volume ratio) was added to the support-free hydrophilized peptide (20 mg), and the mixture was stirred at 0°C for 4 hours. When diethyl ether (40 mL) was added to the TFA layer, a solid precipitated. After centrifugal separation, the supernatant was removed, and the solid was further washed with diethyl ether three times. Diethyl ether was removed under reduced pressure to obtain 22 mg of a crude product.

<Obtaining hydrophobic peptide (removal of solubilized part)>

The linker-cleaved hydrophobic peptide obtained by the previous step was washed with 1 mL of a phosphate buffer (pH 7.2) twice and further washed with 1 mL of water twice. The residue was vacuum-dried to obtain 12 mg of a hydrophobic peptide Leu-Val-Phe-Phe-Ala-Glu-Asp-Val-Gly-Ser-Asn-Lys-Gly-Ala-Ile-Ile-Gly-Leu-Met-Val-Gly-Gly-Val-Val as white powder.

MALDI-TOF MS analysis of the above hydrophobic peptide was carried out. A measured value was 2395.1 (calculated value 2392.287). The HPLC analysis results and the mass spectrometry analysis results of the obtained hydrophobic peptide are shown in FIGS. 11 and 12.

Example 5

<Production of supported crude peptide: TentaGel resin-supported 24-residue hydrophobic peptide LVFFAEDVGSNKGAIIGLMVGGVV (GRAVY score: 1.63)>

A solid phase resin (TentaGel resin) supporting a 24 residue peptide composed of Leu-Val-Phe-Phe-Ala-Glu(tBu)-Asp(tBu)-Val-Gly-Ser(tBu)-Asn(Trt)-Lys(Boc)-Gly-Ala-Ile-Ile-Gly-Leu-Met-Val-Gly-Gly-Val-Val was obtained by Fmoc solid-phase peptide synthesis using the automatic peptide synthesizer.

<Linker bonding step (connection of activated carbonate type linker (3a) to TentaGel resin-supported 24-residue hydrophobic peptide LVFFAEDVGSNKGAIIGLMVGGVV)>

The activated carbonate type linker (3a) (144 mg, 0.24 mmol) dissolved in DMF (3 mL) was mixed with the TentaGel resin-supported 24-residue hydrophobic peptide LVFFAEDVGSNKGAIIGLMVGGVV (0.15 mmol/g, 800 mg, 0.12 mmol), and the mixture was shaken at 25°C for 2 hours. Next, the mixture was filtered, and the obtained supported linker-bonded compound was washed with DMF (10 mL) and methylene chloride (10 mL) to obtain 846 mg of a supported

linker-bonded compound to which the activated carbonate type linker (3a) is connected.

<Hydrophilic unit bonding step>

The supported linker-bonded compound (1.60 g) was washed with DMF (3 mL, twice) and $CH_2Cl_2$ (3 mL, once) in order, and the excessive solvent was removed under reduced pressure. Deprotection of an Fmoc group was performed by adding a 20% (v/v) piperidine/DMF solution (3 mL) to the supported linker-bonded compound, shaking the mixture at 25°C for 20 minutes, and then performing filtration. This piperidine treatment operation was repeated four times in total. After washing with DMF, deprotection of an Fmoc group and coupling of an Fmoc amino acid were repeated by the same Fmoc solid-phase peptide synthesis as in Example 1. Accordingly, a 6-residue peptide chain composed of (Fmoc)Arg(Pbf), (Fmoc)Arg(Pbf), (Fmoc)Arg(Pbf), (Fmoc)Arg(Pbf), (Fmoc)Arg(Pbf), and (Fmoc)Arg(Pbf) in this order was connected to the linker molecule, and 2.11 g of a supported hydrophilized peptide 7 was obtained.

<Support cleavage step>

A mixed solution of TFA : phenol : water : thioanisole : 3,6-dioxa-1,8-octanedithiol = 80:8:5:5:2 (volume ratio, 5 mL) was added to the well-dried supported hydrophilized peptide (1.24 g, 0.0954 mmol), and the mixture was shaken at 25°C for 2 hours. The support was separated by filtration, and the solid was washed with TFA. The filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 196 mg of a crude product of a support-free hydrophilized peptide.

<Chromatographic purification step>

The chromatographic purification step is preferably performed by the same method as in Example 1.
MALDI-TOF MS: (M+H+) m/z = 3590.686 (calculated value 3589.897)

<Linker cleavage step (cleavage of linker under acidic condition)>

4.0 mL of a mixed solution of TFA : thioanisole : TMSOTf = 68:12:20 (volume ratio) was added to the support-free hydrophilized peptide (20 mg), and the mixture was stirred at 0°C for 4 hours. When diethyl ether (40 mL) was added to the TFA layer, a solid precipitated. After centrifugal separation, the supernatant was removed, and the solid was further washed with diethyl ether three times. Diethyl ether was removed under reduced pressure to obtain 22 mg of a crude product.

<Obtaining hydrophobic peptide (removal of solubilized part)>

The linker-cleaved hydrophobic peptide obtained by the previous step was washed with 1 mL of a phosphate buffer (pH 7.2) twice and further washed with 1 mL of water twice. The residue was vacuum-dried to obtain 12.0 mg of a hydrophobic peptide Leu-Val-Phe-Phe-Ala-Glu-Asp-Val-Gly-Ser-Asn-Lys-Gly-Ala-Ile-Ile-Gly-Leu-Met-Val-Gly-Gly-Val-Val as white powder.
MALDI-TOF MS analysis of the above hydrophobic peptide was carried out. A measured value was 2392.683 (calculated value 2392.287).

Example 6

<Production of supported crude peptide: TentaGel resin-supported 24-residue hydrophobic peptide LVFFAEDVGSNKGAIIGLMVGGVV (GRAVY score: 1.63)>

A solid phase resin (TentaGel resin) supporting a 24 residue peptide composed of Leu-Val-Phe-Phe-Ala-Glu(tBu)-Asp(tBu)-Val-Gly-Ser(tBu)-Asn(Trt)-Lys(Boc)-Gly-Ala-Ile-Ile-Gly-Leu-Met-Val-Gly-Gly-Val-Val was obtained by Fmoc solid-phase peptide synthesis using the automatic peptide synthesizer.

<Linker bonding step (connection of activated carbonate type linker (3a) to TentaGel resin-supported 24-residue hydrophobic peptide LVFFAEDVGSNKGAIIGLMVGGVV)>

The activated carbonate type linker (3a) (144 mg, 0.24 mmol) dissolved in DMF (3 mL) was mixed with the TentaGel resin-supported 24-residue hydrophobic peptide LVFFAEDVGSNKGAIIGLMVGGVV (0.15 mmol/g, 800 mg, 0.12 mmol), and the mixture was shaken at 25°C for 2 hours. Next, the mixture was filtered, and the obtained supported linker-bonded compound was washed with DMF (10 mL) and methylene chloride (10 mL) to obtain 846 mg of a supported linker-bonded compound to which the activated carbonate type linker (3a) is connected.

<Hydrophilic unit bonding step>

The supported linker-bonded compound (1.60 g) was washed with DMF (3 mL, twice) and $CH_2Cl_2$ (3 mL, once) in order, and the excessive solvent was removed under reduced pressure. Deprotection of an Fmoc group was performed by adding a 20% (v/v) piperidine/DMF solution (3 mL) to the supported linker-bonded compound, shaking the mixture at 25°C for 20 minutes, and then performing filtration. This piperidine treatment operation was repeated four times in total. After washing with DMF, deprotection of an Fmoc group and coupling of an Fmoc amino acid were repeated by the same Fmoc solid-phase peptide synthesis as in Example 1. Accordingly, a 6-residue peptide chain composed of (Fmoc)Asp(tBu), (Fmoc)Asp(tBu), (Fmoc)Asp(tBu), (Fmoc)Asp(tBu), (Fmoc)Asp(tBu), and (Fmoc)Asp(tBu) in this order was connected to the linker molecule, and 1.98 g of a supported hydrophilized peptide was obtained.

<Support cleavage step>

A mixed solution of TFA : phenol : water : thioanisole : 3,6-dioxa-1,8-octanedithiol = 80:8:5:5:2 (volume ratio, 5 mL) was added to the well-dried supported hydrophilized peptide (1.24 g, 0.0954 mmol), and the mixture was shaken for 2 hours. The support was separated by filtration, and the solid was washed with TFA. The filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 196 mg of a crude product of a support-free hydrophilized peptide.

<Chromatographic purification step>

The chromatographic purification step is preferably performed by the same method as in Example 1.
MALDI-TOF MS: (M+H+) m/z = 3343.304 (calculated value 3343.452)

<Linker cleavage step (cleavage of linker under acidic condition)>

4.0 mL of a mixed solution of TFA : thioanisole : TMSOTf = 68:12:20 (volume ratio) was added to the support-free hydrophilized peptide (20 mg), and the mixture was stirred at 0°C for 4 hours. When diethyl ether (40 mL) was added to the TFA layer, a solid precipitated. After centrifugal separation, the supernatant was removed, and the solid was further washed with diethyl ether three times. Diethyl ether was removed under reduced pressure to obtain 22 mg of a crude product.

<Obtaining hydrophobic peptide (removal of solubilized part)>

The linker-cleaved hydrophobic peptide obtained by the previous step was washed with 1 mL of a phosphate buffer (pH 7.2) twice and further washed with 1 mL of water twice. The residue was vacuum-dried to obtain 12.0 mg of a hydrophobic peptide Leu-Val-Phe-Phe-Ala-Glu-Asp-Val-Gly-Ser-Asn-Lys-Gly-Ala-Ile-Ile-Gly-Leu-Met-Val-Gly-Gly-Val-Val as white powder.
MALDI-TOF MS analysis of the above hydrophobic peptide was carried out. A measured value was 2393.210 (calculated value 2392.287).

Example 7

<Production of supported crude peptide: TentaGel resin-supported 24-residue hydrophobic peptide LVFFAEDVGSNKGAIIGLMVGGVV (GRAVY score: 1.63)>

A solid phase resin (TentaGel resin) supporting a 24 residue peptide composed of Leu-Val-Phe-Phe-Ala-Glu(tBu)-Asp(tBu)-Val-Gly-Ser(tBu)-Asn(Trt)-Lys(Boc)-Gly-Ala-Ile-Ile-Gly-Leu-Met-Val-Gly-Gly-Val-Val was obtained by Fmoc solid-phase peptide synthesis using the automatic peptide synthesizer.

<Linker bonding step (connection of activated carbonate type linker (3d) to TentaGel resin-supported 24-residue hydrophobic peptide LVFFAEDVGSNKGAIIGLMVGGVV)>

The activated carbonate type linker (3d) (39 mg, 0.068 mmol) dissolved in DMF (1.5 mL) was mixed with the TentaGel resin-supported 24-residue hydrophobic peptide LVFFAEDVGSNKGAIIGLMVGGVV (0.15 mmol/g, 300 mg, 0.045 mmol), and the mixture was shaken at 25°C for 2 hours. Next, the mixture was filtered, and the obtained supported linker-bonded compound was washed with DMF (10 mL) and methylene chloride (10 mL) to obtain a supported linker-bonded compound to which the activated carbonate type linker (3d) is connected.

<Hydrophilic unit bonding step>

Deprotection of Fmoc was performed by adding a 20% piperidine/DMF solution (2 mL) to the supported linker-bonded compound and shaking the mixture for 20 minutes. This piperidine treatment operation was repeated four times in total. After washing with DMF, deprotection of an Fmoc group and coupling of an Fmoc amino acid were repeated by the same Fmoc solid-phase peptide synthesis as in Example 1. Accordingly, a 3-residue peptide chain composed of (Fmoc)Lys(Boc), (Fmoc)Lys(Boc), and (Fmoc)Lys(Boc) in this order was connected to the linker molecule, and a supported hydrophilized peptide was obtained.

<Support cleavage step>

A mixed solution of TFA : phenol : water : thioanisole : 3,6-dioxa-1,8-octanedithiol = 80:8:5:5:2 (volume ratio, 1 mL) was added to the well-dried supported hydrophilized peptide (50 mg, 0.000311 mmol), and the mixture was shaken for 2 hours. The support was separated by filtration, and the solid was washed with TFA. The filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 196 mg of a crude product of a support-free hydrophilized peptide.

<Chromatographic purification step>

The chromatographic purification step is preferably performed by the same method as in Example 1.
MALDI-TOF MS: (M+H+) m/z = 3037.812 (calculated value 3037.568)

<Linker cleavage step (cleavage of linker under acidic condition)>

1.0 mL of a mixed solution of TFA : thioanisole : TMSOTf = 68:12:20 (volume ratio) was added to the support-free hydrophilized peptide (5 mg), and the mixture was stirred at 0°C for 4 hours. When diethyl ether (40 mL) was added to the TFA layer, a solid precipitated. After centrifugal separation, the supernatant was removed, and the solid was further washed with diethyl ether three times. Diethyl ether was removed under reduced pressure to obtain 6.9 mg of a crude product.

<Obtaining hydrophobic peptide (removal of solubilized part)>

The linker-cleaved hydrophobic peptide (6.9 mg) obtained by the previous step was washed with 1 mL of a phosphate buffer (pH 7.2) twice and further washed with 1 mL of water twice. The residue was vacuum-dried to obtain 3.0 mg of a hydrophobic peptide Leu-Val-Phe-Phe-Ala-Glu-Asp-Val-Gly-Ser-Asn-Lys-Gly-Ala-Ile-Ile-Gly-Leu-Met-Val-Gly-Gly-Val-Val as white powder.
MALDI-TOF MS analysis of the above hydrophobic peptide was carried out. A measured value was 2392.939

(calculated value 2392.287).

Example 8

<Production of supported crude peptide: TentaGel resin-supported 24-residue hydrophobic peptide LVFFAEDVGSNKGAIIGLMVGGVV (GRAVY score: 1.63)>

A solid phase resin (TentaGel resin) supporting a 24 residue peptide composed of Leu-Val-Phe-Phe-Ala-Glu(tBu)-Asp(tBu)-Val-Gly-Ser(tBu)-Asn(Trt)-Lys(Boc)-Gly-Ala-Ile-Ile-Gly-Leu-Met-Val-Gly-Gly-Val-Val was obtained by Fmoc solid-phase peptide synthesis using the automatic peptide synthesizer.

<Linker bonding step (connection of activated carbonate type linker (3b) to TentaGel resin-supported 24-residue hydrophobic peptide LVFFAEDVGSNKGAIIGLMVGGVV)>

The activated carbonate type linker (3b) (46 mg, 0.068 mmol) dissolved in DMF (1.5 mL) was mixed with the TentaGel resin-supported 24-residue hydrophobic peptide LVFFAEDVGSNKGAIIGLMVGGVV (0.15 mmol/g, 300 mg, 0.045 mmol), and the mixture was shaken at 25°C for 2 hours. Next, the mixture was filtered, and the obtained supported linker-bonded compound was washed with DMF (10 mL) and methylene chloride (10 mL) to obtain a supported linker-bonded compound to which the activated carbonate type linker (3b) is connected.

<Hydrophilic unit bonding step>

Deprotection of Fmoc was performed by adding a 20% piperidine/DMF solution (2 mL) to the supported linker-bonded compound and shaking the mixture for 20 minutes. This piperidine treatment operation was repeated four times in total. After washing with DMF, deprotection of an Fmoc group and coupling of an Fmoc amino acid were repeated by the same Fmoc solid-phase peptide synthesis as in Example 1. Accordingly, a 3-residue peptide chain composed of (Fmoc)Lys(Boc), (Fmoc)Lys(Boc), and (Fmoc)Lys(Boc) in this order was connected to the linker molecule.

<Support cleavage step>

A mixed solution of TFA : phenol: water : thioanisole : 3,6-dioxa-1,8-octanedithiol = 80:8:5:5:2 (volume ratio, 1 mL) was added to the well-dried supported hydrophilized peptide (44 mg, 0.00410 mmol), and the mixture was shaken for 2 hours. The support was separated by filtration, and the solid was washed with TFA. The filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 6.0 mg of a crude product of a support-free hydrophilized peptide.

<Chromatographic purification step>

The chromatographic purification step is preferably performed by the same method as in Example 1.
MALDI-TOF MS: (M+H+) m/z = 3125.946 (calculated value 3125.474)

<Linker cleavage step (cleavage of linker under acidic condition)>

1.0 mL of a mixed solution of TFA : thioanisole : TMSOTf = 68:12:20 (volume ratio) was added to the support-free hydrophilized peptide (5 mg), and the mixture was stirred at 0°C for 4 hours. When diethyl ether (40 mL) was added to the TFA layer, a solid precipitated. After centrifugal separation, the supernatant was removed, and the solid was further washed with diethyl ether three times. Diethyl ether was removed under reduced pressure to obtain 7.5 mg of a crude product.

<Obtaining hydrophobic peptide (removal of solubilized part)>

The linker-cleaved hydrophobic peptide obtained by the previous step was washed with 1 mL of a phosphate buffer (pH 7.2) twice and further washed with 1 mL of water twice. The residue was vacuum-dried to obtain 13.0 mg of a hydrophobic peptide Leu-Val-Phe-Phe-Ala-Glu-Asp-Val-Gly-Ser-Asn-Lys-Gly-Ala-Ile-Ile-Gly-Leu-Met-Val-Gly-Gly-Val-Val as white powder.

MALDI-TOF MS analysis of the above hydrophobic peptide was carried out. A measured value was 2392.168 (calculated value 2392.287).

Example 9

<Production of supported crude peptide: TentaGel resin-supported 24-residue hydrophobic peptide LVFFAEDVGSNKGAIIGLMVGGVV (GRAVY score: 1.63)>

A solid phase resin (TentaGel resin) supporting a 24 residue peptide composed of Leu-Val-Phe-Phe-Ala-Glu(tBu)-Asp(tBu)-Val-Gly-Ser(tBu)-Asn(Trt)-Lys(Boc)-Gly-Ala-Ile-Ile-Gly-Leu-Met-Val-Gly-Gly-Val-Val was obtained by Fmoc solid-phase peptide synthesis using the automatic peptide synthesizer.

<Linker bonding step (connection of activated carbonate type linker (3c) to TentaGel resin-supported 24-residue hydrophobic peptide LVFFAEDVGSNKGAIIGLMVGGVV)>

The activated carbonate type linker (3c) (39 mg, 0.068 mmol) dissolved in DMF (1.5 mL) was mixed with the TentaGel resin-supported 24-residue hydrophobic peptide LVFFAEDVGSNKGAIIGLMVGGVV (0.15 mmol/g, 300 mg, 0.045 mmol), and the mixture was shaken at 25°C for 2 hours. Next, the mixture was filtered, and the obtained supported linker-bonded compound was washed with DMF (10 mL) and methylene chloride (10 mL) to obtain a supported linker-bonded compound to which the activated carbonate type linker (3c) is connected.

<Hydrophilic unit bonding step>

Deprotection of Fmoc was performed by adding a 20% piperidine/DMF solution (2 mL) to the supported linker-bonded compound and shaking the mixture for 20 minutes. This piperidine treatment operation was repeated four times in total. After washing with DMF, deprotection of an Fmoc group and coupling of an Fmoc amino acid were repeated by the same Fmoc solid-phase peptide synthesis as in Example 1. Accordingly, a 3-residue peptide chain composed of (Fmoc)Lys(Boc), (Fmoc)Lys(Boc), and (Fmoc)Lys(Boc) in this order was connected to the linker molecule.

<Support cleavage step>

A mixed solution of TFA : phenol: water : thioanisole : 3,6-dioxa-1,8-octanedithiol = 80:8:5:5:2 (volume ratio, 1 mL) was added to the well-dried supported hydrophilized peptide (44 mg, 0.00410 mmol), and the mixture was shaken for 2 hours. The support was separated by filtration, and the solid was washed with TFA. The filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 8.2 mg of a crude product of a support-free hydrophilized peptide.

<Chromatographic purification step>

The chromatographic purification step is preferably performed by the same method as in Example 1.
MALDI-TOF MS: (M+H+) m/z = 3014.550 (calculated value 3014.638)

<Linker cleavage step (cleavage of linker under acidic condition)>

1.0 mL a mixed solution of TFA : thioanisole : TMSOTf = 68:12:20 (volume ratio) was added to the support-free hydrophilized peptide (5 mg), and the mixture was stirred at 0°C for 4 hours. When diethyl ether (4 mL) was added to the TFA layer, a solid precipitated. After centrifugal separation, the supernatant was removed, and the solid was further washed with diethyl ether three times. Diethyl ether was removed under reduced pressure to obtain 7.0 mg of a crude product.

<Obtaining hydrophobic peptide (removal of solubilized part)>

The linker-cleaved hydrophobic peptide obtained by the previous step was washed with 1 mL of a phosphate buffer (pH 7.2) twice and further washed with 1 mL of water twice. The residue was vacuum-dried to obtain 13.0 mg of a hydrophobic peptide Leu-Val-Phe-Phe-Ala-Glu-Asp-Val-Gly-Ser-Asn-Lys-Gly-Ala-Ile-Ile-Gly-Leu-Met-Val-Gly-Gly-Val-Val as white powder.

MALDI-TOF MS analysis of the above hydrophobic peptide was carried out. A measured value was 2396.099 (calculated value 2392.287).

Example 10

<Production of supported crude peptide: HMP NovaGEL resin-supported 12-residue hydrophobic peptide CEWNSAHFIAYK (GRAVY score: - 0.31)>

A solid phase resin (HMP NovaGEL resin) supporting a 12-residue peptide composed of Ac-Cys(Trt)-Glu(Ot-Bu)-Trp(Boc)-Asn(Trt)-Ser(tBu)-Ala-His(Trt)-Phe-Ile-Ala-Tyr(tBu)-Lys(Boc) was obtained by Fmoc solid-phase peptide synthesis using the automatic peptide synthesizer.

<Linker bonding step (connection of activated carbonate type linker (3a) to HMP NovaGEL resin-supported 12-residue hydrophobic peptide CEWNSAHFIAYK)>

The activated carbonate type linker (3a) (179 mg, 0.30 mmol) dissolved in DMF (3 mL) was mixed with the HMP NovaGEL resin-supported 12-residue hydrophobic peptide CEWNSAHFIAYK (0.27 mmol/g, 500 mg, 0.14 mmol), and the mixture was shaken at 25°C for 2 hours. Next, the mixture was filtered, and the obtained supported linker-bonded compound was washed with DMF (10 mL) and methylene chloride (10 mL) to obtain a supported linker-bonded compound to which the activated carbonate type linker (3a) is connected.

<Hydrophilic unit bonding step>

Deprotection of Fmoc was performed by adding a 20% piperidine/DMF solution (3 mL) to the supported linker-bonded compound and shaking the mixture for 20 minutes. This piperidine treatment operation was repeated four times in total. After washing with DMF, deprotection of an Fmoc group and coupling of an Fmoc amino acid were repeated by the same Fmoc solid-phase peptide synthesis as in Example 1. Accordingly, a 3-residue peptide chain composed of (Fmoc)Lys(Boc), (Fmoc)Lys(Boc), and (Fmoc)Lys(Boc) in this order was connected to the linker molecule.

<Support cleavage step>

A mixed solution of TFA : phenol : water : thioanisole : 3,6-dioxa-1,8-octanedithiol = 80:8:5:5:2 (volume ratio, 1 mL) was added to the well-dried supported hydrophilized peptide (50 mg, 0.0088 mmol), and the mixture was shaken for 2 hours. The support was separated by filtration, and the solid was washed with TFA. The filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 8.2 mg of a crude product of a support-free hydrophilized peptide.

<Chromatographic purification step>

The chromatographic purification step is preferably performed by the same method as in Example 1.
MALDI-TOF MS: (M+H+) m/z = 2028.559 (calculated value 2027.864)

<Linker cleavage step (cleavage of linker under acidic condition)>

1.0 mL of a mixed solution of TFA : thioanisole : TMSOTf = 68:12:20 (volume ratio) was added to the support-free hydrophilized peptide (5 mg), and the mixture was stirred at 0°C for 4 hours. When diethyl ether (4 mL) was added to the TFA layer, a solid precipitated. After centrifugal separation, the supernatant was removed, and the solid was further washed with diethyl ether three times. Diethyl ether was removed under reduced pressure to obtain 6.9 mg of a crude product.

<Obtaining hydrophobic peptide (removal of solubilized part)>

The linker-cleaved hydrophobic peptide obtained by the previous step was washed with 1 mL of a phosphate buffer (pH 7.2) twice and further washed with 1 mL of water twice. The residue was vacuum-dried to obtain a hydrophobic peptide Ac-Cys-Glu-Trp-Asn-Ser-Ala-His-Phe-Ile-Ala-Tyr-Lys (3.0 mg) as white powder.

MALDI-TOF MS analysis of the above hydrophobic peptide was carried out. A measured value was 1510.951 (calculated value 1510.678).

Example 11

<Production of supported crude peptide: TentaGel resin-supported 24-residue hydrophobic peptide LVFFAEDVGSNKGAIIGLMVGGVV (GRAVY score: 1.63)>

A solid phase resin (TentaGel resin) supporting a 24 residue peptide composed of Leu-Val-Phe-Phe-Ala-Glu(tBu)-Asp(tBu)-Val-Gly-Ser(tBu)-Asn(Trt)-Lys(Boc)-Gly-Ala-Ile-Ile-Gly-Leu-Met-Val-Gly-Gly-Val-Val was obtained by Fmoc solid-phase peptide synthesis using the automatic peptide synthesizer.

<Linker bonding step (connection of activated carbonate type linker (3e) to TentaGel resin-supported 24-residue hydrophobic peptide LVFFAEDVGSNKGAIIGLMVGGVV)>

The activated carbonate type linker (3e) (55 mg, 0.068 mmol) dissolved in DMF (1.5 mL) was mixed with the TentaGel resin-supported 24-residue hydrophobic peptide LVFFAEDVGSNKGAIIGLMVGGVV (0.15 mmol/g, 300 mg, 0.045 mmol), and the mixture was shaken at 25°C for 2 hours. Next, the mixture was filtered, and the obtained supported linker-bonded compound was washed with DMF (10 mL) and methylene chloride (10 mL) to obtain a supported linker-bonded compound to which the activated carbonate type linker (3e) is connected.

<Hydrophilic unit bonding step>

Deprotection of Fmoc was performed by adding a 20% piperidine/DMF solution (2 mL) to the supported linker-bonded compound and shaking the mixture for 20 minutes. This piperidine treatment operation was repeated four times in total. After washing with DMF, deprotection of an Fmoc group and coupling of an Fmoc amino acid were repeated by the same Fmoc solid-phase peptide synthesis as in Example 1. Accordingly, a 3-residue peptide chain composed of (Fmoc)Lys(Boc), (Fmoc)Lys(Boc), and (Fmoc)Lys(Boc) in this order was connected to the linker molecule.

<Support cleavage step>

A mixed solution of TFA : phenol : water : thioanisole : 3,6-dioxa-1,8-octanedithiol = 80:8:5:5:2 (volume ratio, 1 mL) was added to the well-dried supported hydrophilized peptide (44 mg, 0.00410 mmol), and the mixture was shaken for 2 hours. The support was separated by filtration, and the solid was washed with TFA. The filtrate and the washing liquid were mixed, hexane (5 mL) was added to the mixture, and the mixture was vigorously shaken. Then, the mixture was allowed to stand to be separated into two layers. After the hexane layer (upper layer) was removed, and diethyl ether (5 mL) was added to the remaining TFA layer, a solid precipitated. After centrifugal separation, the supernatant was removed, and the solid was further washed with diethyl ether three times. Diethyl ether was removed under reduced pressure to obtain 8.2 mg of a crude product of a support-free hydrophilized peptide.

<Chromatographic purification step>

The chromatographic purification step is preferably performed by the same method as in Example 1.
MALDI-TOF MS: (M+H+) m/z = 3014.550 (calculated value 3014.638)

<Linker cleavage step (cleavage of linker under acidic condition)>

1.0 mL of a mixed solution of TFA : thioanisole : TMSOTf = 68:12:20 (volume ratio) was added to the support-free hydrophilized peptide (5 mg), and the mixture was stirred at 0°C for 4 hours. When diethyl ether (4 mL) was added to the TFA layer, a solid precipitated. After centrifugal separation, the supernatant was removed, and the solid was further washed with diethyl ether three times. Diethyl ether was removed under reduced pressure to obtain 7.0 mg of a crude product.

<Obtaining hydrophobic peptide (removal of solubilized part)>

The linker-cleaved hydrophobic peptide obtained by the previous step was washed with 1 mL of a phosphate buffer (pH 7.2) twice and further washed with 1 mL of water twice. The residue was vacuum-dried to obtain 13.0 mg of a hydrophobic peptide Leu-Val-Phe-Phe-Ala-Glu-Asp-Val-Gly-Ser-Asn-Lys-Gly-Ala-Ile-Ile-Gly-Leu-Met-Val-Gly-Gly-Val-Val as white powder.

MALDI-TOF MS analysis of the above hydrophobic peptide was carried out. A measured value was 2396.099 (calculated value 2392.287).

## Claims

1. A method for producing a purified peptide from a supported crude peptide including a support and a first peptide chain bonded to the support at a C-terminus, the method comprising:

   a hydrophilization step A of introducing a linker and a hydrophilic unit to an amino group of the supported crude peptide in this order stepwise or at a single step to obtain a support-free hydrophilized peptide;
   a support cleavage step B of cleaving a bond between the first peptide chain and the support at any stage before bonding the linker to the supported crude peptide until the hydrophilized peptide is obtained, or after a supported hydrophilized peptide is obtained;
   a chromatographic purification step of treating the support-free hydrophilized peptide obtained by the hydrophilization step A and the support cleavage step B, by liquid chromatography; and
   a linker cleavage step of cleaving a bond between the linker and the first peptide chain included in the chromatographically-purified support-free hydrophilized peptide, by chemical treatment.

2. The method for producing the purified peptide according to claim 1, wherein the linker cleavage step is performed by catalytic reduction or an acid.

3. The method for producing the purified peptide according to claim 2, wherein the linker cleavage step is performed by using a mixed solution containing an acid and a Lewis acid.

4. The method for producing the purified peptide according to claim 3, wherein the linker cleavage step is performed by using a mixed solution containing TFA and a Lewis acid.

5. The method for producing the purified peptide according to claim 4, wherein the linker cleavage step is performed by using a mixed solution containing TFA, TMSOTf, and thioanisole.

6. The method for producing the purified peptide according to claim 2, wherein the linker cleavage step is performed by using an acid having pKa of not higher than -2.

7. The method for producing the purified peptide according to claim 2, wherein the linker cleavage step is performed by using a metal supported catalyst.

8. The method for producing the purified peptide according to any one of claims 1 to 7, wherein the hydrophilic unit is a hydrophilic peptide, a polyether, or a polyamine.

9. The method for producing the purified peptide according to any one of claims 1 to 8, wherein
   the hydrophilization step A comprises a linker bonding step of bonding the linker to the amino group of the crude peptide to obtain a linker-bonded compound, and a hydrophilic unit bonding step of bonding the hydrophilic unit to a linker moiety of the obtained linker-bonded compound,
   wherein, the hydrophilic unit bonding step comprises
   bonding a plurality of amino acids to the linker moiety stepwise by Fmoc solid-phase peptide synthesis,
   bonding a previously prepared hydrophilic peptide chain to the linker moiety,
   bonding a previously prepared polyether to the linker moiety, or
   bonding a previously prepared polyamine to the linker moiety.

10. The method for producing the purified peptide according to any one of claims 1 to 9, wherein the support cleavage step B is performed after the supported hydrophilized peptide is obtained by the hydrophilization step A.

11. The method for producing the purified peptide according to any one of claims 1 to 10, wherein washing with water or a water-containing solvent is performed after the linker cleavage step.

12. The method for producing the purified peptide according to any one of claims 1 to 11, wherein a plurality of amino acids are sequentially bonded to the support by Fmoc solid-phase peptide synthesis to form the supported crude peptide.

13. The method for producing the purified peptide according to any one of claims 1 to 12, wherein the linker is bonded to the first peptide chain by forming a benzyloxycarbonylamino group or a benzylamino group.

**14.** The method for producing the purified peptide according to any one of claims 1 to 13, wherein the linker has a first group capable of forming a benzyloxycarbonylamino group or a benzylamino group together with the crude peptide and a second group capable of making a chemical bond to the hydrophilic unit.

**15.** The method for producing the purified peptide according to any one of claims 1 to 14, wherein the hydrophilic unit has a log P (P is an octanol-water partition coefficient) value of not higher than -1.

**16.** The method for producing the purified peptide according to any one of claims 1 to 15, wherein the hydrophilic unit is a hydrophilic peptide chain having two or more residues of at least one or more types of amino acid residues selected from the group of arginine, asparagine, glutamine, histidine, and lysine.

**17.** The method for producing the purified peptide according to any one of claims 1 to 16, wherein the hydrophilic unit is a hydrophilic peptide chain having two or more residues of at least one or more types of amino acid residues selected from the group of aspartic acid and glutamic acid.

**18.** The method for producing the purified peptide according to any one of claims 8 to 17, wherein the number of the amino acid residues of the hydrophilic peptide chain is not less than 2 and not greater than 35.

**19.** A linker, comprising:

a first group capable of forming a benzyloxycarbonylamino group or a benzylamino group together with a primary or secondary amine, and
a second group capable of making a chemical bond to a hydrophilic unit.

**20.** A linker represented by formula (1):

[Chemical formula 1]

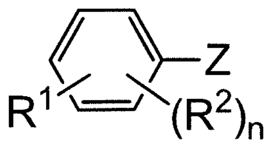

(1)

wherein $R^1$ is an organic group having a protected primary amino group, $R^2$ is an electron-withdrawing group, n is an integer of 0 to 4, and Z is a halogenated methyl group, a formyl group, or a carbonate group represented by formula (a):

[Chemical formula 2]

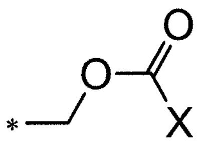

(a)

wherein X is a leaving group and * indicates a site bonded to the benzene ring of the compound (1).

**21.** A linker represented by formula (2), (4), or (6):

[Chemical formula 3]

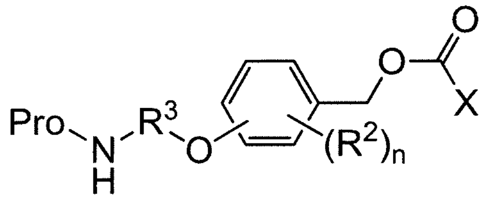

(2)

wherein $R^2$ is an electron-withdrawing group, n is an integer of 0 to 4, Pro is an amino-protecting group, X is $-OR^x$ ($R^x$ is a heterocyclic imide group), and $R^3$ is an alkylene group having 1 to 10 carbon atoms;

[Chemical formula 4]

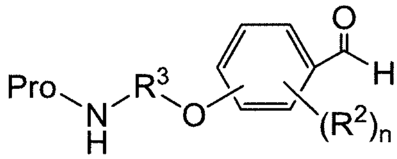

(4)

wherein $R^2$ is an electron-withdrawing group, n is an integer of 0 to 4, Pro is an amino-protecting group, and $R^3$ is an alkylene group having 1 to 10 carbon atoms; and

[Chemical formula 5]

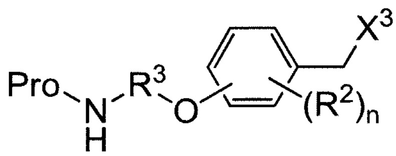

(6)

wherein $R^2$ is an electron-withdrawing group, n is an integer of 0 to 4, Pro is an amino-protecting group, $R^3$ is an alkylene group having 1 to 10 carbon atoms, and $X^3$ is a halogen atom.

**22.** A linker represented by formula (3a):

[Chemical formula 6]

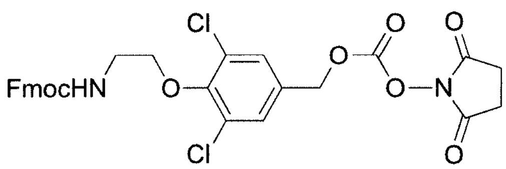

(3a)

Fig. 1

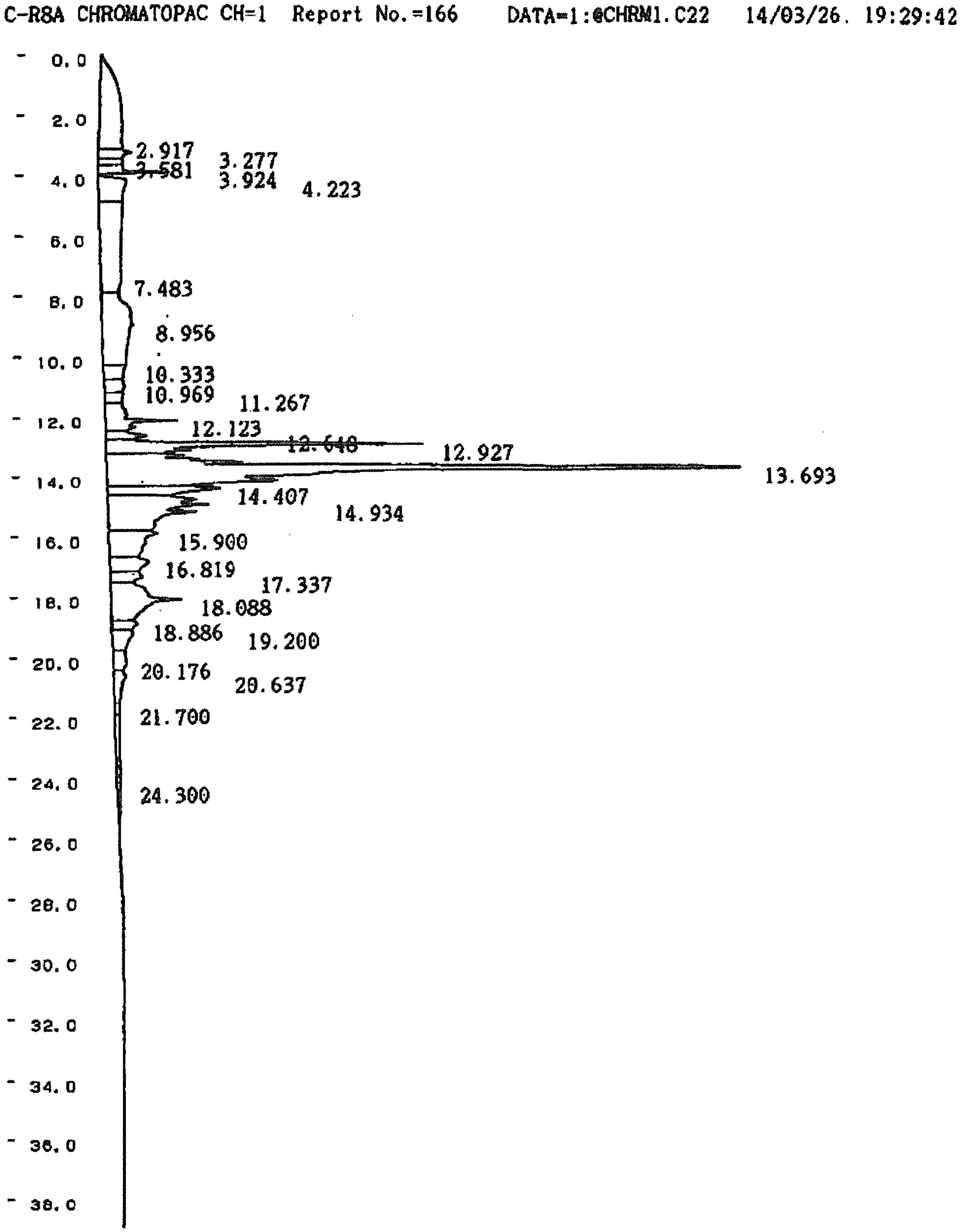
C-R8A CHROMATOPAC CH=1 Report No.=166 DATA=1:@CHRM1.C22 14/03/26. 19:29:42
0.0
2.0
4.0
6.0
8.0
10.0
12.0
14.0
16.0
18.0
20.0
22.0
24.0
26.0
28.0
30.0
32.0
34.0
36.0
38.0
2.917
3.277
3.581
3.924
4.223
7.483
8.956
10.333
10.969
11.267
12.123
12.640
12.927
13.693
14.407
14.934
15.900
16.819
17.337
18.088
18.886
19.200
20.176
20.637
21.700
24.300

Fig. 2

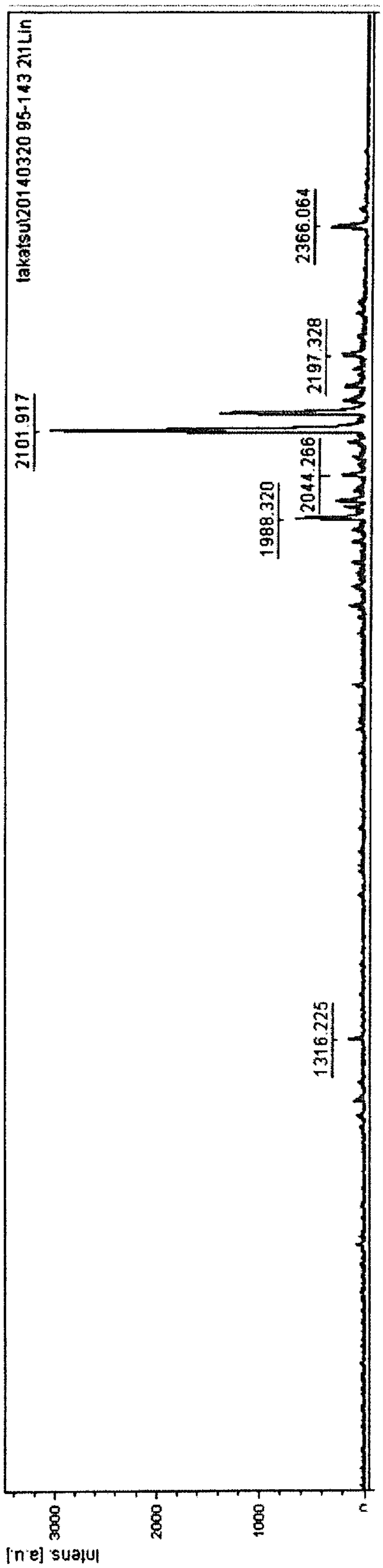
takatsu\20140320 95-143 2\1\Lin
2101.917
2197.328
2366.064
1988.320
2044.266
1316.225
Intens. [a.u.]
3000
2000
1000
0

Fig. 3

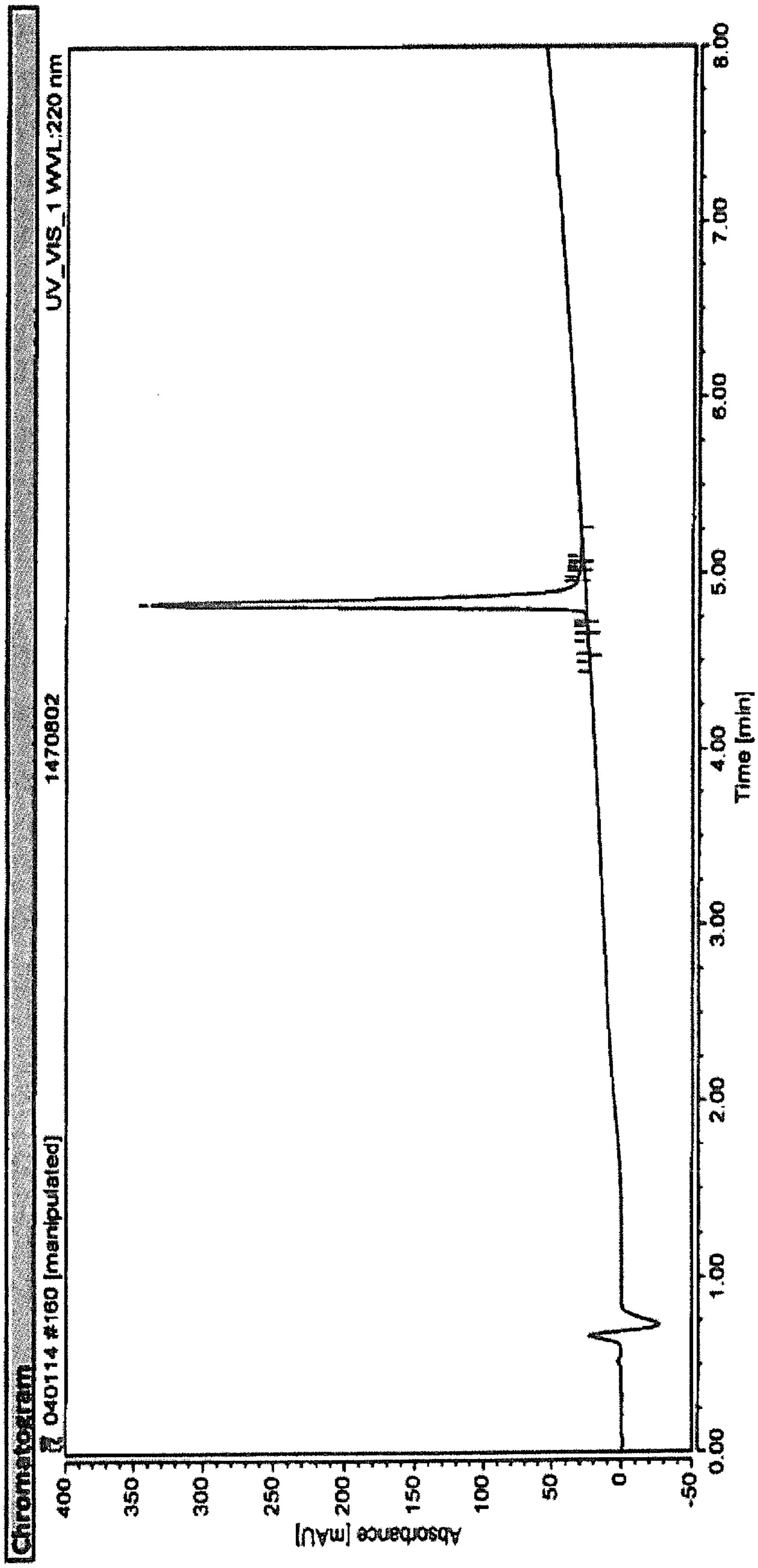
Chromatogram
040114 #160 [manipulated]
1470802
UV_VIS_1 WVL:220 nm
Absorbance [mAU]
400
350
300
250
200
150
100
50
0
-50
Time [min]
0.00
1.00
2.00
3.00
4.00
5.00
6.00
7.00
8.00

Fig. 4

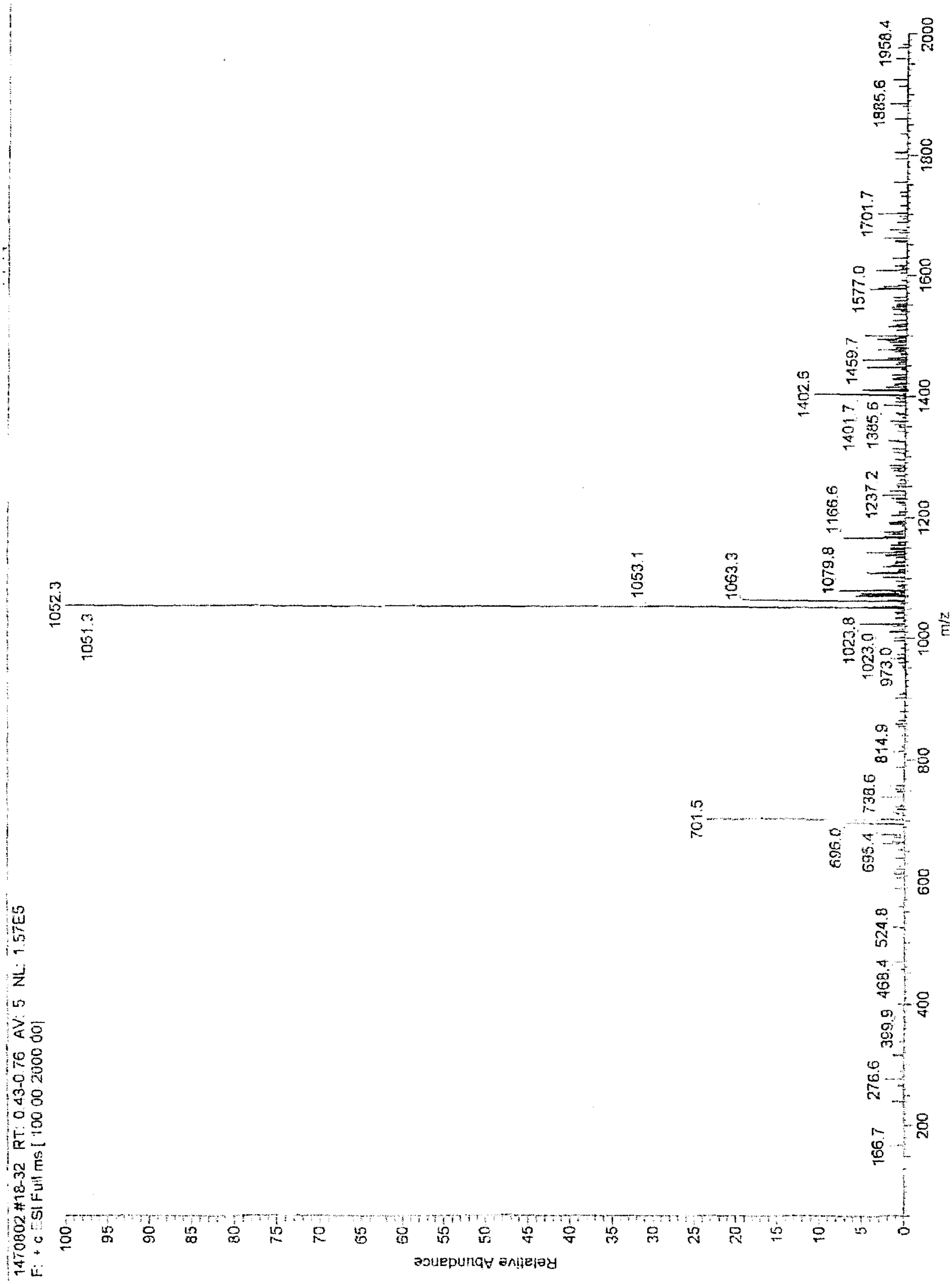
1470802 #18-32 RT: 0.43-0.76 AV: 5 NL: 1.57E5
F: + c ESI Full ms [ 100.00-2000.00]
Relative Abundance
m/z
1052.3
1051.3
1053.1
1063.3
1079.8
1166.6
1237.2
1402.6
1401.7
1385.6
1459.7
1577.0
1701.7
1885.6
1958.4
1023.8
1023.0
973.0
814.9
738.6
701.5
696.0
695.4
524.8
468.4
399.9
276.6
166.7

Fig. 5

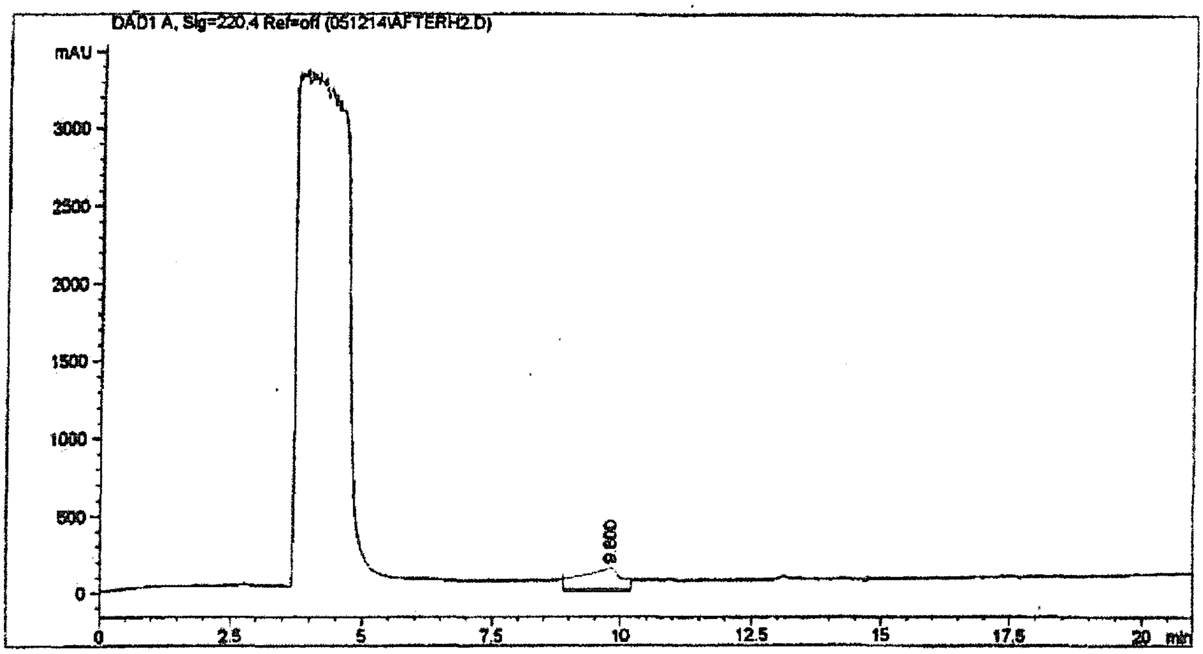
DAD1 A, Sig=220,4 Ref=off (051214\AFTERH2.D)
mAU
3000
2500
2000
1500
1000
500
0
9.800
0
2.5
5
7.5
10
12.5
15
17.5
20
min

Fig. 6

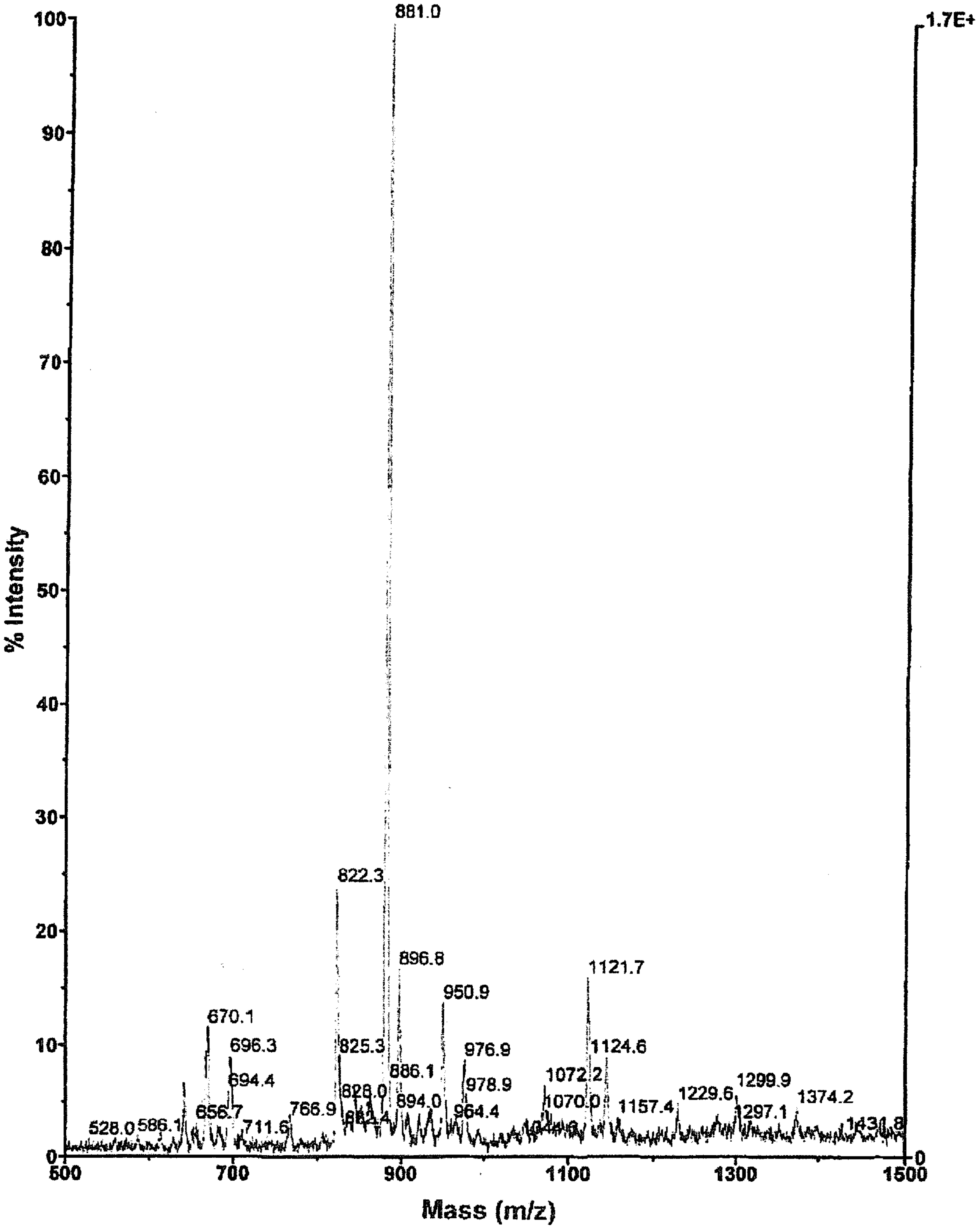
881.0
1.7E+
% Intensity
100
90
80
70
60
50
40
30
20
10
0
822.3
896.8
1121.7
950.9
670.1
696.3
825.3
976.9
1124.6
694.4
886.1
1072.2
978.9
828.0
894.0
1070.0
1157.4
1229.6
1299.9
1374.2
656.7
766.9
964.4
1297.1
528.0
586.1
711.6
1431.8
500
700
900
1100
1300
1500
Mass (m/z)

Fig. 7

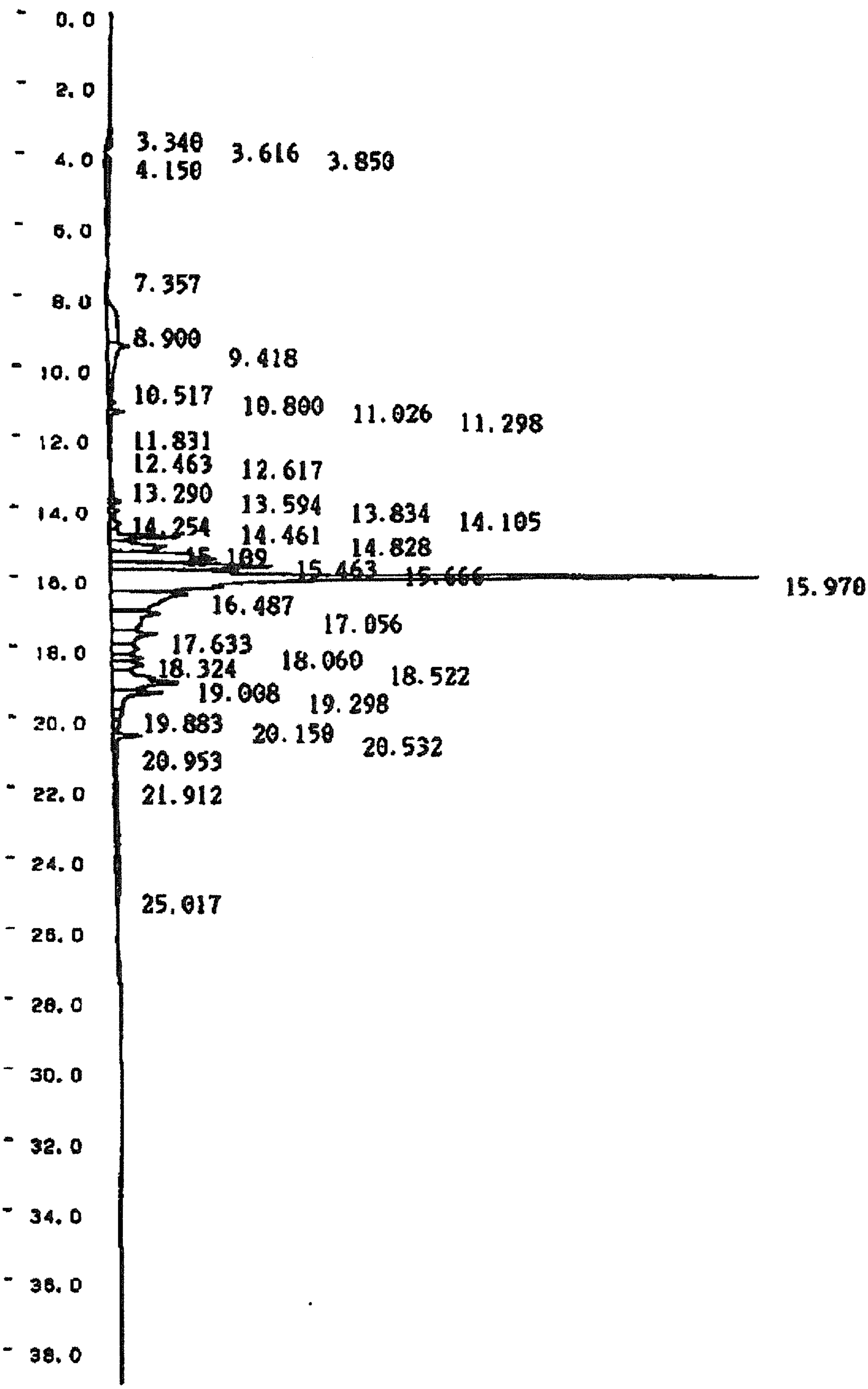
0.0
2.0
3.340
3.616
3.850
4.0
4.150
6.0
7.357
8.0
8.900
9.418
10.0
10.517
10.800
11.026
11.298
12.0
11.831
12.463
12.617
13.290
13.594
13.834
14.105
14.0
14.254
14.461
14.828
15.109
15.463
15.666
16.0
15.970
16.487
17.056
17.633
18.0
18.060
18.324
18.522
19.008
19.298
19.883
20.0
20.150
20.532
20.953
21.912
22.0
24.0
25.017
26.0
28.0
30.0
32.0
34.0
36.0
38.0

Fig. 8

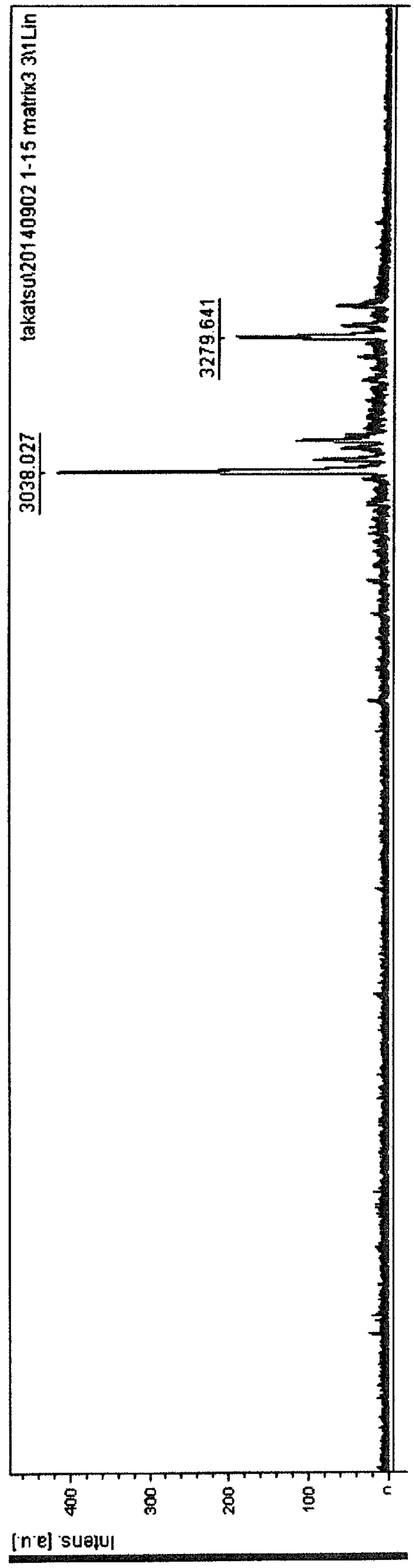
takatsu\20140902 1-15 matrix3 3\1Lin
3038.027
3279.641
400
300
200
100
0
Intens. [a.u.]

Fig. 9

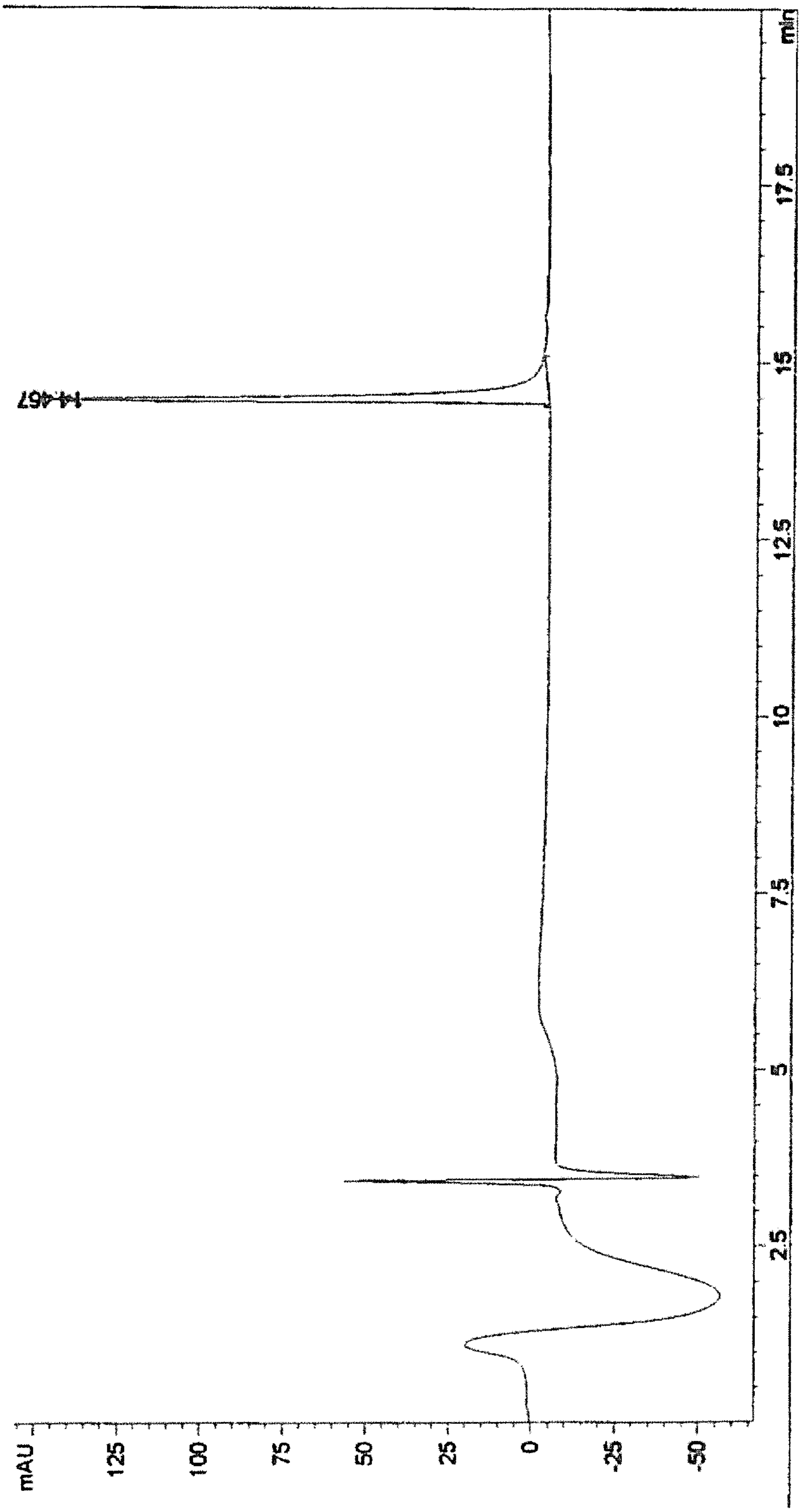

Fig. 10

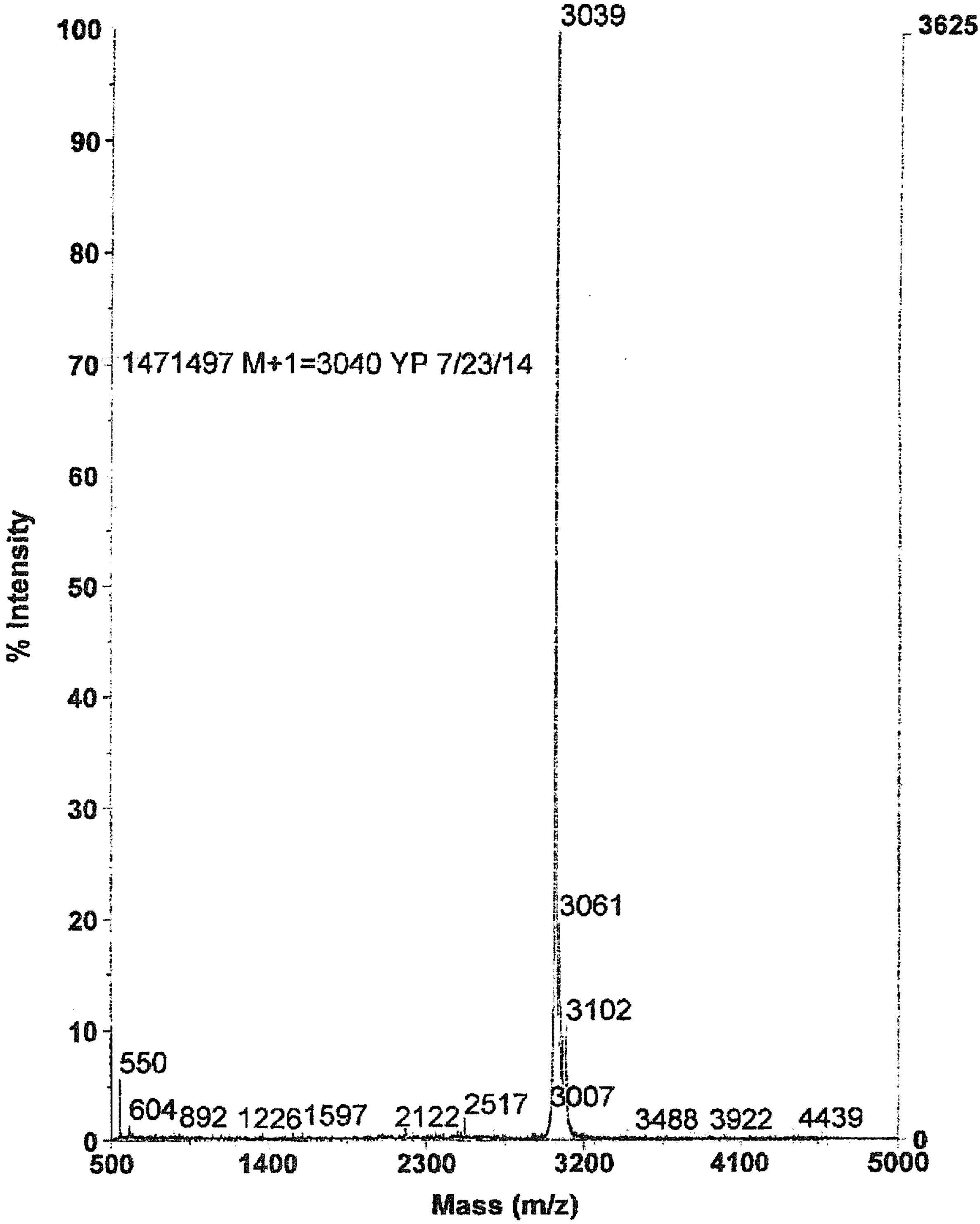
3039
3625
100
90
80
70
60
50
40
30
20
10
0
1471497 M+1=3040 YP 7/23/14
% Intensity
3061
3102
550
604
892
1226
1597
2122
2517
3007
3488
3922
4439
0
500
1400
2300
3200
4100
5000
Mass (m/z)

Fig. 11

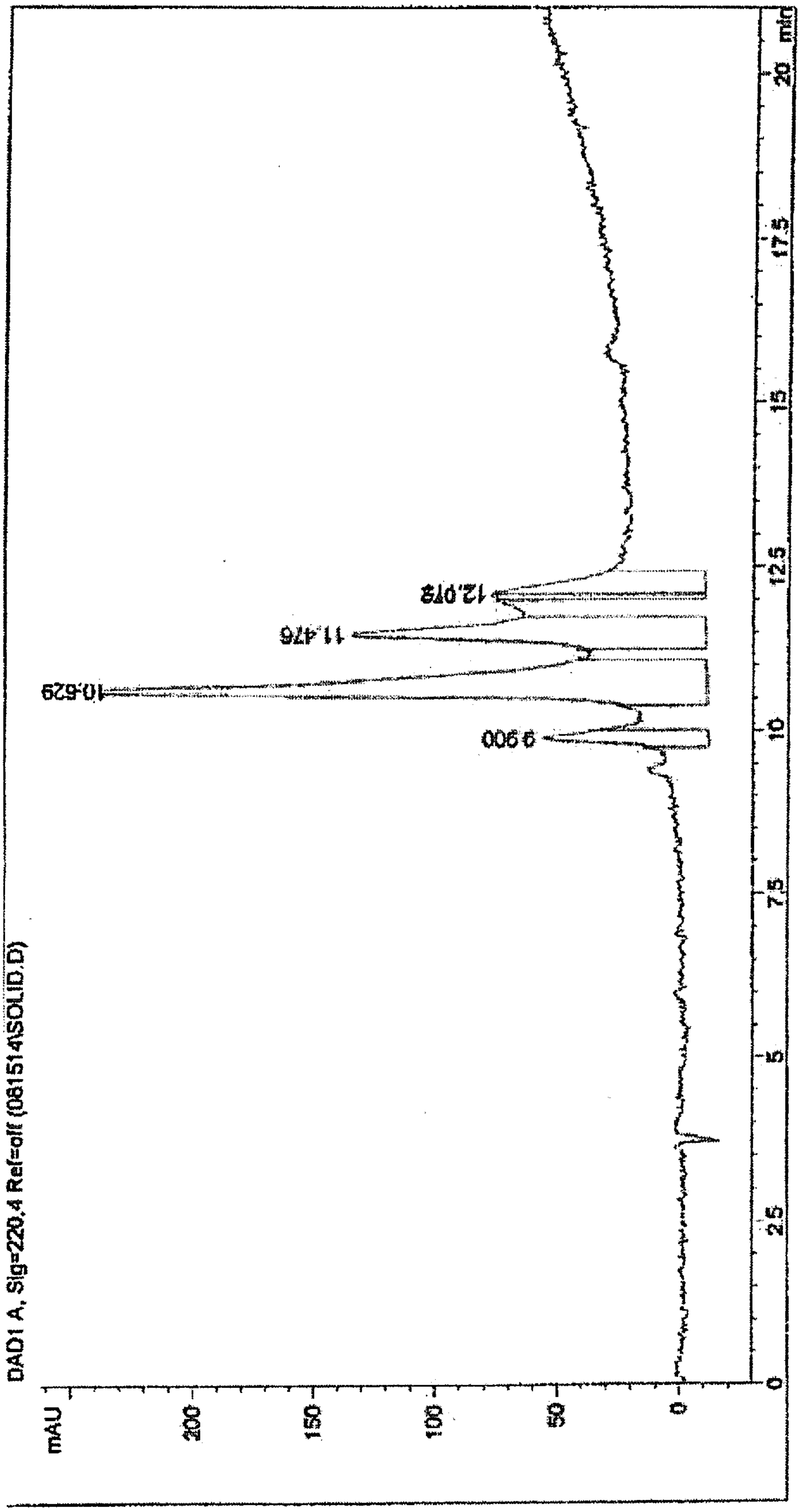
DAD1 A, Sig=220,4 Ref=off (081514\SOLID.D)
mAU
200
150
100
50
0
9.900
10.629
11.476
12.078
0
2.5
5
7.5
10
12.5
15
17.5
20
min

Fig. 12

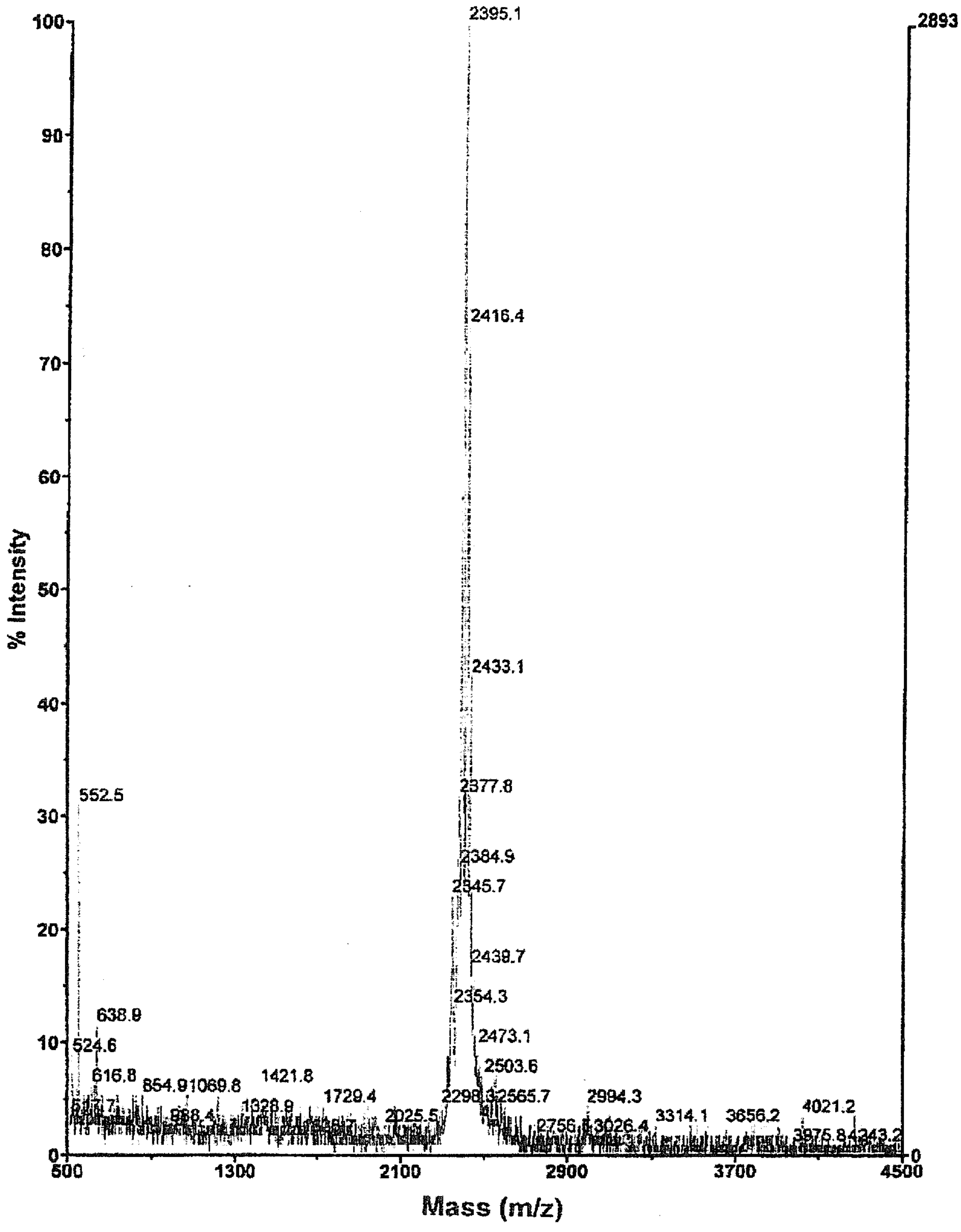

% Intensity
Mass (m/z)
2395.1
2893
2416.4
2433.1
2377.8
2384.9
2345.7
2439.7
2354.3
2473.1
2503.6
2565.7
552.5
638.9
524.6
616.8
854.9
1069.8
1421.8
1328.9
1729.4
2025.5
2994.3
3026.4
3314.1
3656.2
4021.2
3875.8
4243.2

## INTERNATIONAL SEARCH REPORT

International application No.
PCT/JP2015/077220

A. CLASSIFICATION OF SUBJECT MATTER
*C07K1/14*(2006.01)i, *C07K1/04*(2006.01)i

According to International Patent Classification (IPC) or to both national classification and IPC

B. FIELDS SEARCHED

Minimum documentation searched (classification system followed by classification symbols)
C07K1/14, C07K1/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII), CAplus/MEDLINE/EMBASE/BIOSIS(STN), DWPI(Thomson Innovation)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | VIZZAVONA Jean et al., "Covalent capture purification of polypeptides after SPPS via a linker removable under very mild conditions", Tetrahedron Letters, 2002, Vol.43, p.8693-8696 | 1-2,12<br>1-21<br>22 |
| X<br>Y<br>A | WO 2013/185771 A1 (CARLSBERG A/S, CARLSBERG FORSKNINGSCENTER),<br>19 December 2013 (19.12.2013),<br>compound 17<br>& US 2015/0140573 A1 | 19-21<br>1-21<br>22 |
| X<br>Y<br>A | HARA Toshiaki et al., "2-Nitrobenzylcarbamate-type photocleavable linker having Fmoc-aminoalkyloxy group: preparation and application to peptide purification", Peptide Science, 2008, Vol.45th, p.199-200 | 19-21<br>1-21<br>22 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 December 2015 (07.12.15) | 15 December 2015 (15.12.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/077220

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 7-102000 A (F. Hoffmann-La Roche AG.),<br>18 April 1995 (18.04.1995),<br>claims<br>& US 5750374 A<br>claims<br>& EP 641861 A2 & DE 69432449 A<br>& CN 1098416 A | 1-21<br>22 |
| A | WO 1992/06107 A1 (Nihon Millipore Ltd.),<br>16 April 1992 (16.04.1992),<br>claims; examples 5 to 7; fig. 1, 2<br>& US 5648462 A<br>claims; fig. 1 to 2<br>& US 5994588 A & EP 552368 A1 | 1-22 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H07102000 B
- US 62055991 B


### Non-patent literature cited in the description


- **ENGLEBRETSEN, D.R. et al.** *Tetrahedron,* 1999
- **HOSSAIN, M.A et al.** *Bioconjugate. Chem.,* 2009, vol. 20, 1390
- **CHOMA, C.T. et al.** *Tetrahedron Letters,* 1998, vol. 39, 2417
- **VORHERR, T.** *Chimica Oggi,* 2007, vol. 25, 22
- **DICK, F et al.** *Eur. Pept. Symp. 29th,* 2006
- **J. KYTE ; RF. DOOLITTLE.** *J Mol. Biol,* 1982, vol. 152, 105
- Chemical Reviews. *PARTITION COEFFICIENTS AND THEIR USES,* 1971, vol. 71, 525

SEQUENCE LISTING

```
<110>  Kaneka Corporation
       AnaSpec, Inc.

<120>  Method for Producing Hydrophobic Peptide

<130>  F15-109PCT

<150>  US62/055,991
<151>  2014-09-26

<160>  7

<170>  PatentIn version 3.5

<210>  1
<211>  9
<212>  PRT
<213>  Artificial Sequence

<220>
<223>  Hydrophobic peptide which is synthesized in the Examples.

<400>  1

Gly Ile Leu Thr Val Ser Val Ala Val
1               5


<210>  2
<211>  24
<212>  PRT
<213>  Artificial Sequence

<220>
<223>  Hydrophobic peptide which is synthesized in the Examples.

<400>  2

Leu Val Phe Phe Ala Glu Asp Val Gly Ser Asn Lys Gly Ala Ile Ile
1               5                   10                  15


Gly Leu Met Val Gly Gly Val Val
            20


<210>  3
<211>  12
<212>  PRT
<213>  Artificial Sequence

<220>
<223>  Hydrophobic peptide which is synthesized in the Examples.

<400>  3

Cys Glu Trp Asn Ser Ala His Phe Ile Ala Tyr Lys
1               5                   10


<210>  4
```

<211> 11
<212> PRT
<213> Artificial Sequence

<220>
<223> Hydrophilic peptide which is linked to the linker in the Examples.

<400> 4

Gly Lys Gly Lys Gly Lys Gly Lys Gly Lys Gly
1 5 10

<210> 5
<211> 3
<212> PRT
<213> Artificial Sequence

<220>
<223> Hydrophilic peptide which is linked to the linker in the Examples.

<400> 5

Lys Lys Lys
1

<210> 6
<211> 6
<212> PRT
<213> Artificial Sequence

<220>
<223> Hydrophilic peptide which is linked to the linker in the Examples.

<400> 6

Arg Arg Arg Arg Arg Arg
1 5

<210> 7
<211> 6
<212> PRT
<213> Artificial Sequence

<220>
<223> Hydrophilic peptide which is linked to the linker in the Examples.

<400> 7

Asp Asp Asp Asp Asp Asp
1 5